(19)

Europäisches
Patentamt
European
Patent Office
Office européen
des brevets

(11) **EP 3 452 985 B1**

(12) **EUROPEAN PATENT SPECIFICATION**

(45) Date of publication and mention
of the grant of the patent:
**26.07.2023  Bulletin 2023/30**

(21) Application number: **17730378.1**

(22) Date of filing: **03.05.2017**

(51) International Patent Classification (IPC):
**G06T 7/00** *(2017.01)*     **G01N 33/50** *(2006.01)*

(52) Cooperative Patent Classification (CPC):
**G06T 7/0012; G01N 33/5026; G01N 33/6875;**
G06T 2207/10056; G06T 2207/20036;
G06T 2207/20076; G06T 2207/30024

(86) International application number:
**PCT/EP2017/060532**

(87) International publication number:
**WO 2017/191187 (09.11.2017 Gazette 2017/45)**

(54) **NUCLEOLAR STRUCTURE EVALUATION AND MANIPULATION**

BEWERTUNG UND MANIPULATION VON NUKLEOLARER STRUKTUR

ÉVALUATION ET MANIPULATION DE STRUCTURE NUCLÉOLAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.05.2016  EP 16168087**

(43) Date of publication of application:
**13.03.2019  Bulletin 2019/11**

(73) Proprietor: **Université Libre de Bruxelles**
**1050 Brussels (BE)**

(72) Inventors:
  • **LAFONTAINE, Denis**
    **1342 Limelette (BE)**
  • **DE VLEESCHOUWER, Christophe**
    **5020 Vedrin (BE)**
  • **NICOLAS, Emilien**
    **6740 Etalle (BE)**
  • **PARISOT, Pascaline**
    **1348 Louvain-la-Neuve (BE)**

(74) Representative: **De Clercq & Partners**
**Edgard Gevaertdreef 10a**
**9830 Sint-Martens-Latem (BE)**

(56) References cited:
  • **Pia Muñoz Trallero ET AL: "Visual feature
    extraction to discriminate nucleolus phenotypes
    in fluorescence microscopy", Thesis submitted
    to the Ecole Polytechnique de Louvain in partial
    fulfillment for the degree of Electrical
    Engineering, 1 January 2014 (2014-01-01),
    XP055688491, Retrieved from the Internet:
    URL:https://upcommons.upc.edu/bitstream/ha
    ndle/2099.1/22748/thesis_PiaMuñoz.pdf?sequ
    ence=5&isAllowed=y [retrieved on 2020-04-22]**
  • **Qiang Wu ET AL: "Microscope Image
    Processing" In: "Microscope Image Processing",
    1 January 2008 (2008-01-01), Elsevier/Acad.
    Press, Amsterdam [u.a.], XP055618641, ISBN:
    978-0-12-372578-3 pages v-xx, * the whole
    document ***
  • **MARIE-FRANÇOISE O'DONOHUE ET AL:
    "Functional dichotomy of ribosomal proteins
    during the synthesis of mammalian 40S
    ribosomal subunits", THE JOURNAL OF CELL
    BIOLOGY : JCB, vol. 190, no. 5, 6 September 2010
    (2010-09-06), pages 853-866, XP055396312, US
    ISSN: 0021-9525, DOI: 10.1083/jcb.201005117**

EP 3 452 985 B1

**(Cont. next page)**

- T. TENG ET AL: "Loss of Tumor Suppressor RPL5/RPL11 Does Not Induce Cell Cycle Arrest but Impedes Proliferation Due to Reduced Ribosome Content and Translation Capacity", MOLECULAR AND CELLULAR BIOLOGY., vol. 33, no. 23, 23 September 2013 (2013-09-23), pages 4660-4671, XP055402814, US ISSN: 0270-7306, DOI: 10.1128/MCB.01174-13
- GOLOMB LIOR ET AL: "p53 and ribosome biogenesis stress: The essentials", FEBS LETTERS, ELSEVIER, AMSTERDAM, NL, vol. 588, no. 16, 18 April 2014 (2014-04-18), pages 2571-2579, XP029008738, ISSN: 0014-5793, DOI: 10.1016/J.FEBSLET.2014.04.014
- BURSAC SLADANA ET AL: "Activation of the tumor suppressor p53 upon impairment of ribosome biogenesis", BIOCHIMICA ET BIOPHYSICA ACTA. MOLECULAR BASIS OF DISEASE, vol. 1842, no. 6, 26 October 2013 (2013-10-26), pages 817-830, XP028599496, ISSN: 0925-4439, DOI: 10.1016/J.BBADIS.2013.08.014
- TAKAO KURODA ET AL: "RNA content in the nucleolus alters p53 acetylation via MYBBP1A : MYBBP1A transduces nucleolar stress signal to p53", EMBO JOURNAL., vol. 30, no. 6, 16 March 2011 (2011-03-16) , pages 1054-1066, XP055402775, GB ISSN: 0261-4189, DOI: 10.1038/emboj.2011.23
- HANNA T. GAZDA ET AL: "Ribosomal Protein L5 and L11 Mutations Are Associated with Cleft Palate and Abnormal Thumbs in Diamond-Blackfan Anemia Patients", AMERICAN JOURNAL OF HUMAN GENETICS, vol. 83, no. 6, 1 December 2008 (2008-12-01), pages 769-780, XP055402659, US ISSN: 0002-9297, DOI: 10.1016/j.ajhg.2008.11.004
- HAITONG SU ET AL: "Identification of Novel Markers That Demarcate the Nucleolus during Severe Stress and Chemotherapeutic Treatment", PLOS ONE, vol. 8, no. 11, 6 November 2013 (2013-11-06), page e80237, XP055402751, DOI: 10.1371/journal.pone.0080237
- EMILIEN NICOLAS ET AL: "Involvement of human ribosomal proteins in nucleolar structure and p53-dependent nucleolar stress", NATURE COMMUNICATIONS, vol. 7, 6 June 2016 (2016-06-06), page 11390, XP055402736, DOI: 10.1038/ncomms11390
- Pia Muñoz Trallero ET AL: "Visual feature extraction to discriminate nucleolus phenotypes in fluorescence microscopy", Thesis submitted to the Ecole Polytechnique de Louvain in partial fulfillment for the degree of Electrical Engineering, 1 January 2014 (2014-01-01), XP055688491, Retrieved from the Internet: URL:https://upcommons.upc.edu/bitstream/handle/2099.1/22748/thesis_PiaMuñoz.pdf?sequence=5&isAllowed=y [retrieved on 2020-04-22]
- Qiang Wu ET AL: "Microscope Image Processing" In: "Microscope Image Processing", 1 January 2008 (2008-01-01), Elsevier/Acad. Press, Amsterdam [u.a.], XP055618641, ISBN: 978-0-12-372578-3 pages v-xx, * the whole document *

Remarks:

The file contains technical information submitted after the application was filed and not included in this specification

## Description

### Field of the invention

The present invention relates to means and methods for evaluating nucleolar morphology.

### Background to the invention

Within the nucleus, the nucleolus is a specialized functional domain essential to gene expression (1). It is the site where the initial steps of ribosome biogenesis take place (2). Ribosomes are ribonucleoprotein (RNP) nanomachines converting the genetic information encoded in mRNAs into proteins. The human ribosome contains four ribosomal RNA (rRNAs) and eighty r-proteins organized in two subunits, each performing specialized functions in translation (3, 4). The small subunit (SSU), which consists of a single rRNA (18S) and thirty-three r-proteins, decodes the mRNA, while the large subunit (LSU), comprising three rRNAs (5S, 5.8S, and 28S) and forty-seven r-proteins, bears the peptidyl transferase center where amino acids are joined together into proteins. In the nucleolus, the 18S, 5.8S, and 28S rRNAs are synthesized by RNA polymerase I (Pol I) as long precursors, pre-rRNAs are modified, folded, and processed, and most r-proteins are assembled to form ribosomal subunits (2).

r-proteins are not involved in ribosome-mediated catalysis of peptide bond formation (3,5). Nonetheless, r-proteins play essential roles in shaping and maintaining the overall structure of the ribosomal subunits, and mutations in r-proteins are frequently associated with developmental disorders and human diseases (6). Notably, ribosomopathies are cancer predisposition syndromes caused by ribosome biogenesis dysfunction (7), due to mutations in r-proteins or ribosomal assembly factors, r-proteins are intimately linked to tumorigenesis, being directly involved in regulating the steady-state level of the anti-tumor protein p53 (8). This occurs via activation of specific anti-tumor surveillance pathways, through direct binding of specific r-proteins to the p53 regulator Hdm2 (see below and (9)).

The nucleolus is not limited by a lipid membrane. This makes it a highly dynamic structure that responds promptly, sometimes by profound morphological and compositional alterations, to cell stresses such as viral infections, DNA damage, and drug treatments (10,11). During interphase, the nucleoli of amniotic eukaryotes display three morphologically distinct layers (12, 13), which can be drastically re-organized under stress (14). During mitosis, the nucleolus undergoes a dramatic cycle of disassembly/reassembly that parallels Pol I activity controlled by specific phosphorylations (15,16). The number of nucleoli per cell nucleus and the shape and size of the nucleoli also vary greatly in proliferative diseases such as cancers (17). Cancer cells are more sensitive than non-cancer cells to inhibition of ribosome synthesis, and are killed selectively by treatment with Pol I inhibitors (18, 19). Despite the importance of the nucleolus as a cell stress sensor (20), disease biomarker and target for cancer therapy (21), how its structural integrity is maintained remains totally unclear.

While the principles of assembly and maintenance of the nucleolus are far from being understood (14), r-proteins which are very abundant, very basic, and which assemble mostly in the nucleolus onto pre-rRNAs to form ribosomal subunit precursors are likely to play an important role. The assembly of r-proteins is not random but follows a precise sequence of events. Groups of r-proteins have been defined on the basis of their assembly at early, intermediate, or late stages of ribosomal subunit biogenesis (22). Compromising the timely association of r-proteins with rRNA can indeed lead to severe pre-rRNA processing inhibitions, ribosomal subunit synthesis abortion, and sometimes to nucleolar structural alterations visible at the microscopic level (23). To date, no attempt has been made to systematically address the involvement of r-proteins in nucleolar structure maintenance or to grade their involvement in this process.

Muñoz Trallero ("Visual feature extraction to discriminate nucleolus phenotypes in fluorescence microscopy" (2014) Thesis submitted to the Ecole Polytechnique de Louvain in partial fulfillment for the degree of Electrical Engineering, Université catholique de Louvain, Belgium) describes image analysis tools for automated analysis and quantitative characterization of the visual appearance of the nucleolus conformation.

Wu et al. ("Microscope Image Processing" (2008) Elsevier Inc, Academic Press, Oxford, UK) relates to processing digital cell images, among which image segmentation as well as object measurement and classification.

While the nucleolus is a long-known cancer biomarker (17) and a recently demonstrated therapeutic target (19), it is not widely used by pathologists, however, for lack of reliable clinical assays. Accordingly, there is a need in the art to improve qualitative as well as quantitative nucleolus assessment, in order to aid for instance in diagnostics, such as stratification of nucleolar morphology, but as well in the development of model systems for instance for use in drug development or evaluation, in particular associated with or otherwise dependent on nucleolar phenotypes, including morphology, such as nucleolar disruption or nucleolar integrity.

### Summary of the invention

An object of the present invention is to provide methods and systems for discriminating populations of cells (e.g. normal/healthy versus abnormal/disease), for instance based on the analysis of the light captured by a sensor, when observing each population through an imaging device (e.g. a microscope).

To characterize nucleolar disruption both qualitatively and quantitatively, we developed a specific image-processing algorithm, of which a particular embodiment is detailed below. Briefly, we first segmented the observed nuclei on the basis of shape- and size-consistent adaptive thresholding of a nuclear stain (DAPI signal). Then, within each nuclear mass, GFP signal thresholding and mathematical morphology (see Methods) were applied to segment nucleoli into connected components. In order to optimize discrimination of nucleoli of cells depleted of an r-protein from those of SCR-treated control cells, five shape and textural features (or morphometric characteristics) were extracted from the largest connected components (LCC) of each nucleolus. These five features, selected from a set of eleven as the most discriminant ones, were: area, elliptical regularity, percentage of pixels below an optimized intensity threshold, smallest intensity, and number of local minima. For each of the five features, a $d_k$ value corresponding to a statistically significant distance between the feature distribution in cells depleted of an r-protein and control cells was computed.

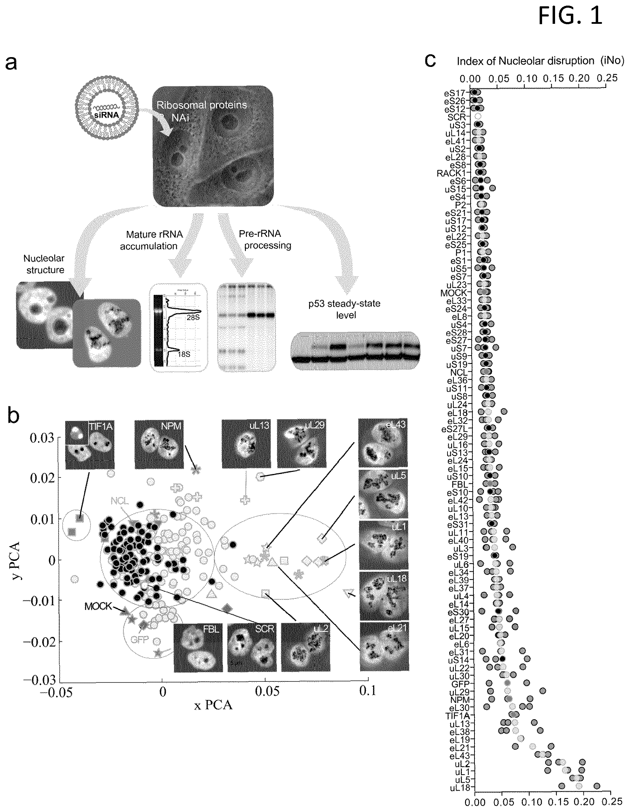

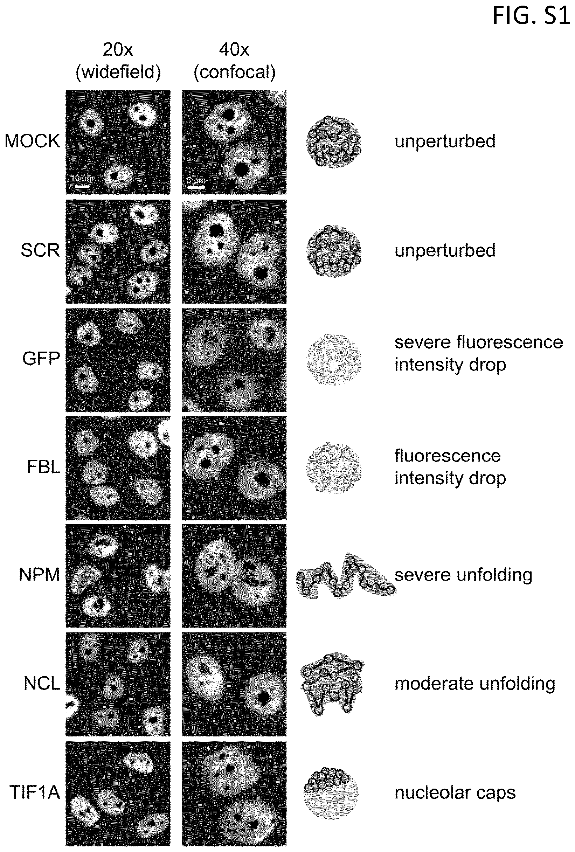

Each population of cells was thus characterized by five $d_k$ values. Principal component analysis (PCA) was used to reduce these five dimensions to two, allowing ready visualization of the data in a scatter plot (Fig. 1b) where each dot corresponds to a population of cells treated with one siRNA.

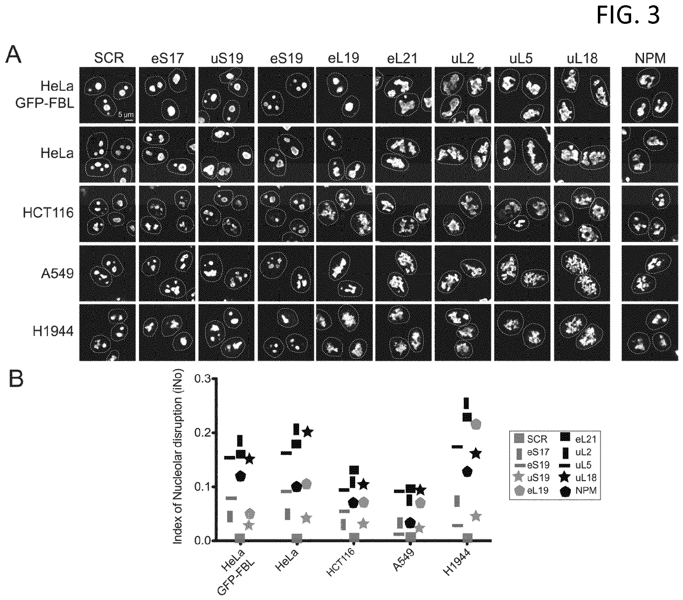

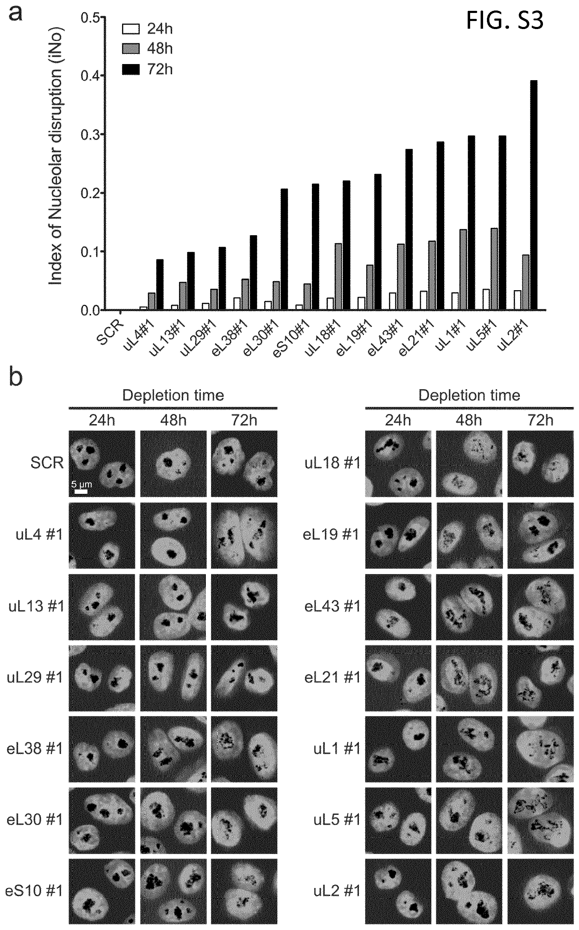

The nucleolus is a long-known cancer biomarker (17) and a recently demonstrated therapeutic target (19). It is not widely used by pathologists, however, for lack of reliable clinical assays. The image-processing algorithm and index of nucleolar disruption (iNo) developed here are robust and versatile tools for characterizing nucleolar morphological alterations both qualitatively and quantitatively. We have used them consistently in multiple cell lines, in time course analyses, and with either the dense fibrillar component or the granular component of the nucleolus (Fig. 3, Supplementary Figs S3,S4). We believe they hold great diagnostic and prognostic potential in cancer biology and research on ribosomopathies, several of which involve marked disruption of nucleolar integrity due to r-protein loss or mutation.

The present invention is defined by the appended claims.

## Figure legends

**FIG. 1**: Panel a) All eighty r-proteins were depleted one by one in human cells by use of specific siRNAs. Panel b) Principal component analysis (PCA) showing a classification of r-proteins according to their requirement for nucleolar structure maintenance. The image-processing algorithm that we designed involves selecting five discriminant shape and textural features (or morphometric characteristics), computing five dk values, and reducing the five dimensions to two by PCA. In the resulting plot, each dot represents one population of cells treated with one siRNA. Dot intensity is indicative of the targeted protein: black shapes for small subunit (SSU) r-proteins and white shapes for large subunit (LSU) r-proteins. The mean of three populations of cells treated with a non-targeting control siRNA (SCR) is indicated with a line connected to the corresponding image. The grey symbols represent the six calibration controls (FBL, GFP, NCL, NPM, MOCK, and TIF1A, see Supplementary Fig. 1). Insets show images of the nuclei of cells depleted of representative proteins with the DNA stained brightly highlighted and the nucleoli appearing as black shapes (fibrillarin). Panel c) r-proteins and calibration controls classified according to the severity of nucleolar disruption caused by their absence. The iNo or index of nucleolar disruption was defined as the sum of the dk values of the five most discriminant shape and textural features identified in this work (L1-norm of the discrepancy vectors computed from the images presented in our experimental screens of 80 r-proteins (see Example 2). Higher iNo correspond to more severe disruption. Color-coding as in panel b; i.e., black for small subunit (SSU) r-proteins and white for large subunit (LSU) r-proteins. The gray dots are the means of three individual experiments. Note: The r-proteins are named according to a recently revised nomenclature, where the "e" prefix stands for eukaryote-specific and "u" for universal (present in bacteria, archaea, and eukaryotes).

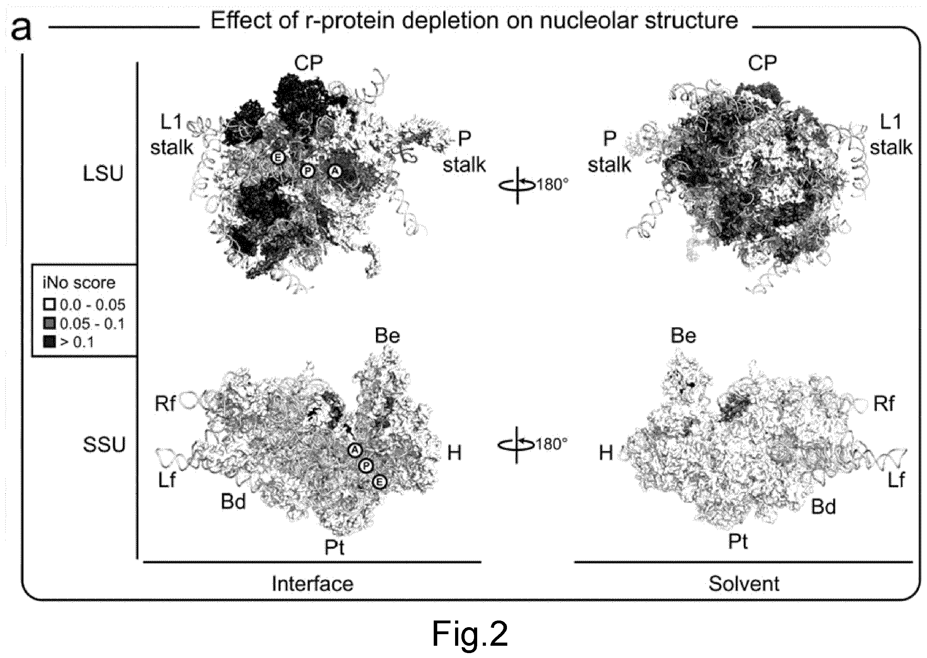

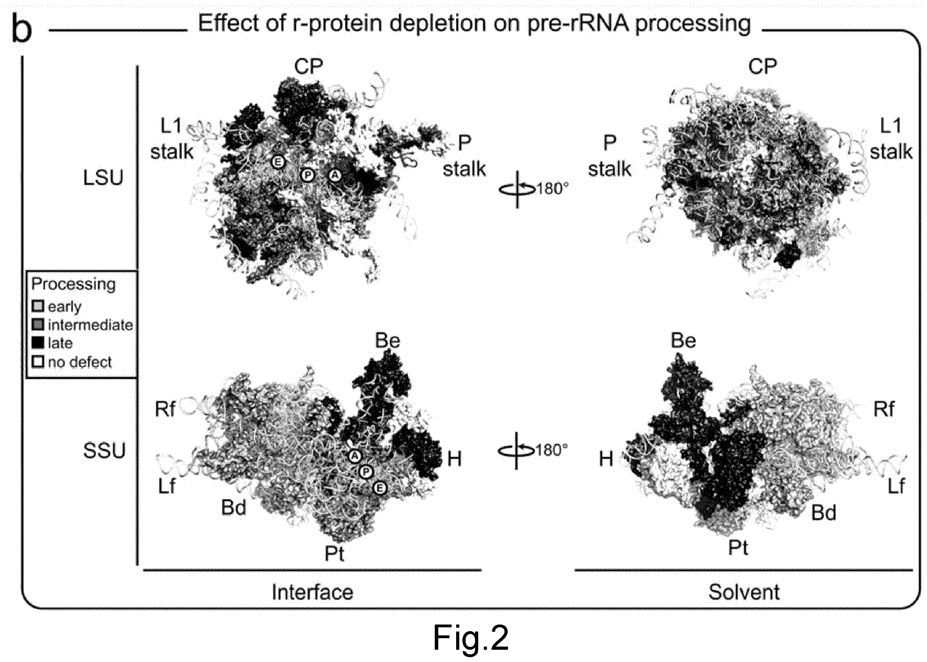

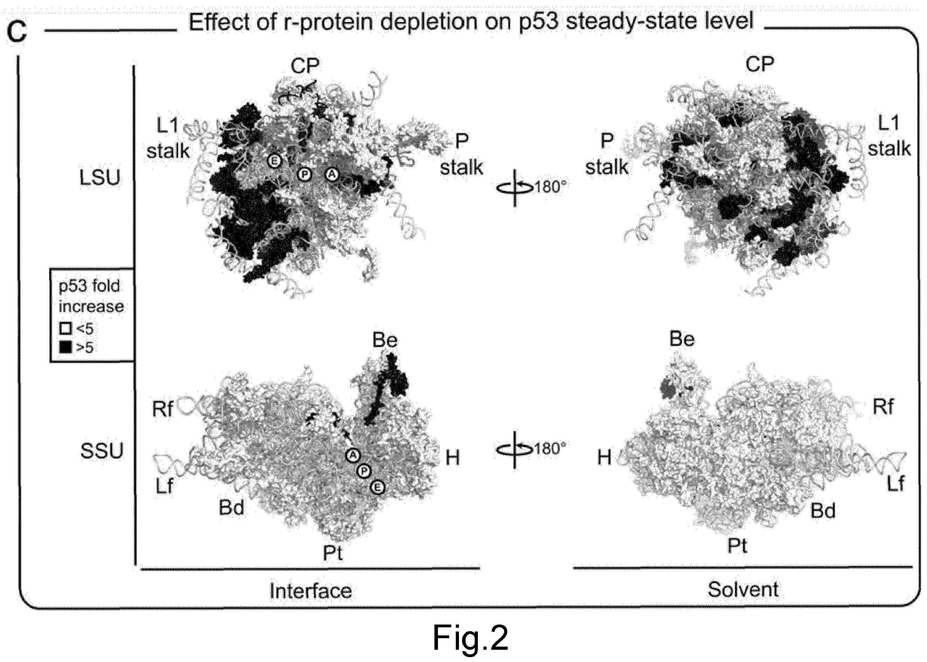

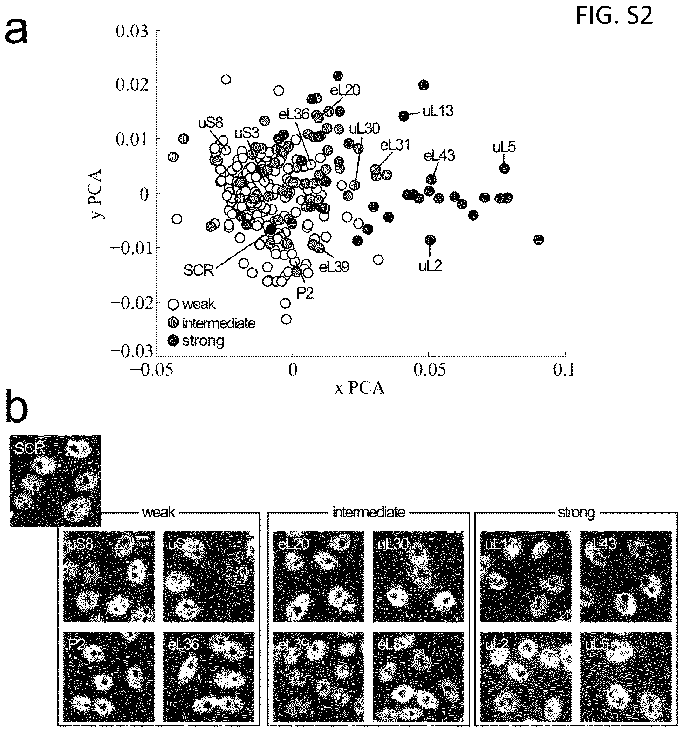

**FIG. 2**: The r-proteins are color-coded according to the impact of their depletion on nucleolar structure (iNo values) (a), pre-rRNA processing (b), or the p53 steady-state level (c). Left, subunit interface views; right, solvent-exposed views.

**FIG. 3**: illustrates the quantitative monitoring of nucleolar morphology in different human cell lines, based on detection of endogenous PES1. The data show, for a selection of eight representative r-proteins, that the r-proteins contributing weakly or strongly to nucleolar structure maintenance are largely the same in multiple cell lines.

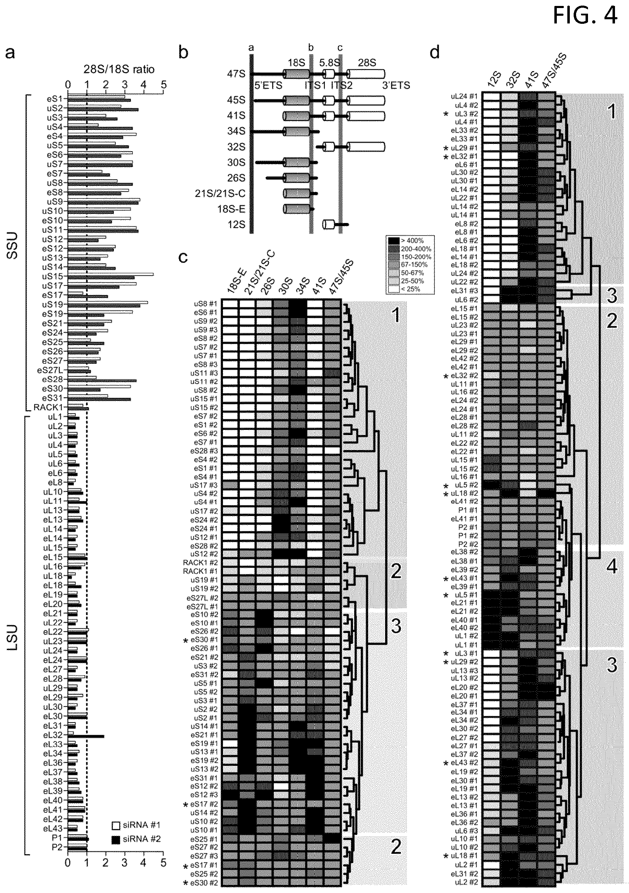

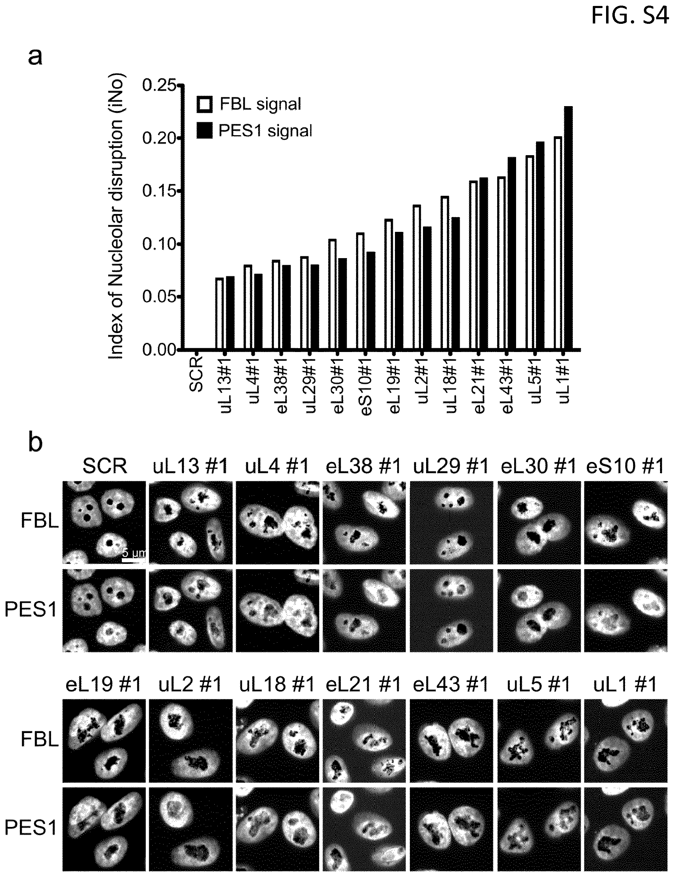

**FIG. 4** - not covered by the claims: depicts schematically the involvement of human r-proteins in pre-rRNA processing, (a) The 28S/18S ratio calculated from Agilent bioanalyzer electropherograms. Data are shown for the two different siRNAs used (siRNA #1 and #2). (b) Major pre-rRNA intermediates and probes used in this work. Three of the four rRNAs are produced by RNA Pol I as a long 47S primary transcript. The 18S, 5.8S, and 28S rRNAs are separated by noncoding external (ETS) and internal (ITS) transcribed spacers. Probes a, b, and c are the oligonucleotides LD1844, LD1827, and LD1828, respectively (see Example 1). (c) Pre-rRNA processing inhibitions upon depletion of SSU r-proteins. On the northern blots (see Supplementary Figs S5,S6, S11), all RNA species were quantified with a Phosphorimager, normalized with respect to the non-targeting control (SCR), and their abundances represented on a heatmap using the color code indicated. The heatmap profiles were clustered with "R" and the corresponding proteins grouped in classes of r-proteins affecting the same or similar processing steps. The different siRNAs used are indicated (#). Asterisks refer to r-proteins assigned to two groups according to the siRNA used. (d) As in panel c for LSU r-proteins.

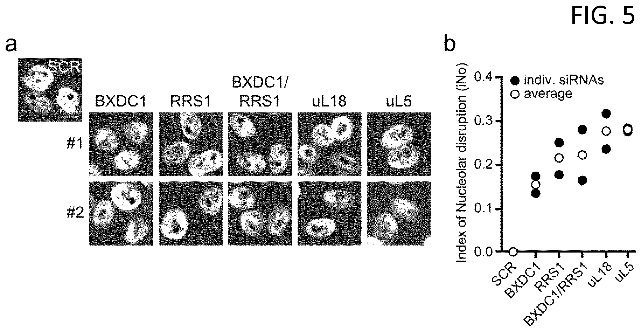

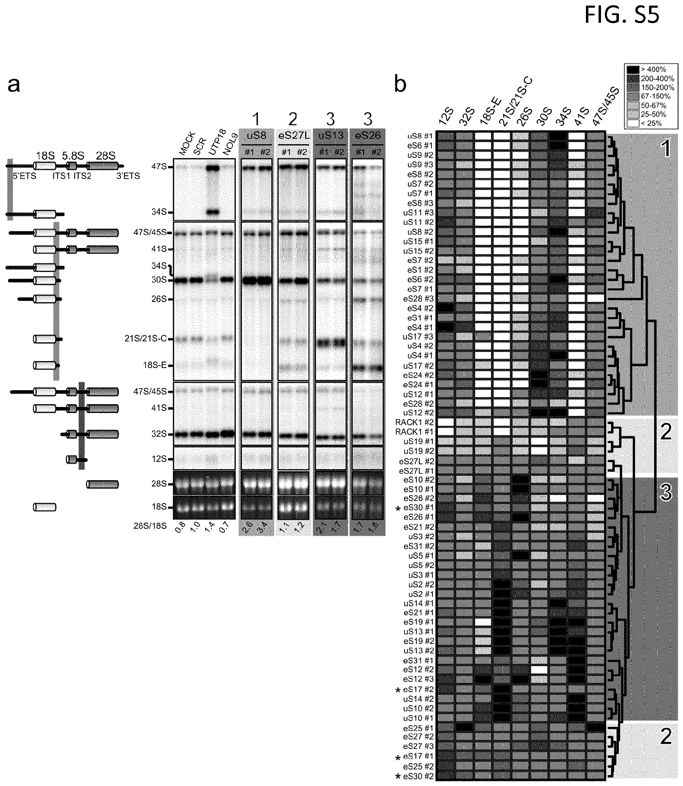

FIG. 5: Two independent siRNAs (#1 and #2) were used in each case. Cells treated with a non-targeting (SCR) siRNA control are shown for reference. (b) For each depletion, the nucleolar disruption index (iNo) was calculated (see Fig. 1 and Example 1).

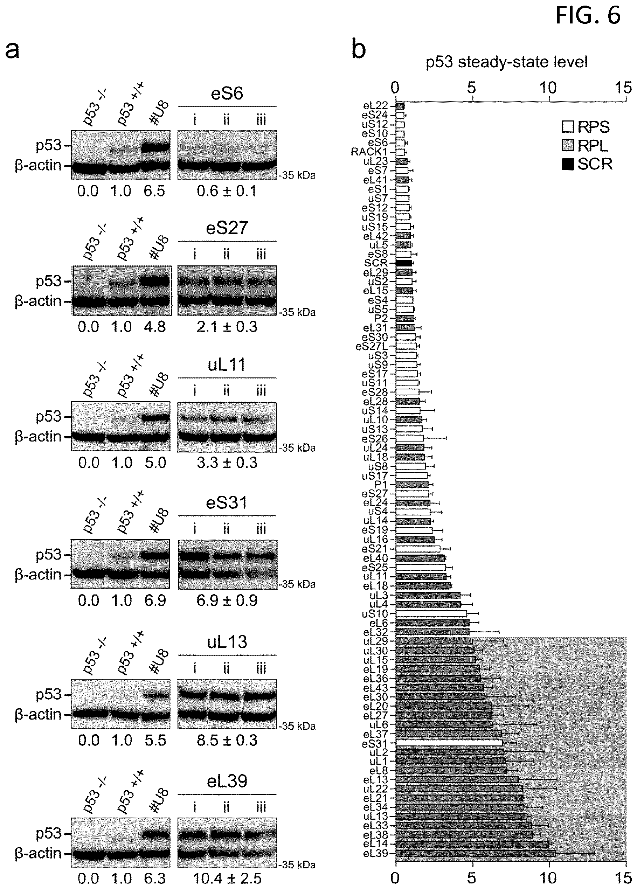

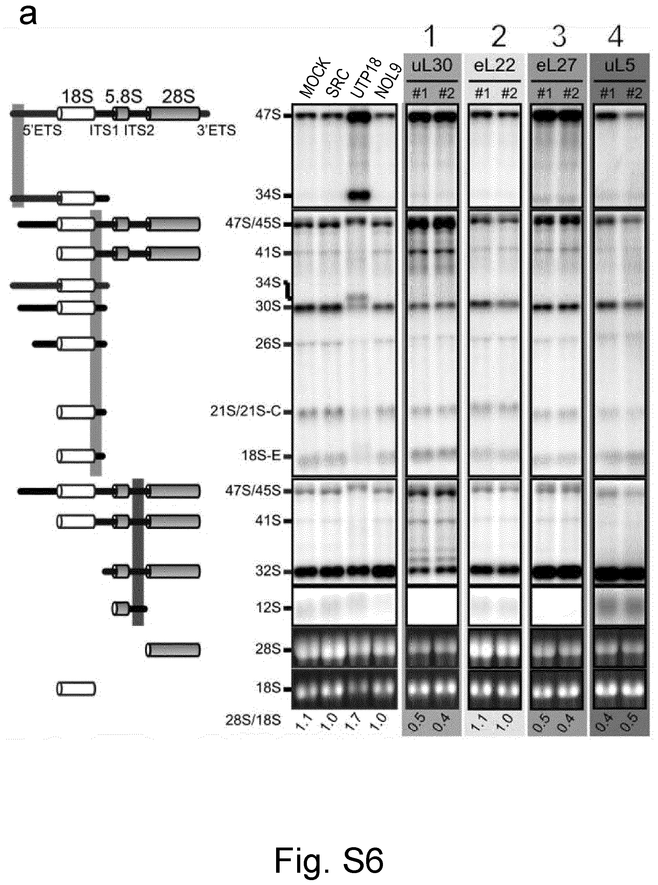

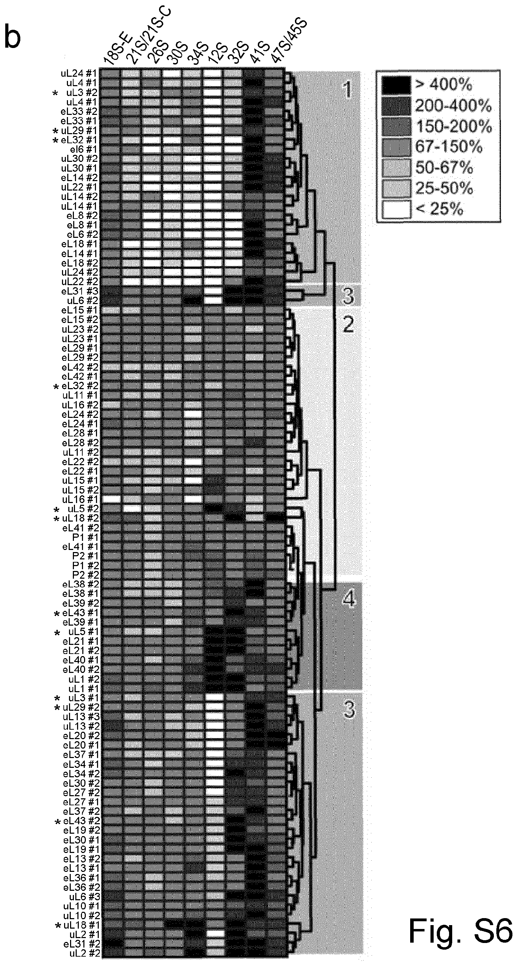

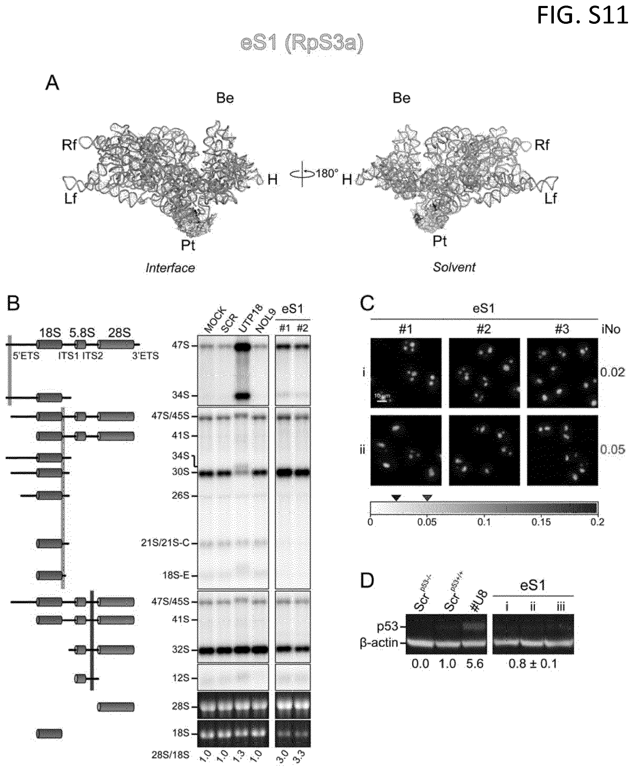

FIG. 6 - not covered by the claims: illustrates the involvement of human r-proteins in p53 homeostasis. (a) Steady-state level of p53 determined by quantitative fluorescent western blotting. Western blots analysis are shown for representative r-proteins, with the p53 level indicated underneath as a mean of biological triplicates obtained after treatment of cells with the same siRNA (i, ii, and iii). The siRNA used was selected on the basis of its proven efficacy in the processing and nucleolar screens (Figs 1 and 4). The p53 signal corrected for loading (using β-actin as reference) was expressed with respect to the level observed in cells treated with a non-targeting siRNA control (p53 +/+). The upper signals correspond with p53; the bottom signals with β-actin. A complete data set for all eighty r-proteins is available in Supplementary Fig. 11. As loading control we used HCT116 p53+/+ cells transfected with a non-targeting siRNA (p53 +/+) providing the basal level of p53 or with an antisense oligonucleotide suppressing the activity of the box C/D snoRNA U8 (#U8), thereby stimulating p53 accumulation up to 6-fold (J.-L.L, E.N. and D.L.J.L., submitted). As background control, we used a matched isogenic HCT116 cell line that does not express p53 (HCT116 p53-/-, ref.39) treated with a non-targeting siRNA (p53 -/-).

(b) r-proteins classified according to their impact on the p53 steady-state level. The non-targeting (SCR) control is shown in black, the SSU r-proteins in white, and the LSU r-proteins in grey. The histogram bars are the means of triplicates with SD. r-proteins whose depletion leads to a 5-fold increase in p53 level are highlighted in a gray box.

**Supplementary FIG. 1** - not covered by the claims: The nucleolar calibration set. The calibration set used consisted of four control proteins whose depletion, we established, strongly disrupts nucleolar structure. These control proteins are: the RNA polymerase I (Pol I) transcription factor TIF1A, nucleolin (NCL), and nucleophosmin (NPM). As further standardization controls, we used mock-treated (MOCK) cells, cells treated with an siRNA targeting FBL or GFP, and cells treated with a non-targeting siRNA (SCR). White signal, DNA stain (DAPI); black signal within white, GFP. Left column, images captured at 20x magnification in widefield mode. Right column, images captured at 40x magnification in confocal mode. To the right of these images, schematics depicting the effect of siRNA-mediated depletion on nucleolar structure and signal intensity.

**Supplementary FIG. 2**: Benchmarking the automated unsupervised classification of nucleolar disruption phenotypes. To benchmark our novel classification algorithm, we compared the automated classification described in the text with a manual one. The manual classification was based on the fine visual inspection of representative images obtained after depletion with each siRNA used and on the assignment of nucleolar disruption phenotypes to three arbitrarily defined classes corresponding to weak, intermediate, and strong disruption. Superimposition of the automatically and manually obtained classifications made us highly confident that assignment to phenotypic classes on the basis of our automated procedure is robust. (a) PCA (see Fig. 1b). Each circle represents a population of cells treated with one siRNA specific to one r-protein. The circles are intensity-coded according to a manual classification based on the fine visual inspection of the microscopic images. The non-targeting control (Scramble, SCR) is indicated with an arrow. Strong contributors to nucleolar structure maintenance are black; intermediate contributors are in grey; weak contributors are in white. (b) Representative images illustrating the manual classification of r-proteins as weak, intermediate, and strong contributors to nucleolar structure maintenance. White signal, DNA stain; black signal, fibrillarin.

**Supplementary FIG. 3**: Kinetics of nucleolar disruption after siRNA-mediated r-protein depletion. The data show that the computed iNo values reliably reflect phenotype severity, and that nucleolar disruption is best scored in cells having undergone two successive rounds of nucleolar breakdown/nucleolar genesis, corresponding to two cell divisions (~72h). (a) Values of the nucleolar disruption index (iNo) obtained after siRNA-mediated depletion of the indicated r-protein for 24 h, 48 h, and 72 h, which are white, grey, and black, respectively. (b) Representative images for thirteen r-proteins tested at each time point. White signal, DNA stain; Black signal, fibrillarin. #1 refers to the siRNA used (see Example 1).

**Supplementary FIG. 4**: Quantitative monitoring of nucleolar morphology based on detection of the endogenous granular component marker PES1. The data show that the iNo values can be computed equally on the basis of DFC (fibrilarin, FBL) or GC (PES 1) antigen detection. (a) Values of the nucleolar disruption index (iNo) obtained after 3 days of siRNA-mediated depletion of the indicated r-protein as calculated on the basis of the fibrillarin signal (white bars) or the PES1 signal (black bars). (b) Representative images for the thirteen r-proteins tested. In each case the siRNA n°1 (#1) was used (see Example 1). White signal, DNA stain; black signal, fibrillarin (top rows) or PES1 (bottom rows).

**Supplementary FIG. 5** - not covered by the claims: Involvement of small subunit r-proteins in pre-rRNA processing, (a) Representative examples of northern blots for each of the three classes of SSU r-proteins defined in this work (for a full dataset see Supplementary Fig. 11). A calibration set consisting of mock-treated cells and cells treated with a non-targeting siRNA (SCR) or a siRNA targeting UTP18 or NOL9 was used systematically (see ref.1). Schematics of the RNA intermediates detected are shown on the left. Ratios of 28S to 18S mature rRNA were calculated from bioanalyzer electropherograms. (b) Expanded version of Fig. 4c, showing all RNA intermediates detected and quantified.

**Supplementary FIG. 6** - not covered by the claims: Involvement of large subunit r-proteins in pre-rRNA processing, (a) Representative examples of northern blots for each of the four classes of LSU r-proteins defined in this work (for a full dataset see Supplementary Fig. 11). A calibration set consisting of mock-treated cells and cells treated with a non-targeting siRNA (SCR) or a siRNA targeting UTP18 or NOL9 was used systematically (see ref.1). Schematics of the RNA intermediates detected are shown on the left. 28S/18S mature rRNA ratios were calculated from bioanalyzer electropherograms. (b) Expanded version of Fig. 4d, showing all RNA intermediates detected and quantified.

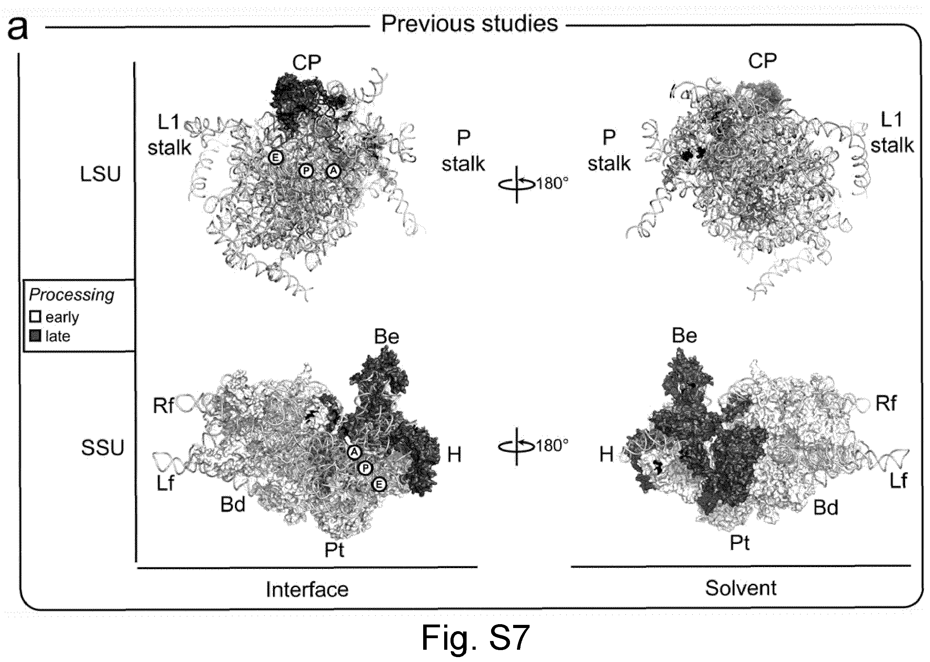

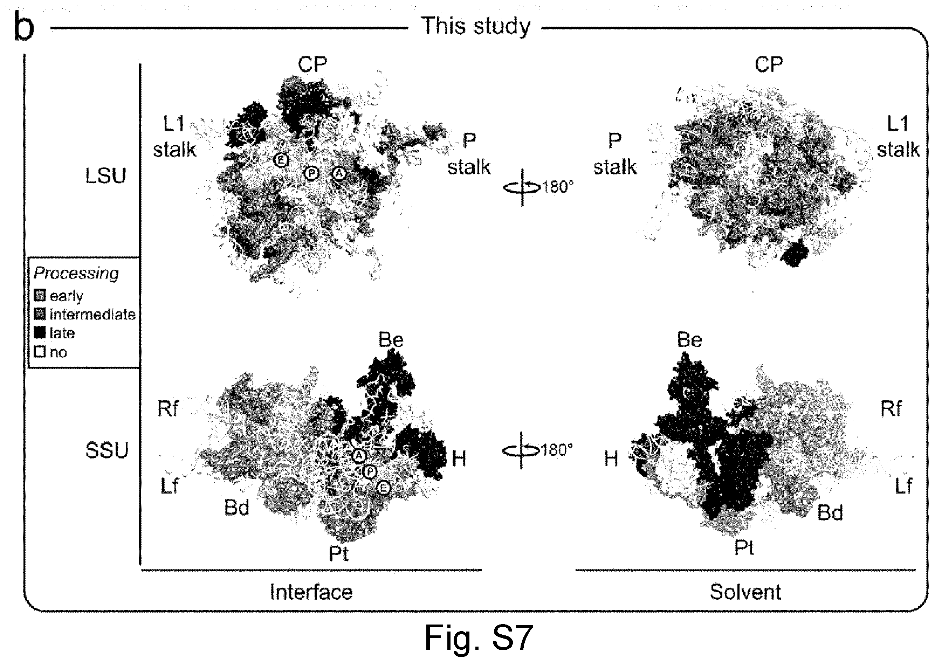

**Supplementary FIG. 7** - not covered by the claims: Comparison of our classification of r-proteins according to their involvement in pre-rRNA processing with previous studies. The figure shows that our work either confirms (small subunit r-proteins) or considerably complements (large subunit r-proteins) the literature. 3-D models of human ribosomal subunits based on PDB entries 3J3D, 3J3A, 3J3F, and 3J3B. Left, subunit interface views; right, solvent-exposed views. The aminoacyl (A), peptidyl (P), and exit (E) tRNA sites are indicated. Morphological features of the subunits are highlighted. On the LSU: the L1-stalk, central protuberance (CP), and phospho-stalk (P-stalk). On the SSU, the beak (Be), head (H), platform (Pt), body (Bd), left foot (Lf), and right foot (Rf). (a) Previous studies: conducted on cervix cancer cells where p53 expression is disrupted by HPV integration (HeLa cells). The SSU r-proteins were tested in ref. 2; six out of the forty-seven LSU r-proteins were tested in ref. 3. (b) This work: conducted on colon carcinoma cells expressing p53 normally (HCT116 p53+/+)(based on Fig. 4 and Supplementary Figs. S5,S6,S11). All r-proteins were tested.

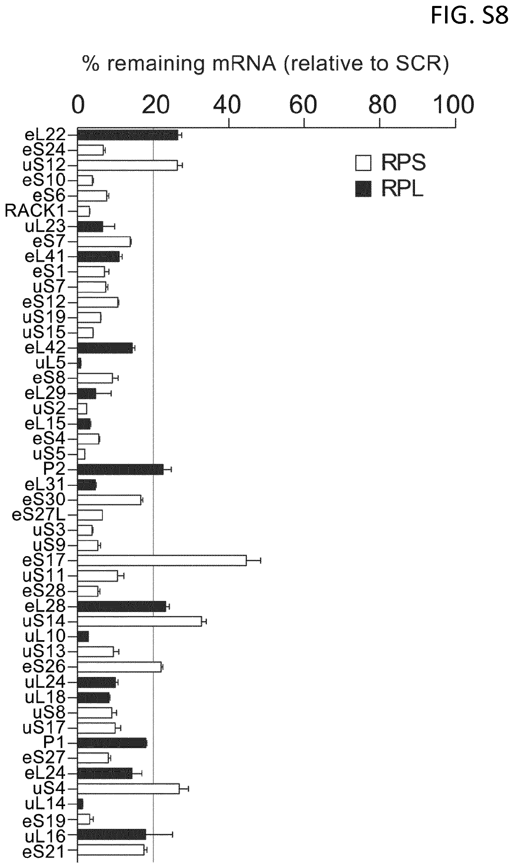

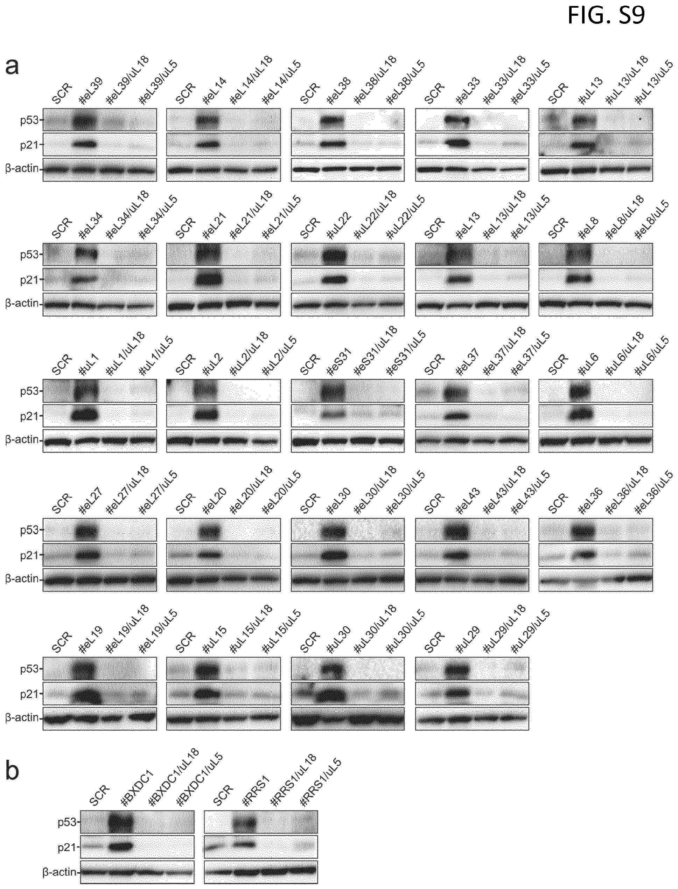

**Supplementary FIG. 8** - not covered by the claims: Efficiency of r-protein depletion established at the mRNA level by RTqPCR. For forty-eight r-proteins whose depletion did not significantly affect p53 accumulation (see Fig. 6b), the residual level of mRNA was established by RTqPCR and found to be below 20% for forty of them, and to range between 20% and 45% for the remaining eight. Total RNA was extracted from HCT116 cells treated for 2 days with an siRNA specific to transcripts encoding the indicated r-proteins. Residual levels of mRNA were established by RTqPCR and normalized to those observed in cells treated with a non-targeting siRNA control (SCR). Each experiment was performed in triplicate. **Supplementary FIG. 9** - not covered by the claims: Depletion of twenty-four r-proteins out of eighty leads to a significant five-fold-increased level of p53, and this increase requires the presence of uL5 and uL18. HCT116 p53+/+ cells were depleted for 2 days with an siRNA specific to transcripts encoding the indicated protein. Each protein was depleted by itself, or in combination with uL18 or uL5 depletion. As control, cells were treated with a non-targeting siRNA (SCR). Total protein was extracted and processed by western blotting with antibodies targeting p53, p21 (a transcriptional target of p53), and, as loading control, β-actin. Bands were revealed by luminescence. (a) Effect of individually depleting each of the 24 r-proteins. (b) Effect of depleting the central protuberance assembly factor BXDC1 or RRS1.

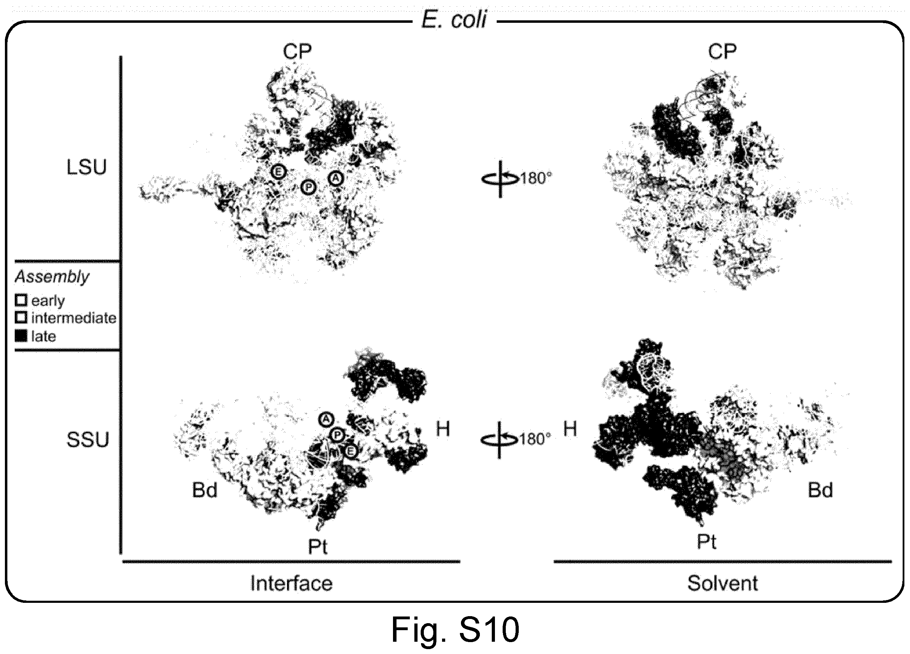

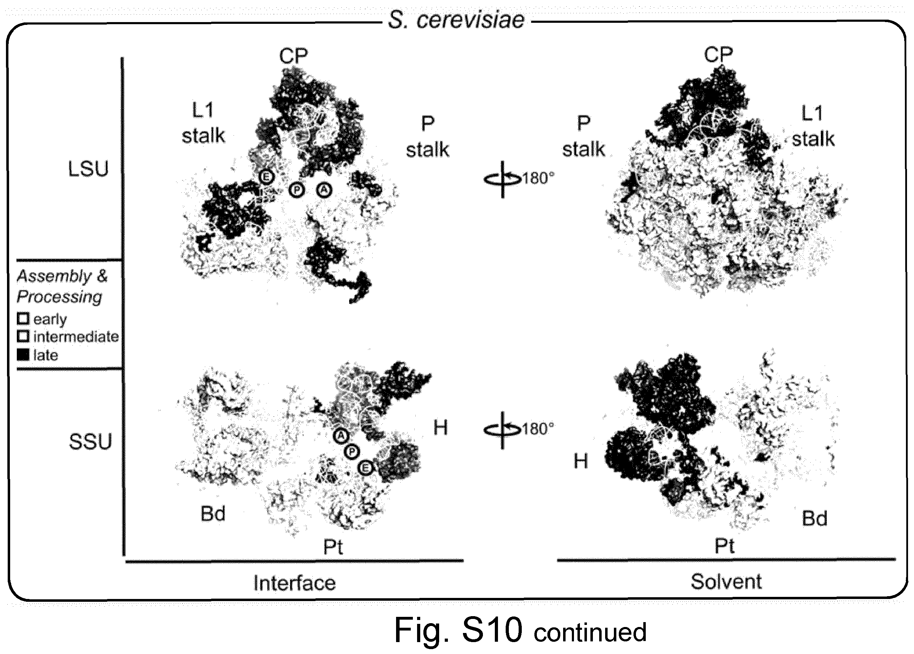

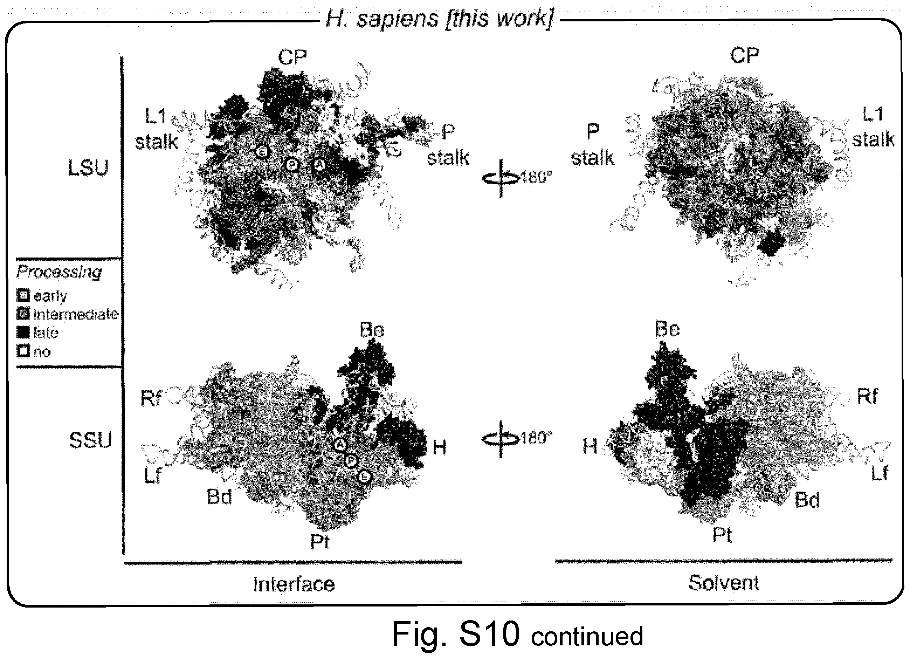

**Supplementary FIG. 10** - not covered by the claims: The sequence of assembly of r-proteins onto assembling ribosomal subunits has been remarkably conserved throughout evolution. (a) 3-D models of bacterial ribosomal subunits based on PDB entries 2AVY and 2AW4. The r-proteins are intensity-coded according to their early, intermediate, or late order of assembly, as established in vitro (refs 4-8). (b) 3-D models of budding yeast ribosomal subunits based on PDB entries 3U5B, 3U5C, 3U5D, and 3U5E. The r-proteins are labeled on the basis of their being required for specific early, intermediate, or late pre-rRNA processing steps in yeast cells (refs 9, 10). This largely corresponds to their kinetics of assembly established in vivo (ref. 11). (c) 3-D models of human ribosomal subunits based on PDB entries 3J3D, 3J3A, 3J3F, and 3J3B. The r-proteins are classified with respect to their impact on specific processing reactions in human cells according to this work (based on Fig. 4, Supplementary Figs. S5,S6,S11). Left, subunit interface views; right, solvent-exposed views. The aminoacyl (A), peptidyl (P), and exit (E) tRNA sites are indicated. Morphological features of the subunits are highlighted. On the LSU: the L1-stalk, central protuberance (CP), and phospho-stalk (P-stalk). On the SSU, the beak (Be), head (H), platform (Pt), body (Bd), left foot (Lf), and right foot (Rf).

**Supplementary FIG. 11** - not covered by the claims: An example of a complete data sheet for one r-protein. The datasheets for all the eighty human r-proteins is available on the companion website at www.ribosomalProteins.com. The data sheet shows the position of the r-protein on mature subunit (A), its impacts on pre-rRNA processing and mature rRNA accumulation (B), on nucleolar structure (C), and on the p53 steady-state level (D).

(A) 3-D models of human ribosomal subunits based on PDB entries 3J3D and 3J3A, for SSU r-proteins, and 3J3F and 3J3B, for LSU r-proteins. The positions of individual r-proteins on the mature subunits are highlighted. On small subunits, the 18S rRNA is shown in gray; on large subunits, the 5S, 5.8S, and 28S rRNAs are shown in red, blue, and gray, respectively. Left, interface view; right, solvent view. The main ribosomal features are indicated (see Supplementary Fig. 10). (B) Effects of r-protein depletion on pre-rRNA processing and mature rRNA accumulation:

northern blots and ethidium-bromide-stained denaturing agarose gels showing all the pre-rRNA intermediates and mature rRNAs detected. The 28S/18S rRNA ratio was calculated from electropherograms. A calibration set (described in Supplementary Figs S5,S6) is included for reference. Schematics representing the RNAs detected are shown to the left. Quantifications are available in Fig 4. and Supplementary Figs S5,S6. (C) Effect of r-protein depletion on nucleolar structure: representative microscopic images (blue signal, DAPI; green signal, fibrillarin) obtained after treatment in duplicate screens (i and ii) performed with three different siRNAs (#1, #2, and #3). iNo values for each screen are indicated to the right and on a scaled bar at the bottom. The iNo value ranges between 0 (unperturbed nucleolus) and 0.2 (severely disrupted structure).

(D) Effect of r-protein depletion on the p53 steady-state level: fluorescent quantitative western blotting was performed in triplicate (i, ii, iii). The p53 signal was corrected for loading, using □-actin detection as a reference, and expressed with respect to the signal obtained in cells treated with a non-targeting (Scr) control (lane 2). The p53 signal was expressed as a mean of three independent experiments (see Fig. 6 for details). A calibration set (described in Fig. 6) is included for reference.

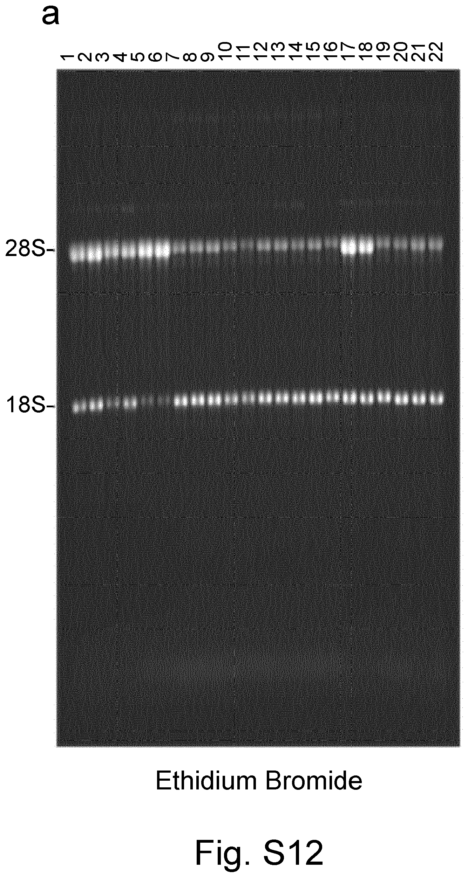

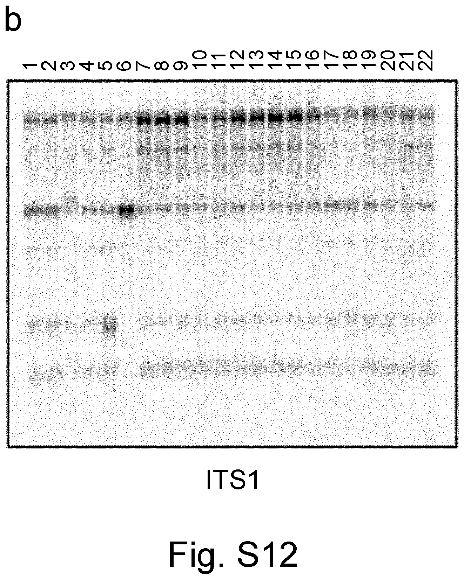

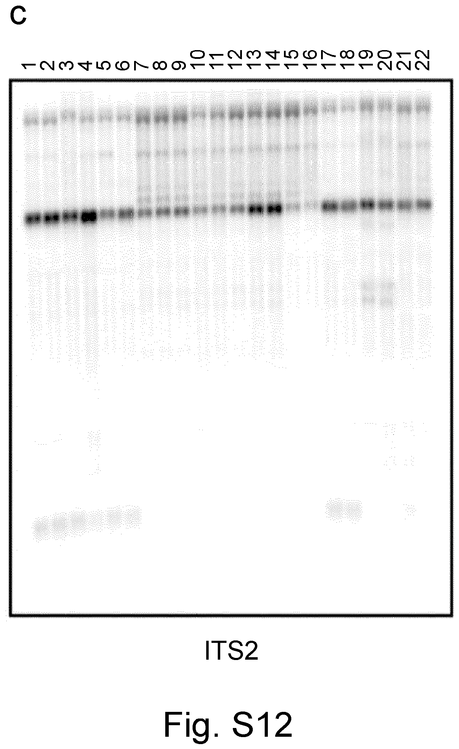

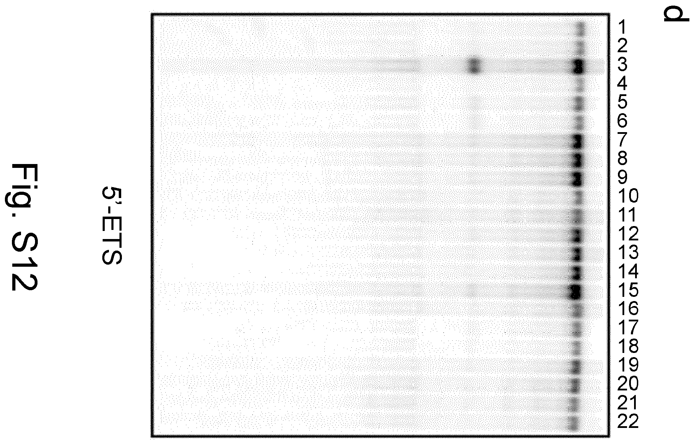

**Supplementary FIG. 12** - not covered by the claims: Examples of uncropped Northern blots. An example of a high resolution denaturing agarose gel is shown. (a) Ethidium bromide staining reveals the mature 18S and 28S rRNAs. (b,c,d) Northern blotting with specific probes reveals the pre-rRNAs. All RNA species were identified by differential hybridization with specific probes, and by reference to a calibration set consisting of two proteins (UTP18 and NOL9) whose depletion leads to well-characterized pre-rRNA processing inhibitions, as described in ref. 1. The probes used (ITS1 in b, ITS2 in c, and 5'-ETS in d) are described in Example 1. Lane 1, mock; lane 2, SCR; lane 3, UTP18; lane 4, NOL9; lane 5, uS3; lane 6, uS17; lane 7, uL30#1; lane 8, uL30#2; lane 9, uL13#1; lane 10, uL13#2; lane 11, eL14#1; lane 12, eL14#2; lane 13, u122#1; lane 14, u122#2; lane 15, eL18#1; lane 16, eL18#2; lane 17, eL22#1; lane 18, eL22#2; lane 19, uL14#1; lane 20, uL14#2; lane 21, uL24#1; lane 22, uL24#2. #1 and #2 refer to the siRNAs used (see Example 1).

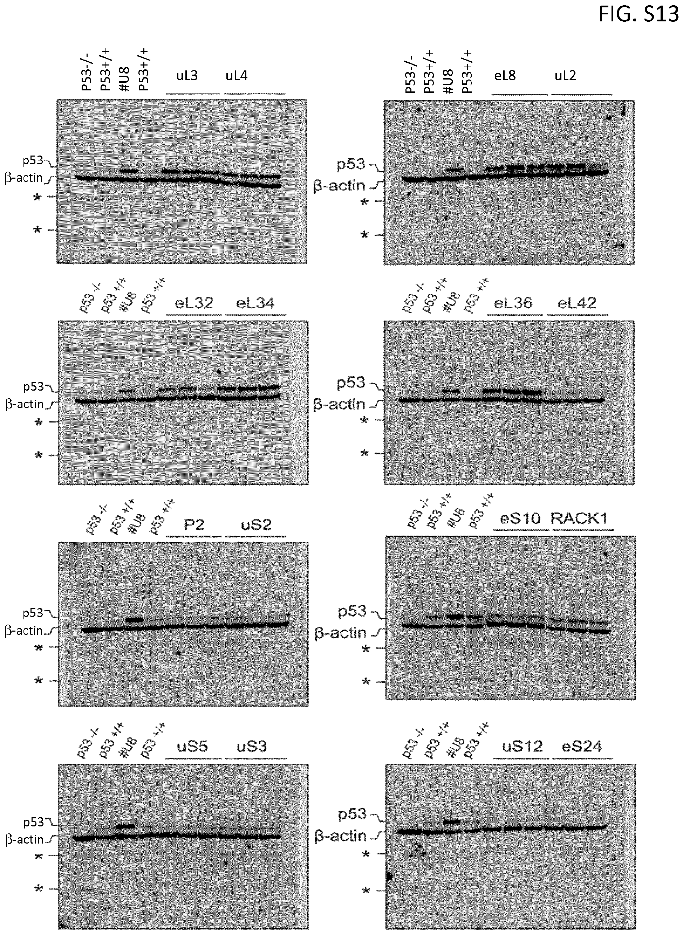

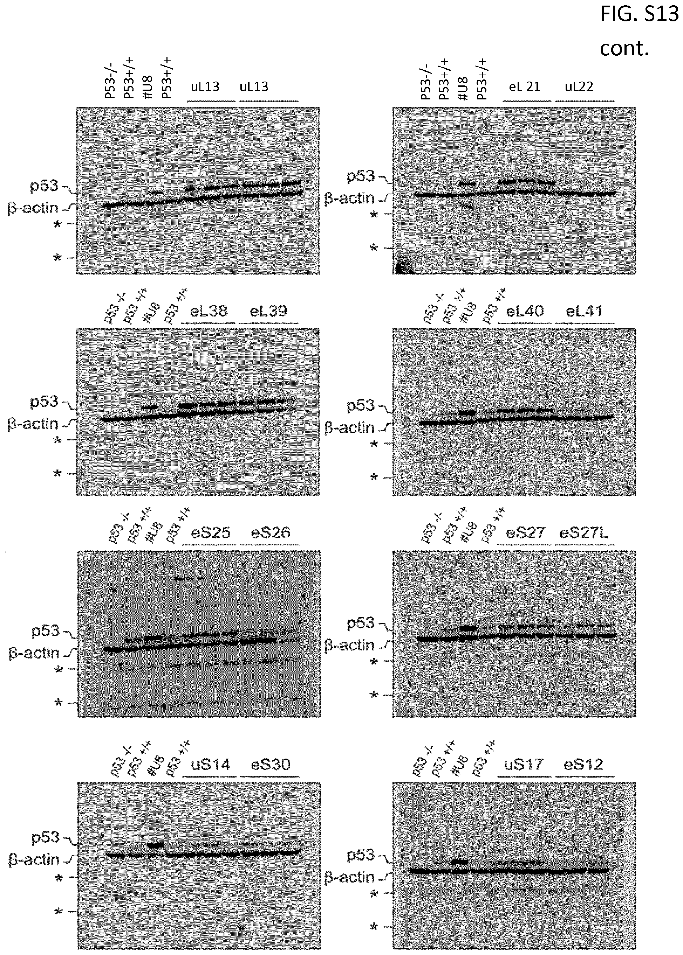

**Supplementary FIG. 13** - not covered by the claims: Examples of uncropped Western blots. All western blot hybridizations performed in this work used well-characterized commercially available antibodies (see Example 1). On fluorescent (a) and luminescent (b) detections, p53 migrated between the 35 kDa and the 55 kDa molecular weight bands, as expected (p53 has a molecular weight of 43.7 kDa). The p53 signal was increased upon nucleolar stress activation (U8 depletion in a, and uL1 depletion in b, by comparison to the signals observed in cells treated with a non-targeting Scr control). The p53 level was severely reduced upon codepletion of uL1 with uL5 or uL18 (b). On the fluorescent screening gels (representative examples shown in c), the β-actin loading control (bottom rows) was detected as a single band immediately below the p53 signal, as expected (β-actin has a molecular weight of 42 kDa). The p53 band (top row) was consistently detected at low levels in the duplicated HCT116 p53 +/+ cells treated with a non-targeting siRNA (p53 +/+ lanes), this served as a baseline control. The p53 levels increased substantially upon nucleolar stress caused by U8 snoRNA depletion (#U8 lanes), this served as a positive control. The p53 band was never detected in the negative control provided by the HCT116 p53 -/- isogenic cell line (p53 -/-). The stars denote nonspecific bands.

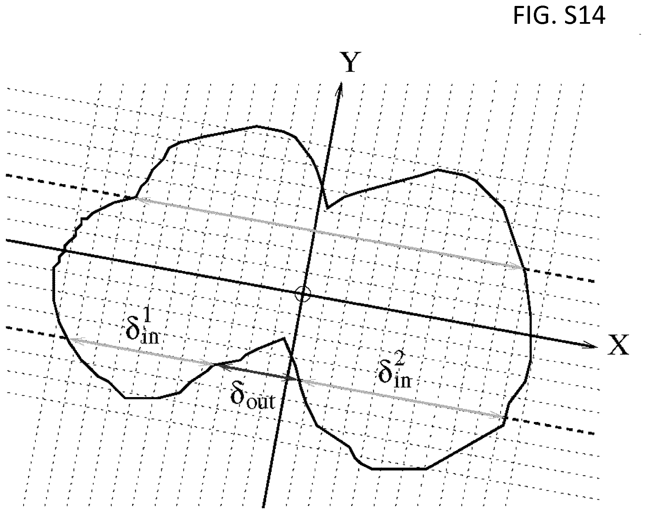

**Supplementary FIG. 14**: Nuclei aggregates are eliminated on the basis of a shape convexity analysis. Two segmented juxtaposed nuclei are illustrated. In the direction of the principal axis X and Y, if δout, δ1in, and δ2in are above a threshold on one of the lines parallel to the principal axis, the shape is considered to include more than one nucleus.

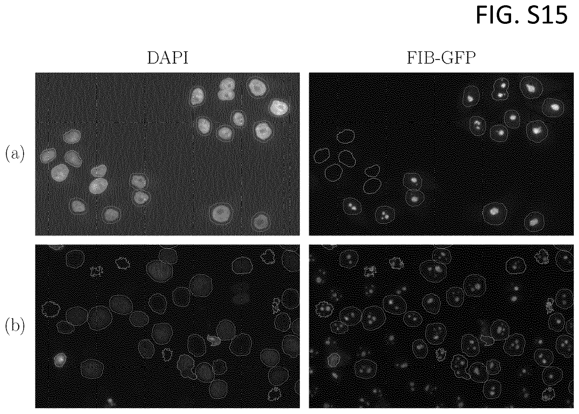

**Supplementary FIG. 15**: Results of segmentation of nuclei for highly contrasted (a) and weakly contrasted (b) DAPI images. The connected components are detected based on the proposed hierarchical thresholding. Irregular regions are rejected because of their concavity. Some regions are rejected because the DAPI/GFP intensity analysis indicates that cells are probably not in interphase. Only remaining nuclei circled are maintained in our analysis, and for the subsequent analysis, we consider the segmentation, obtained after morphological operations: 13x13 dilation, followed by 3x3 erosion.

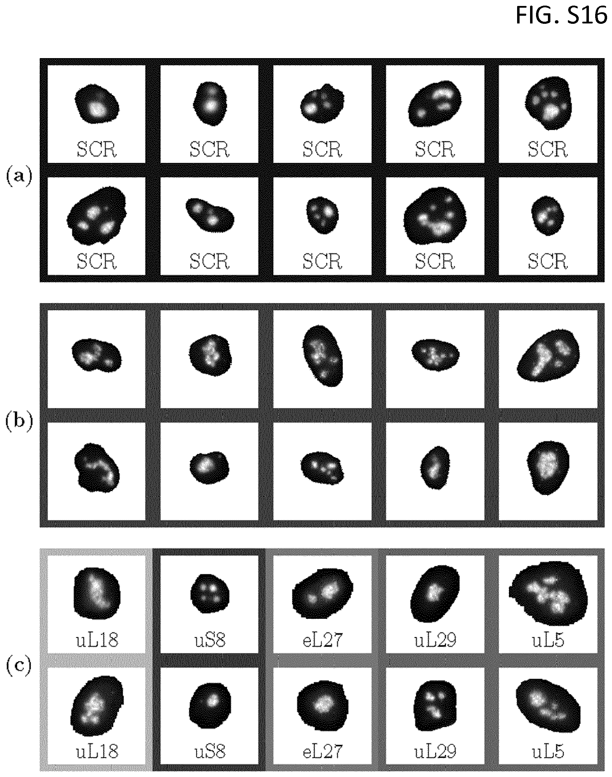

**Supplementary FIG. 16**: Samples of nucleoli from SCR-treated control cells (a), from cells with a range of high level of nucleolar disruption (b) and from cells depleted of a specific protein of interest (c). Cell nuclei in each column were treated with the same siRNA. Images were normalized by percentile 99.9%.

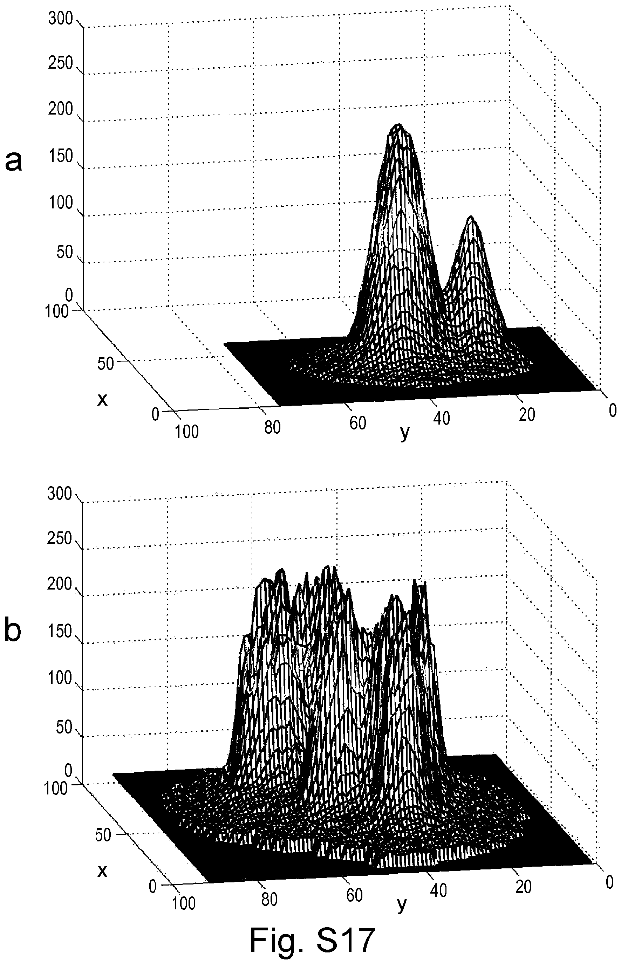

**Supplementary FIG. 17**: Fibrillarin-GFP intensity profile of nucleoli in a SCR-treated control cell (a) or in a cell depleted for the protein uL5 (b). (a) corresponds to the nucleus in row 1, column 2 in Supplementary Fig. 16, panel a. (b) corresponds to the nucleus in row 1, column 5 in Supplementary Fig. 16, panel c.

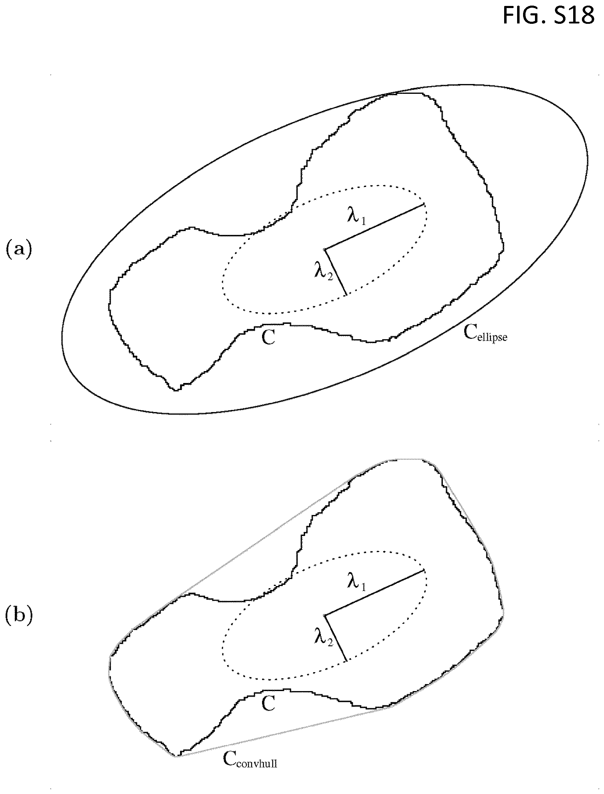

Supplementary FIG. 18: Illustration of the shape factors associated to a connected component C (inner shape black contour). (a) The elongation factor reflects the ratio between the principal axes second order moments, while the elliptical regularity measures the area ratio of the smallest external ellipsoid $C_{ellipse}$ (outer shape in grey contour) to the connected component. (b) The concavity ratio measures the area ratio between the convex hull $C_{convhull}$ (outer shape in grey contour) and the connected component.

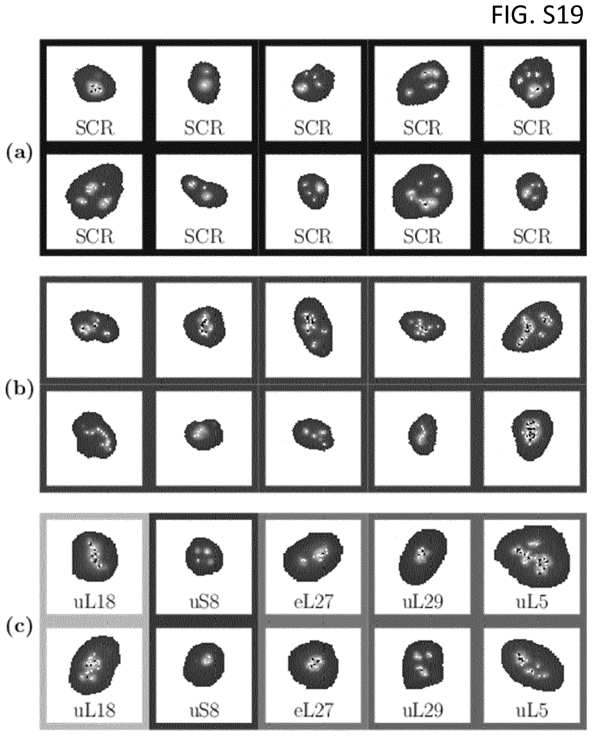

**Supplementary FIG. 19**: Distribution of local maxima (white dots) and local minima (black dots) in the images shown in Supplementary Fig. 16. It is apparent that the density of local minima is higher in nucleoli from cells with high level of nucleolar disruption (panel b) or from cells depleted of a protein of interest (panel c) than in nucleoli from SCR-treated control cells (panel a).

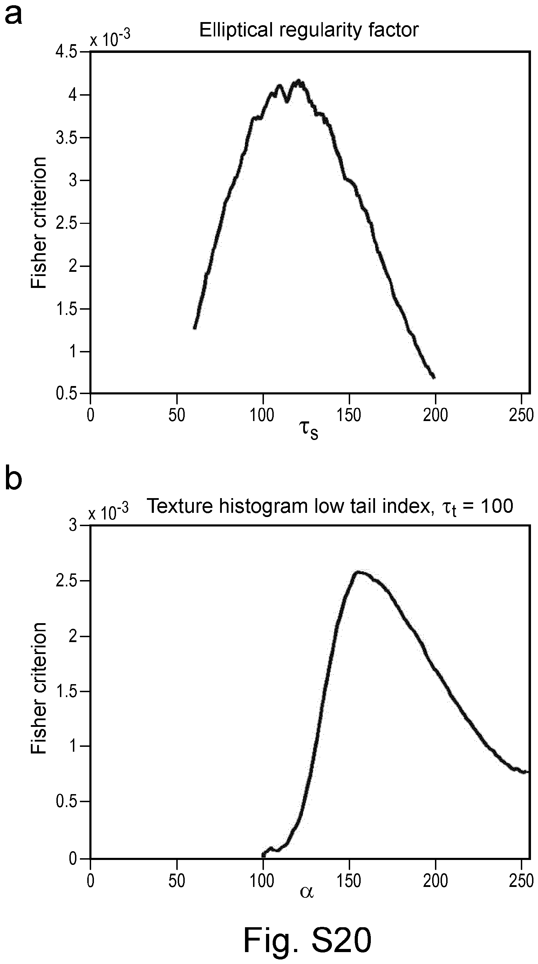

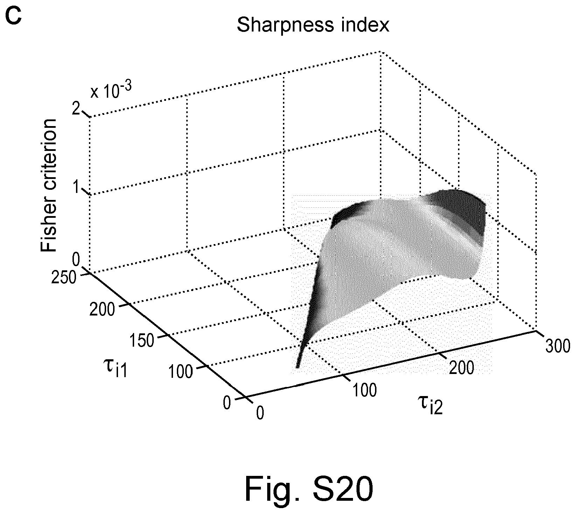

**Supplementary FIG. 20**: Fisher's optimization criterion as a function of three features (a, b, and c) parameters.

**Detailed description**

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of". Whereas the terms "one or more" or "at least one", such as one or more or at least one member(s) of a group of members, is clear *per se*, by means of further exemplification, the term encompasses *inter alia* a reference to any one of said members, or to any two or more of said members, such as, *e.g.*, any $\geq 3$, $\geq 4$, $\geq 5$, $\geq 6$ or $\geq 7$ etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In an aspect, the invention relates to a computer implemented method for scoring nucleolar disruption , comprising determining morphometric characteristics of the nucleolar support area and the nucleolar intensity pattern;

wherein nucleolar disruption is scored based on a nuclear stain image of eukaryotic cell or tissue images, preferably based on a nuclear stain image of population of cells;wherein said morphometric characteristics are

- the nucleolus area of the largest nucleolus or largest nucleolar connected component in a cell,
- the elliptical regularity factor, wherein the elliptical regularity factor is determined as the ratio between the area of the largest nucleolus or nucleolar connected component and the area of the smallest ellipse lying outside the largest nucleolus or nucleolar connected component and having the same center, the same principal axes, and the same elongation as the largest nucleolus or nucleolar connected component,
- the texture histogram low tail index, wherein the texture histogram low tail index is defined as the percentage of pixels that lie below a pixel intensity threshold $\alpha$ of the largest nucleolus or largest nucleolar connected component in a cell,
- the texture valleys index, wherein the texture valleys index is defined as the number of local minima in the nucleolus or nucleolar connected component of the largest nucleolus or largest nucleolar connected component in a cell, and
- the texture local minimum, wherein the texture local minimum is defined as the intensity of the smallest local minimum in the nucleolus or nucleolar connected component of the largest nucleolus or largest nucleolar connected component in a cell;

wherein the nucleolus is identified based on image pixel intensity thresholding as a connected component in the thresholded nuclear stain image;
wherein the nucleolar support area is determined based on an area segmentation threshold, wherein the nucleolar shape regularity is determined based on a shape segmentation threshold, and the nucleolar texture pattern is determined based on a texture segmentation threshold, each threshold being defined to maximize the discriminant power of corresponding morphometric characteristics;
wherein the nucleolar disruption is scored based on the discrepancy with respect to a standard or reference nucleolus wherein said discrepancy is determined as a discrepancy vector based on the distances between the distributions of each morphometric characteristic of a population of said nucleoli or nucleolar connected components and the distribution of the corresponding morphometric characteristic of a population of standard or reference nucleoli or nucleolar connected components, wherein the discrepancy vector component associated with a given morphometric characteristic (i) for a particular nucleolus or nucleolar connected component population d is preferably determined according to the following formula representing a discrepancy vector component:

$$d(i) = \frac{\mu(i) - \mu_r(i)}{\sqrt{\sigma^2(i) + \sigma_r^2(i)}}$$

wherein $\mu(i)$ and $\mu(i)$ are the mean of morphometric characteristic (i) of said nucleoli or nucleolar connected components and said standard or reference nucleoli or nucleolar connected components, respectively; and wherein $\sigma(i)$ and $\sigma_r(i)$ are the standard deviation of morphometric characteristic (i) of said nucleoli or nucleolar connected components and said standard or reference nucleoli or nucleolar connected components, respectively; wherein nucleolar disruption is scored based on the index of nucleolar disruption (iNo) which is derived from an increasing function of the absolute value of the morphometric characteristic discrepancy vector components, preferably as the L1-norm of the discrepancy vector, wherein said iNo is determined according to the following formula:

$$iNo = \sum_{}^{N} (a(i) * |d(i)|)$$

wherein N is equal to 5 and is the number of said morphometric characteristics; wherein a(i) is a weighing factor for the $i^{th}$ morphometric characteristic, wherein preferably $0 < a(i) < 1$; and wherein d(i) is the discrepancy vector component for the $i^{th}$ morphometric characteristic; wherein the iNo value is the score and is indicative of the severity of said nucleolar disruption.

Also described herein, but not encompassed by the wording of the claims, is a method for characterizing nucleolar morphology, architecture, or integrity, comprising measuring or determining one or more morphometric characteristics of the nucleolar support area and the nucleolar intensity pattern.

Each population of cells may be described by the stochastic distributions of a set of discriminant nucleolar features (i.e. morphometric characteristics). A nucleolar feature is a scalar value that reflects one particular aspect of the spatial organization of nucleolar masses within one individual cell nucleus.

A feature discriminates between two populations of cells when the statistical distribution of the feature values measured over the two populations have a limited overlap, as for example measured by a large difference of averages compared to their mean standard deviation.

Also described herein, but not encompassed by the wording of the claims, is a set of nucleolar features that discriminate between two types of cell populations may be derived in the following steps:

1) A set of arbitrary parametric features is defined, e.g. by an expert in the field, to capture the main salient differences observed between representative nucleoli samples of each population;
2) The parameters associated to each feature are then selected to minimize the overlap between pairs of exemplars of the populations to discriminate;
3) Only the features, and their associated parameters, leading to a large separation between populations are kept as discriminant features.

In an example not encompassed by the wording of the claims, such method comprises the steps of:

a) defining a set of arbitrary parametric morphometric characteristics to capture the most salient morphometric differences observed between representative nucleoli samples of two populations of interest;
b) selecting the parameters associated to each morphometric characteristic to maximize their discriminating power and/or to minimize the overlap between the morphometric characteristic probabilistic distributions derived from each population, preferably according to Fisher's criterium; and
c) selecting the morphometric characteristics and their associated optimized parameters leading to a large separation between populations to discriminate;
d) computing the distance between the two cells or cell populations based on a combination, e.g. using a weighted summation, of the distances measured in terms of the selected morphometric characteristics.

Also described herein, but not encompassed by the wording of the claims, is the use of the above method for identifying features that discriminate between two types of cell populations, such as identifying features that discriminate between two types of cell populations with different nucleolar morphology or architecture. In certain examples, the combination results in the (weighed) sum of the absolute value of a normalized distance between the feature distributions (e.g. the Fisher score) associated to the population of cells we want to compare. Other features and combinations are possible.

As used herein, the terms "morphology" and "architecture" may generally refer to the overall appearance of nucleoli, such as for instance identified on images of cells or tissues. "nucleolar integrity" and the related "nucleolar disruption" may generally refer to the similarity or dissimilarity of the nucleolar morphology or architecture in a sample cell (population) or tissue compared to a reference nucleolar morphology or architecture. Preferably, such reference nucleolar morphology or architecture is a "normal" nucleolar morphology or architecture, e.g. the nucleolar morphology or architecture of non-diseased cells, or cells known not to have an abnormal nucleolar morphology or architecture (and as such may also include diseased cells, but which do not have an abnormal nucleolar morphology or architecture). In certain embodiments, a standard or reference nucleolar morphology may be cell type specifically determined (e.g. for neuronal cells, epithelial cells, etc.). As used herein, the term "morphometric characteristic" may generally refer to any feature capable of describing the visual appearance, including morphology or form (encompassing size and shape) and inner pattern structure (encompassing spatial organization and variation), and which is measurable and quantifiable.

The term "nucleolar support area" may generally refer to the physical location of the nucleolus, or the area enclosing a nucleolus, e.g. on an image, as defined herein elsewhere. The term "nucleolar intensity pattern" may generally refer to specific intensity-associated morphometric features and their distributions, as described herein elsewhere.

According to the invention, the morphometric characteristics are determined on images of cells, cell populations, or tissues. In certain embodiments, the morphometric characteristics are determined on images of tissue sections. Cells or tissues may be prepared by techniques known in the art, and which allow cell or tissue imaging. In certain embodiments, the images are or include fluorescent components, e.g. DAPI, for nuclear staining, or fluorescent labels (e.g. antibodies) for specific cell structures or molecules (e.g. nucleolus-specific components, structures, or molecules). The present invention is however not limited to the use of fluorescent imaging. The skilled person can readily envisage alternative imaging methods and techniques. Also in vivo imaging methods are possible in certain embodiments according to the invention (see for instance US 2015/157254).

Also described herein, but not encompassed by the wording of the claims, is a method comprising the steps:

a) obtaining or providing an image of one or more eukaryotic cell, or eukaryotic tissue, or preferably of a population of cells ;
b) optionally segmenting said image thereby obtaining individual cells or cell nuclei;
c) segmenting said image, thereby obtaining one or more nucleolar masses, preferably comprising a plurality of connected pixels, which connected pixels constitute or represent a nucleolar connected component;
d) optionally assigning by colocalization nucleolar masses to individual cells or cell nuclei and thus to individual cells;
e) extracting one or more feature (i.e. morphometric characteristic) characterizing the support area, and the intensity pattern of connected components;
f) computing probabilistic distributions of said features (e.g. for cell populations); and
g) comparing said probabilistic distributions with a reference model distribution and/or determining the overlap of said probabilistic distributions to a reference model distribution.

In the above method, b) and/or d) may both be optional. The method above may thus comprise b); b) and d); d); or none of b) and d).

As used herein, the term "connected pixels" refers to adjacent or neighbouring pixels on a graph or image, i.e. pixels which are next to each other (in any direction). According to the invention, the set of connected pixels (and hence by extension the segment or component or connected segment or component) are or comprise or consist of adjacent pixels each having an intensity above a given threshold. Each pixel is a node, and the graph or image edges are directly derived from the definition of a neighborhood system, which typically assigns 4 (top, down, left, right) or 8 neighbors (4 + oblique directions) to each pixel. A connected component (or just component) of an undirected graph or image is a subgraph or subimage in which any two vertices are connected to each other by paths, and which is connected to no additional vertices in the supergraph or superimage. A vertex with no incident edges is itself a connected component. Hence, a nucleolar connected component is a set of nucleolar pixels in which any two pixels can be connected through a path (a sequence of pixel adjacency relationships) that only include nucleolar pixels. According to the invention, as an initial step to delimit cell nucleoli from individual cells, the cell nucleus is segmented. Each cell has a single nucleus. The nucleoli are specialized subnuclear domains, which, by definition, are all contained within the nucleus. Segmenting the nucleus, which is dense, compact and easy to score, is thus a mean to delimit the cellular volume that contains nucleoli in each individual cell.

Nuclei may in certain embodiments be stained with a DNA stain, such as for example, and without limitation with DAPI. The nuclei contours may in certain embodiments be extracted from the DAPI channel (or other stain or means of visualization of the nucleus) in two steps. Firstly (step 1), large connected components of relatively high intensity are identified using a thresholding method, such as for instance an adaptive thresholding method as described in Example 2. Optionally secondly (step 2), the connected components that show significant concavity, indicating they likely correspond to aggregated nuclei, may be rejected in certain embodiments.

Step 1 may in certain embodiments consist in a stepwise thresholding of the DAPI images (or other stain or means of visualization of the nucleus) such that nuclei corresponding to sufficiently large connected components of pixels lying above an intensity threshold are selected. To address stochastic variation, in certain embodiments, a hierarchical thresholding strategy may be adopted that progressively refines the segmentation by considering a sequence of increasing thresholds, while exploiting prior knowledge about the size range of human cell nuclei.

In step 2, the rejection of the connected components that likely correspond to multiple nuclei is considered. For this, in certain embodiments, the convexity of each connected component is analyzed. By means of example, and without limitation, in certain embodiments, a number of lines are drawn in parallel to the two principal axis of the connected component. When the connected component is convex, either one or zero segment lies inside the contour, for all parallel lines. In contrast, when the connected component represents aggregated nuclei, it presents a strong concavity and there exist parallel lines that include two or more segments lying inside the contour. If one parallel line supports two sufficiently long inner segments that are separated by a sufficiently long outer segment, the connected component is rejected. In certain embodiments, a threshold, such as a threshold of a particular number of pixels is set based on which is determined the length of inner and outer segments in order to include or exclude particular components. Those lengths in certain embodiments have to be larger than a threshold of for instance at least 1, 2, 3, 4, or preferably at least 5 pixels to reject the component. The threshold value may in certain embodiments be set empirically to drastically reduce the number of multiple nuclei while keeping most of the single nuclei, compared to a manually generated ground truth.

In certain embodiments, only cells in interphase are analyzed. To identify them, in certain embodiments, the DAPI image (or other stain or means of visualization of the nucleus) and/or the distribution of nucleolus-specific stain (or other means of visualization of the nucleolus) in the segmented component are considered. A cell in certain embodiments is considered to be in interphase if the DAPI (or other stain or means of visualization of the nucleus) is sufficiently dense and spread (if for example at least 50% of the pixels in the nucleus have a normalized DAPI value larger than 0.3, preferably larger than 0.4, more preferably larger than 0.47, with the DAPI image (or other stain or means of visualization of the nucleus) being normalized by its maximal value), and/or if the nucleolus specific stain (or other means of visualization of the nucleolus) is sufficiently localized (if at least 50% of the pixels in the nucleus have a normalized nucleolus specific stain (or other means of visualization of the nucleolus) value lower than 0.7, preferably lower than 0.6, more preferably lower than 0.53, with the nucleolus specific stain (or other means of visualization of the nucleolus) image being normalized by its maximal value). The thresholds in certain embodiments are set on the basis of a manual ground truth annotation of the images.

Finally, as a post-processing step, in certain embodiments, to ascertain all nucleoli of a cell are contained in each segmented regions, a basic mathematical morphology to close and enlarge each connected regions is applied. In certain embodiments, a dilation by a 13 x 13 structuring element is followed by a 3 x 3 erosion. In certain embodiments, the morphometric characteristics as defined herein are independent of the actual position of the nucleoli within the nucleus. In certain embodiments, the morphometric characteristics as defined herein while (still) reflect the spatial spreading of the nucleolar masses. In certain embodiments, those characteristics are defined based on a manually-selected set of image samples, depicting typical normal and abnormal nucleolus patterns. The manual extraction of representative samples in certain embodiments allows to derive features that are relevant to make sure that the set of investigated features are able to discriminate among the variety of nucleolus appearances. According to the invention, each characteristic is systematically defined as a parametric function (advantageously so that its parameters can be optimized over the entire database to make the feature can differentiate between normal and gene-depleted cells images).

In certain embodiments a set of manually-selected cells nucleoli images that are representative of the appearance diversity in reference control cells is selected (cf. Supplementary Fig. 16). In addition, or in the alternative, in certain embodiments, a 2-D or 3-D graph representing nucleolar intensity may be use to visualize nucleolar appearance diversity.

According to the invention, for each nucleus, all morphometric characteristics are defined with respect to the segmentation of the nucleolus masses into a set of disjoint connected components. According to the invention, this segmentation is obtained by nucleolar image thresholding, which means that a pixel is considered to be part of the nucleolus if its intensity lies above the threshold (advantageously obtained with nucleolar specific staining). In certain embodiments, for each characteristic, the segmentation threshold parameter is defined automatically so as to maximize the separation between the distributions of the characteristics for control cells and for test cells, such as cells having disrupted nucleolar morphology or architecture. In certain embodiments, the segmentation threshold is a morphometric characteristic parameter, and might vary from one morphometric characteristic to the other.

According to the invention, the area of support of the nucleolus is characterized. In certain embodiments, the size and number of connected components obtained after thresholding with a so-called area segmentation threshold

$\tau_a$. is considered or determined. According to the invention, AAlcc measures the area of the largest connected component in the thresholded image. Described herein, but not encompassed by the wording of the claims, ANcc denotes the number of connected components in the nucleus. Described herein, but not encompassed by the wording of the claims, the sharpness index AS characterizes the sharpness of the intensity gradient along the frontier delimiting the nucleolar masses, and measures the ratio of the nucleus pixels that respectively lie above two thresholds $\tau_{i1}$ and $\tau_{i2}$, with $\tau_{i1} > \tau_{i2}$.

According to the invention, to characterize the shape and the texture of the nucleolus, only the largest connected component obtained after segmentation is considered.

In certain embodiments, to quantify the nucleolus shape regularity, a shape segmentation threshold $\tau_s$ is adopted. According to the invention, the shape of the largest connected component in the segmented image is characterized. In certain embodiments, each characteristic describes the shape independently of its size. Described herein, but not encompassed by the wording of the claims, the elongation shape factor SElcc, is defined as the square root of the ratio of the two second order moments, $\lambda 1$ and $\lambda 2$, of the connected component c around its principal axes. According to the invention, the elliptical regularity factor SRlcc, is defined as the ratio between the area of the connected component, and the area of the smallest ellipse lying outside the connected component, and having the same center, the same principal axes, and the same elongation than the connected component. Described herein, but not encompassed by the wording of the claims, the concavity factor SClcc, is defined as the ratio between the area of the connected component and the area of its convex hull.

According to the invention, to characterize the nucleolar texture pattern scalar metrics to reflect the distribution of intensities inside the largest connected component is considered. In certain embodiments, the distribution of pixel intensities is determined, preferably following cell-wise dynamic range normalization. In certain embodiments, the connected component is segmented based on a texture segmentation threshold $\tau_t$. According to the invention, the texture histogram low tail index THlcc measures the percentage of pixels that lie below some intensity threshold $\alpha$, preferably while being located inside the erosion of the connected component by a 3 x 3 pixels structuring element. An erosion may in certain embodiments be applied to the shape to get rid of the low intensity pixels lying on the border of the shape. Described herein, but not encompassed by the wording of the claims TUlcc is defined to be the number of connected regions lying above a threshold $\beta$, while being inside the connected component. Described herein, but not encompassed by the wording of the claims, the texture peaks index TPlcc is defined to be the number of local maxima in the connected component. According to the invention, the texture valleys index TVlcc is defined to be the number of local minima in the connected component. According to the invention, the texture local minimum TLMlcc is defined as the intensity of the smallest local minimum in the connected component.

In certain embodiments, selection of the morphometric characteristics is based on the Fisher's criterion introduced by the popular Linear Discriminant Analysis (22). In certain embodiments, those parameters or characteristics are selected so as to maximize the separation between the features distributions that we want to discriminate (see also Example 2). The skilled person will understand that alternative selection criteria may be applied, and which equally maximize the separation between the features to be discriminated. In certain embodiments, a discriminant feature or characteristic is one for which the class-means are well separated, measured relative to the (sum of the) variances of the data assigned to a particular class.

In certain embodiments, considering a feature, parameterized by a vector p lying in a parameter space P, it is assumed that the feature distributions are known as a function of p, for the two classes of observations that we want to best discriminate. The Fishers' optimization criterion indicates that the vector p* that maximizes the separation between the class distributions is defined as:

$$p^* = \underset{p \in P}{\arg\max} \frac{[\mu_1(p) - \mu_2(p)]^2}{\sigma_1(p)^2 + \sigma_2(p)^2} \ ,$$

where $\mu 1(p)$, $\mu 2(p)$ and $\sigma 1(p)$, $\sigma 2(p)$ respectively denote the means and standard deviations of the distributions of the feature of interest, measured with parameter p for the two classes. This Fisher's criterion is equivalent to the Welch's adaptation of the t-test (23), widely used in image-based morphometry (24).

In certain embodiments, the parameters/characteristics are selected so as to maximize the sum of the separation measured between each pair of distributions extracted from control cells, and from test cells, such as cells having a disrupted nucleolar morphology.

According to the invention, a discrepancy vector is defined so as to summarize how the morphometric characteristic distributions associated to a set of nucleoli differ from their corresponding reference distributions. In certain embodiments, for a given image feature, the discrepancy d is defined between a test set and a reference set, preferably to be the ratio of the difference of the mean feature values on each set to the sum of their variance, preferably according to the following formula:

$$d(i) = \frac{\mu(i) - \mu_r(i)}{\sqrt{\sigma^2(i) + \sigma_r^2(i)}}$$

with $\mu(i)$, $\mu r(i)$ and $\sigma(i)$, $\sigma r(i)$ denoting the means and standard deviations of the characteristic/feature; with d preferably being a discrepancy vector component.

According to the invention, to quantify the nucleolar disruption level, an index of nucleolar disruption, or iNo is defined according to the following formula:

$$iNo = \sum_{i=1}^{N} a(i) * |d_k(i)|$$

wherein N is the number of features; wherein a(i) is a weighing factor for the ith feature, wherein preferably 0 < a(i) < 1; and wherein d(i) is the discrepancy vector component for the ith feature. In certain embodiments, the iNo is the L1-norm of the discrepancy vector (i.e. all a(i) are equal).

In certain embodiments, a lower dimensional representation of the data is obtained. In certain embodiments, such representation may be obtained by Principal components analysis (PCA). In certain embodiments, the first two principal components are considered. In certain embodiments, the PCA is applied to the discrepancy vectors that capture the average trends associated with nucleolar morphology, architecture, or integrity. In certain embodiments, the 5 features that have the largest Fisher's score, i.e. which best discriminates normal and altered nucleoli are considered. According to the invention, those features are as listed in the first column of Table 7.

According to the invention, the methods as described herein are computer implemented methods, in particular computer implemented methods for scoring nucleolar disruption according to the invention as described herein. Accordingly, in certain embodiments, the invention relates to a method according to the invention as described herein executed on a computer. In related aspects, the invention provides a computer-readable medium comprising computer-readable instructions which, when loaded on the internal memory of a computer, cause the computer to execute a method according to the invention as described herein. In a further aspect, the invention relates to a computer configured for executing a method according to the invention as described herein. In a further aspect, the invention relates to a data-carrier comprising computer-readable instructions which, when loaded on the internal memory of a computer, cause the computer to execute a method according to the invention as described herein.

Example 1 - not covered by the claims: Materials and methods

**Nucleolar screens**

The nucleolar screens were performed on an automated high throughput platform. For each r-protein, three different siRNAs were used, and for each siRNA, 2,000 cells imaged. For consistency, the entire screen was duplicated. The efficiency of siRNA-mediated depletion was assessed in a random shotgun RTqPCR assay. A calibration set consisting of four control proteins whose depletion, we established, affects strongly nucleolar structure was used (Supplementary Fig. 1).

**Cell lines**

The cell lines used in this study are listed in Table 1. All cell lines were cultured at 37°C under 5% CO2. Culture media were supplemented with 10% fetal bovine serum (FBS; Sigma) and 1% penicillin-streptomycin (Pen-Strep, Gibco). For consistency, the experiments were performed on cells grown for 10 to 15 passages. The nucleolar screens were conducted in cervical cancer (HeLa) cells stably expressing FBL in fusion with GFP (FIB364). All cell lines were purchased from the ATCC repository and regularly tested for contamination with the LookOut mycoplasma PCR detection kit (Sigma-Aldrich, MP0035).

Table 1

| Name | Origin | Growth medium |
|---|---|---|
| HeLa-GFP-FBL | Epithelial (cervix) | DMEM / FBS / Pen-Strep |
| HeLa | Epithelial (cervix) | DMEM / FBS / Pen-Strep |

(continued)

| Name | Origin | Growth medium |
|---|---|---|
| HCT116 *p53*+/+ | Epithelial (colon) | McCoy / FBS / Pen-Strep |
| HCT116 *p53*-/- | Epithelial (colon) | McCoy / FBS / Pen-Strep |
| A549 | Epithelial (lung) | F12 / FBS / Pen-Strep |
| H 1944 | Epithelial (lung) | RPMI-1640 / FBS / Pen-Strep |

**siRNA depletion**

The FIB364 cell line was transfected with either of 3 distinct siRNAs targeting each r-protein according to the protocol described below (Table 2 for siRNA sequences). The entire screening procedure was duplicated. Depletions were performed in 96-well plates (Porvair Sciences). A transfection reagent mix (0.125 $\mu$l of Interferin and 20 $\mu$l of Optimem) was added to each plate well and left to set for 10 min at RT. siRNA (10 $\mu$l of 100 nM stock) were added to this mix and left to set for another 30 min at RT. Cells (70 $\mu$l of 100,000 cells/ml) were added to each well and the plates were incubated for 3 days. For each individual plate, a set of 7 wells was used for negative and positive controls. Our calibration set consists of mock-treated cell (cells with the transfection reagent mix only) and cells treated with a non-targeting siRNA (Scramble, SCR), or with siRNA specific to GFP, fibrillarin (FBL), nucleophosmin (NPM), nucleolin (NCL), or TIF1A. Cells were fixed in 2% formaldehyde, washed in PBS, incubated 10 min in the presence of DAPI (1:20,000 of 5 mg/ml in PBS, Sigma), washed again and stored in PBS before imaging. The depletion of the central protuberance assembly factors RRS1 and BXDC1 were performed according to the same protocol.

Table 2

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| *RpSA* | uS2 | XM 939738 | #1 | s60434 | CUCCUGGAACC UUCACUAAtt | Ambion, Life Technologies |
| | | | #2 | s51977 | GCACCAAUCU UGACUUCCAtt | Ambion, Life Technologies |
| | | | #3 | s51978 | GCAUCUAUAU CAUAAAUCUtt | Ambion, Life Technologies |
| *Rps2* | uS5 | NM 002952 | #1 | s12252 | CCAAGUCUCCC UAUCAGGAtt | Ambion, Life Technologies |
| | | | #2 | s12253 | UCUCCGCACCU GUGCCUAAtt | Ambion, Life Technologies |
| | | | #3 | s12254 | CCUAAGAAGC UGCUCAUGAtt | Ambion, Life Technologies |
| *RpS3* | uS3 | NM 001005 | #1 | s12255 | UGACUGCUGU AGUUCAGAAtt | Ambion, Life Technologies |
| | | | #2 | s12256 | GCGGAGACCC UGUUAACUAtt | Ambion, Life Technologies |
| | | | #3 | s12257 | AAGCUGAACU GAAUGAGUUtt | Ambion, Life Technologies |
| *RpS3A* | eS] | NM 001006 | #1 | s12258 | GCAACAAUCA GAUACGGAAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #2 | s12259 | AAUUCAAGCU GAUUACUGAtt | Ambion, Life Technologies |
| | | | #3 | s12260 | AGAUUGGUAU GAUGUGAAAtt | Ambion, Life Technologies |
| Rps4x | eS4 | NM 001007 | #1 | s12261 | GCGGUUCAUU AAAAUCGAUtt | Ambion, Life Technologies |
| | | | #2 | s12262 | GCAUGCAGCG GUUCAUUAAtt | Ambion, Life Technologies |
| | | | #3 | s12263 | AGACUUAAGU AUGCCCUGAtt | Ambion, Life Technologies |
| RpS5 | uS7 | NM 001009 | #1 | s12267 | CCGGAACAUU AAGACCAUUtt | Ambion, Life Technologies |
| | | | #2 | s12268 | ACAUUGCAGU GAAGGAGAAtt | Ambion, Life Technologies |
| | | | #3 | s12269 | GCAUGCCUUC GAGAUCAUAtt | Ambion, Life Technologies |
| RpS6 | eS6 | NM 001010 | #1 | s12270 | CCUUAAAUAA AGAAGGUAAtt | Ambion, Life Technologies |
| | | | #2 | s12271 | GGAACAAAUU GCGAAGAGAtt | Ambion, Life Technologies |
| | | | #3 | s12272 | CAGCGUACCA AGAAAAAUAtt | Ambion, Life Technologies |
| RpS7 | eS7 | NM 001011 | #1 | s12288 | CAUAAUCUUU GUUCCCGUUtt | Ambion, Life Technologies |
| | | | #2 | s12289 | GAGUUUCAAU UGUAAACAAtt | Ambion, Life Technologies |
| | | | #3 | s12290 | GUACGCGAAU UGGAGAAAtt | Ambion, Life Technologies |
| RpS8 | eS8 | NM 001012 | #1 | s12291 | GCUAUGUGCU AGAGGGCAAtt | Ambion, Life Technologies |
| | | | #2 | s12292 | GAAAUAUGAU GAAAGGAAAtt | Ambion, Life Technologies |
| | | | #3 | s12293 | AGAGUGUUGU ACUCGUAAAtt | Ambion, Life Technologies |
| RpS9 | uS4 | NM 001013 | #1 | s12294 | AGAUGAAGCU GGAUUACAUtt | Ambion, Life Technologies |
| | | | #2 | s12295 | AGAUAGAGGA UUUCUUAGAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #3 | s12296 | AGCUGAAGCU GAUCGGCGAtt | Ambion, Life Technologies |
| RpS10 | eS10 | NM 001014 | #1 | s12297 | GCAACCGAAU UCCAGUUUAtt | Ambion, Life Technologies |
| | | | #2 | s12298 | CGACCUGCGA GACUCACAAtt | Ambion, Life Technologies |
| | | | #3 | s12299 | CAUUUCUACU GGUACCUUAtt | Ambion, Life Technologies |
| RpS11 | uS17 | NM 001015 | #1 | s12300 | AGAACAUGUC UGUACACCUtt | Ambion, Life Technologies |
| | | | #2 | s200170 | CUACAUCCGCA AGUACAACtt | Ambion, Life Technologies |
| | | | #3 | s200171 | CCAGAUCGGU GACAUCGUCtt | Ambion, Life Technologies |
| RpS12 | eS12 | NM 001016 | #1 | s52989 | CCUUUGUGCU GAACACCAAtt | Ambion, Life Technologies |
| | | | #2 | s194763 | GGCCUUUGUA AAAUUGACAtt | Ambion, Life Technologies |
| | | | #3 | s194764 | ACUGUGAUGA GCCUAUGUAtt | Ambion, Life Technologies |
| RpS13 | uS15 | NM 001017 | #1 | s12304 | GAAAGGAUAA GGAUGCUAAtt | Ambion, Life Technologies |
| | | | #2 | s12305 | GGCCUUACUCC UUCACAGAtt | Ambion, Life Technologies |
| | | | #3 | s12306 | GCUCGAUAUU AUAAGACCAtt | Ambion, Life Technologies |
| RpS14 | uS11 | NM_001025 070 | #1 | s12307 | AGACCGAGAU GAAUCCUCAtt | Ambion, Life Technologies |
| | | | #2 | s12308 | AGGUAAAGGC AGACCGAGAtt | Ambion, Life Technologies |
| | | | #3 | s12309 | CAUCCUUCAA UGACACUUUtt | Ambion, Life Technologies |
| RpS15 | uS19 | NM 001018 | #1 | s12310 | CCUACAAGCCC GUAAAGCAtt | Ambion, Life Technologies |
| | | | #2 | s194765 | CUUCAUCCCUC UCAAGUAAtt | Ambion, Life Technologies |
| | | | #3 | s194766 | GCAUGGUGGG CGUCUACAAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| *RpS15A* | *uS8* | NM 001019 | #1 | s12311 | CCAAAGUCAU CGUCCGGUUtt | Ambion, Life Technologies |
| | | | #2 | s12312 | AGAUUUGACG UGCAACUCAtt | Ambion, Life Technologies |
| | | | #3 | s12313 | GAGUAUCAAC AAUGCCGAAtt | Ambion, Life Technologies |
| *RpS16* | *uS9* | NM 001020 | #1 | s12314 | AGAUUUAUGC UAUCCGUCAtt | Ambion, Life Technologies |
| | | | #2 | s12315 | GCAAUGGUCU CAUCAAGGUtt | Ambion, Life Technologies |
| | | | #3 | s12316 | GGAGCGAUUU GCUGGUGUAtt | Ambion, Life Technologies |
| *RpS17* | *eS17* | NM 001021 | #1 | s12317 | GCCCGGGUCA UCAUAGAAAtt | Ambion, Life Technologies |
| | | | #2 | s12318 | GGAGAUUAUU GAAGUAGAUtt | Ambion, Life Technologies |
| | | | #3 | s12319 | GGAUCAGGAG AUUAUUGAAtt | Ambion, Life Technologies |
| *RpS18* | *uS13* | NM 022551 | #1 | s12320 | UGCGAGUACU CAACACCAAtt | Ambion, Life Technologies |
| | | | #2 | s12321 | UGAUCACCAU UAUGCAGAAtt | Ambion, Life Technologies |
| | | | #3 | s12322 | CGAUGGGCGG CGGAAAAUAtt | Ambion, Life Technologies |
| *RpS19* | *eS19* | NM 001022 | #1 | s12323 | GCCGCAAACU GACACCUCAtt | Ambion, Life Technologies |
| | | | #2 | s194767 | AAACCAUGCU GGGUUAAUAtt | Ambion, Life Technologies |
| | | | #3 | s194768 | AGCUGGCCAA GCACAAAGAtt | Ambion, Life Technologies |
| *RpS20* | *uS10* | NM 001023 | #1 | s12324 | GGACCAGUUC GAAUGCCUAtt | Ambion, Life Technologies |
| | | | #2 | s12325 | GCCGCAACGU AAAAUCCUUtt | Ambion, Life Technologies |
| | | | #3 | s12326 | CUAACAAGCC GCAACGUAAtt | Ambion, Life Technologies |
| *RpS21* | *eS21* | NM 001024 | #1 | s12327 | AGUUUAAAAC UUAUGCUAUtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #2 | s226974 | GUAGGAUGGG UGAGUCAGAtt | Ambion, Life Technologies |
| | | | #3 | s194769 | GCAUCAUCGG UGCCAAGGAtt | Ambion, Life Technologies |
| *RpS23* | *uS12* | NM 001025 | #1 | s12328 | CCAAUGACGG UUGCUUGAAtt | Ambion, Life Technologies |
| | | | #2 | s12329 | GAAGCUCCGU AGUCACCGAtt | Ambion, Life Technologies |
| | | | #3 | s12330 | UGACGGUUGC UUGAACUUUtt | Ambion, Life Technologies |
| *RpS24* | *eS24* | NM 001026 | #1 | s12331 | CCGACUACUUC AGAGGAAAtt | Ambion, Life Technologies |
| | | | #2 | s12332 | AGACAGAAAU UCGGGAAAAtt | Ambion, Life Technologies |
| | | | #3 | s12333 | CCUGGAUUAU GCAAAGAAAtt | Ambion, Life Technologies |
| *RpS25* | *eS25* | NM 001028 | #1 | s12334 | GCACAGAGCU CAAGUAAUUtt | Ambion, Life Technologies |
| | | | #2 | s12335 | GGAGCUCCUU AGUAAAGGAtt | Ambion, Life Technologies |
| | | | #3 | s12336 | GGAAGUUCCC AACUAUAAAtt | Ambion, Life Technologies |
| *Rps26* | *eS26* | NM_001093 731 | #1 | s229467 | ACAGCAAAGU AGUCAGGAAtt | Ambion, Life Technologies |
| | | | #2 | s53606 | CAAGCUGUAU GUGAAGCUAtt | Ambion, Life Technologies |
| | | | #3 | s229468 | AUUCGUCAUU CGAAACAUAtt | Ambion, Life Technologies |
| *RpS27* | *eS27* | NM 001030 | #1 | s12337 | GGAGGAAAAG CAAGGCUUAtt | Ambion, Life Technologies |
| | | | #2 | s12338 | AUGCACAAAC GGUAGUUUUtt | Ambion, Life Technologies |
| | | | #3 | s194771 | GGAGGAAGCA GCACUAAAAtt | Ambion, Life Technologies |
| *RpS27 L* | *eS27L* | NM 015920 | #1 | s27333 | AAGUCCAAAU UCUUACUUUtt | Ambion, Life Technologies |
| | | | #2 | s27334 | AAAUGUCCAG GUUGCUACAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
|  |  |  | #3 | s27335 | UGUCCAGGUU GCUACAAGAtt | Ambion, Life Technologies |
| RpS27A | eS31 | NM 002954 | #1 | s12340 | GUACUUUGUC UGACUACAAtt | Ambion, Life Technologies |
|  |  |  | #2 | s12341 | GGACGUACUU UGUCUGACUtt | Ambion, Life Technologies |
|  |  |  | #3 | s226976 | GAAGAAGUCU UACACCACUtt | Ambion, Life Technologies |
| RpS28 | eS28 | NM 001031 | #1 | s12342 | CCAUCAUCCGC AAUGUAAAtt | Ambion, Life Technologies |
|  |  |  | #2 | s12343 | GCCGAUCCAUC AUCCGCAAtt | Ambion, Life Technologies |
|  |  |  | #3 | s226977 | GCGUGGAAUU CAUGGACGAtt | Ambion, Life Technologies |
| Rps29 | uSl4 | NM_001030 001 | #1 | s227039 | GCACGGUCUG AUCCGGAAAtt | Ambion, Life Technologies |
|  |  |  | #2 | s194772 | CGUCAGUACG CGAAGGAUAtt | Ambion, Life Technologies |
|  |  |  | #3 | s196844 | GGAAAUAUGG CCUCAAUAUtt | Ambion, Life Technologies |
| RpS30 | eS30 | NM 001997 | #1 | s5039 | UGAGAGGUCA GACUCCUAAtt | Ambion, Life Technologies |
|  |  |  | #2 | s5040 | CCAGAUCAAG GCUCAUGUAtt | Ambion, Life Technologies |
|  |  |  | #3 | s194404 | UUCUGGCUUU CUCUAAUAAtt | Ambion, Life Technologies |
| Rackl | Rackl | NM 006098 | #1 | s20340 | CAGGCUAUCU GAACACGGUtt | Ambion, Life Technologies |
|  |  |  | #2 | s20341 | CAAACACCUU UACACGCUAtt | Ambion, Life Technologies |
|  |  |  | #3 | s20342 | GGAUGAGACC AACUAUGGAtt | Ambion, Life Technologies |
| RpL3 | uL3 | NM 000967 | #1 | s12142 | GGCAUAAAUC UAAGAAGAAtt | Ambion, Life Technologies |
|  |  |  | #2 | s12143 | ACGGCAAGCU GAUCAAGAAtt | Ambion, Life Technologies |
|  |  |  | #3 | s12144 | GACUAUGACC UAUCUGACAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| RpL4 | uL4 | NM 000968 | #1 | s12148 | GACCAGAUAU UGUGAACUUtt | Ambion, Life Technologies |
| | | | #2 | s12149 | GGCCGAAUGU UUGCACCAAtt | Ambion, Life Technologies |
| | | | #3 | s12150 | CCGCUUCCCUC AAGAGUAAtt | Ambion, Life Technologies |
| RpL5 | uL18 | NM 000969 | #1 | s12151 | GACGAGAGGG UAAAACUGAtt | Ambion, Life Technologies |
| | | | #2 | s12152 | GGAGGAGAUG UAUAAGAAAtt | Ambion, Life Technologies |
| | | | #3 | s12153 | GAAGUACAUC GGAAGCACAtt | Ambion, Life Technologies |
| RpL6 | eL6 | NM 000970 | #1 | s12154 | AGCGCAAGAU UGAUCAGAAtt | Ambion, Life Technologies |
| | | | #2 | s12155 | CCAAAAUCGA UAUCAGCAAtt | Ambion, Life Technologies |
| | | | #3 | s12156 | CCCAAAACAUC UUACUGAUtt | Ambion, Life Technologies |
| RpL7 | uL30 | NM 000971 | #1 | s352 | CUCGAUCUCU UGGUAAAUAtt | Ambion, Life Technologies |
| | | | #2 | s353 | CACUAUCACA AGGAAUAUAtt | Ambion, Life Technologies |
| | | | #3 | s354 | CGUCAAAUCU UCAAUGGAAtt | Ambion, Life Technologies |
| RpL7A | eL8 | NM 000972 | #1 | s12157 | GGUGAACUCG GAAGACAAAtt | Ambion, Life Technologies |
| | | | #2 | s12158 | CAGCUGUCGU GAAGAAGCAtt | Ambion, Life Technologies |
| | | | #3 | s12159 | AGUACAGACC AGAGACAAAtt | Ambion, Life Technologies |
| RpL8 | uL2 | NM 000973 | #1 | s12160 | GCCGAAUUGA CAAACCCAUtt | Ambion, Life Technologies |
| | | | #2 | s12161 | CCAUGCCUGA GGGUACAAUtt | Ambion, Life Technologies |
| | | | #3 | s12162 | CGGGAUCCGU AUCGGUUUAtt | Ambion, Life Technologies |
| RpL9 | uL6 | NM 000661 | #1 | s12163 | AUGUCGACAU UACUCUGAAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #2 | s12164 | GAAAGAUGAA UUAAUCCUUtt | Ambion, Life Technologies |
| | | | #3 | s12165 | CCAGAAAAUG UCGACAUUAtt | Ambion, Life Technologies |
| RpL10 | uL16 | NM 006013 | #1 | s12166 | GAAACAGGUU GACAACUCAtt | Ambion, Life Technologies |
| | | | #2 | s12167 | CUAUGUCUUU GUAUCUACAtt | Ambion, Life Technologies |
| | | | #3 | s196275 | CCCGAAUUUG UGCCAAUAAtt | Ambion, Life Technologies |
| RpL10 A | uL1 | NM 007104 | #1 | s9421 | GUCCGGGCCU UAUAUAUCAtt | Ambion, Life Technologies |
| | | | #2 | s9422 | CAGAGUCUCU GAUCAAGCAtt | Ambion, Life Technologies |
| | | | #3 | s9423 | UGAAGAAGGU GUUAUGUCUtt | Ambion, Life Technologies |
| RpL11 | uL5 | NM 000975 | #1 | s12168 | GGUGCGGGAG UAUGAGUUAtt | Ambion, Life Technologies |
| | | | #2 | s12169 | CAACUUCUCA GAUACUGGAtt | Ambion, Life Technologies |
| | | | #3 | s12170 | GGAACUUCGC AUCCGCAAAtt | Ambion, Life Technologies |
| RpL12 | uL11 | NM 000976 | #1 | s194741 | CCCAGUCAGU GGGCUGUAAtt | Ambion, Life Technologies |
| | | | #2 | s194742 | CCAGCCAGUU AAGCACAAAtt | Ambion, Life Technologies |
| | | | #3 | s194743 | AGCCCUCAAG GAACCACCAtt | Ambion, Life Technologies |
| RpL13 | eL13 | NM 000977 | #1 | s12171 | GGAAGAGAAG AAUUUCAAAtt | Ambion, Life Technologies |
| | | | #2 | s12172 | AGAAGAAUUU CAAAGCCUUtt | Ambion, Life Technologies |
| | | | #3 | s12173 | CGGCAUACGG GCAAAAAGAtt | Ambion, Life Technologies |
| RpL13 A | uL13 | NM 012423 | #1 | s58865 | AGCUCAUGAG GCUACGGAAtt | Ambion, Life Technologies |
| | | | #2 | s23995 | GGUGUUUGAC GGCAUCCCAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #3 | s23996 | GGAAACAGGC CGAGAAGAAtt | Ambion, Life Technologies |
| RpL14 | eL14 | NM_001034 996 | #1 | s17238 | GACAGAUUUU GAUCGUUUUtt | Ambion, Life Technologies |
| | | | #2 | s17239 | CCAGAAGUAU GUCCGACAAtt | Ambion, Life Technologies |
| | | | #3 | s17240 | AGAUCACCGCC GCGAGUAAtt | Ambion, Life Technologies |
| RpL15 | eL15 | NM 002948 | #1 | s12174 | CCUUUCAAGU GUGAGCUUAtt | Ambion, Life Technologies |
| | | | #2 | s12175 | CCUUAAGAUU GGUAAGCUAtt | Ambion, Life Technologies |
| | | | #3 | s12176 | GGUGUAGACU UUUUAAGUUtt | Ambion, Life Technologies |
| RpL17 | uL22 | NM 000985 | #1 | s12177 | CACGAAAUCA UGCAAAUCAtt | Ambion, Life Technologies |
| | | | #2 | s12178 | CAAUCUUCGU GUUCACUUUtt | Ambion, Life Technologies |
| | | | #3 | s12179 | GCACAUGCUU AAAAACGCAtt | Ambion, Life Technologies |
| RpL18 | eL18 | NM 000979 | #1 | s194744 | GGCUGUUGGU CAAGUUAUAtt | Ambion, Life Technologies |
| | | | #2 | s194745 | AAACUAACCC UGGAUCCUAtt | Ambion, Life Technologies |
| | | | #3 | s194746 | CGGAUGAUCC GGAAGAUGAtt | Ambion, Life Technologies |
| RpL18 A | eL20 | NM 000980 | #1 | s12180 | GUACUUUGUA UCUCAGUUAtt | Ambion, Life Technologies |
| | | | #2 | s12181 | CCCACAACAUG UACCGGGAtt | Ambion, Life Technologies |
| | | | #3 | s12182 | ACUCCAUUCA GAUCAUGAAtt | Ambion, Life Technologies |
| RpL19 | eL19 | NM 000981 | #1 | s12183 | AGACCAAGGA AGCACGCAAtt | Ambion, Life Technologies |
| | | | #2 | s12184 | AGAAGAUACC GUGAAUCUAtt | Ambion, Life Technologies |
| | | | #3 | s12185 | GCUCAGAAGA UACCGUGAAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| *RpL21* | *eL21* | NM 000982 | #1 | s12186 | CACUCUAAGA GCCGAGAUAtt | Ambion, Life Technologies |
| | | | #2 | s12187 | GGAAGAGUCU ACAAUGUUAtt | Ambion, Life Technologies |
| | | | #3 | s12188 | GGUGAUAUUG UAGACAUCAtt | Ambion, Life Technologies |
| *RpL22* | *eL22* | NM 000983 | #1 | s12189 | GAGAGUUACG AAUUACGUUtt | Ambion, Life Technologies |
| | | | #2 | s12190 | CAAAGAGAGU UACGAAUUAtt | Ambion, Life Technologies |
| | | | #3 | s12191 | GGUUGCGCGU AGUUGCUAAtt | Ambion, Life Technologies |
| *RpL23* | *uL14* | NM 000978 | #1 | s17871 | GCAGGAGUCA UAGUGAACAtt | Ambion, Life Technologies |
| | | | #2 | s17872 | CAAUAAAGGC GAGAUGAAAtt | Ambion, Life Technologies |
| | | | #3 | s17873 | CAACGAAAGU CAUACCGUAtt | Ambion, Life Technologies |
| *RpL23 A* | *uL23* | NM 000984 | #1 | s12192 | GAGUUAGUGU CCUAGGAAAtt | Ambion, Life Technologies |
| | | | #2 | s12193 | AGAGAUCUUU GUGACUAGAtt | Ambion, Life Technologies |
| | | | #3 | s12194 | CUGUUCUACU UAUCCUUUUtt | Ambion, Life Technologies |
| *RpL24* | *eL24* | NM 000986 | #1 | s12198 | CGAGCAGUCA AAUUCCAGAtt | Ambion, Life Technologies |
| | | | #2 | s12199 | AGAAACCUGA AGUUAGAAAtt | Ambion, Life Technologies |
| | | | #3 | s12200 | CUACAAAGGC AGCACCUAAtt | Ambion, Life Technologies |
| *RpL26* | *uL24* | NM 000987 | #1 | s12201 | CCACAUUCGA AGGAAGAUUtt | Ambion, Life Technologies |
| | | | #2 | s12202 | GGUUGUACGU GGACACUAUtt | Ambion, Life Technologies |
| | | | #3 | s12203 | GAAAUAUGUU AUCUACAUUtt | Ambion, Life Technologies |
| *RpL27* | *eL27* | NM 000988 | #1 | s12204 | GAGAGAUACA AGACAGGCAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #2 | s12205 | ACAAAACUGU CGUCAAUAAtt | Ambion, Life Technologies |
| | | | #3 | s12206 | AAACUGUCGU CAAUAAGGAtt | Ambion, Life Technologies |
| RpL27 A | uL15 | NM 000990 | #1 | s12210 | GGAUCAACUU CGACAAAUAtt | Ambion, Life Technologies |
| | | | #2 | s12211 | GCCCAACUGUC AACCUUGAtt | Ambion, Life Technologies |
| | | | #3 | s12212 | CACUUAAAGA GGAACCAGAtt | Ambion, Life Technologies |
| RpL28 | eL28 | NM 000991 | #1 | s12213 | CACCGUCUCUA AAAUAAAAtt | Ambion, Life Technologies |
| | | | #2 | s12214 | GCACUGAGCCC AAUAACUUtt | Ambion, Life Technologies |
| | | | #3 | s12215 | UGAUCAAGAG GAAUAAGCAtt | Ambion, Life Technologies |
| RpL29 | eL29 | NM 000992 | #1 | s12216 | CCUGUGCUAU UUGUACAAAtt | Ambion, Life Technologies |
| | | | #2 | s12217 | CGAUCACAAA GAUACGAAUtt | Ambion, Life Technologies |
| | | | #3 | s194747 | GGAACAUGCG CUUUGCCAAtt | Ambion, Life Technologies |
| RpL30 | eL30 | NM 000989 | #1 | s12207 | GGCUAUCAUU GAUCCAGGUtt | Ambion, Life Technologies |
| | | | #2 | s12208 | GGCUCCAACUC GUUAUGAAtt | Ambion, Life Technologies |
| | | | #3 | s12209 | GGAAAUCUGA AAUAGAGUAtt | Ambion, Life Technologies |
| RpL31 | eL31 | NM 000993 | #1 | s12218 | CUCCAGAUGU GCGCAUUGAtt | Ambion, Life Technologies |
| | | | #2 | s194749 | GGAAUGUGCC AUACCGAAUtt | Ambion, Life Technologies |
| | | | #3 | s231333 | CCAAAUAAGC UAUAUACUUtt | Ambion, Life Technologies |
| RpL32 | eL32 | NM 000994 | #1 | s12220 | UGGUUAUGGA AGCAACAAAtt | Ambion, Life Technologies |
| | | | #2 | s12221 | GACUGGUACU CAGAAUUUAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #3 | s227220 | ACAGGGUUCG UAGAAGAUUtt | Ambion, Life Technologies |
| *RpL34* | *eL34* | NM 000995 | #1 | s12222 | AUAGAAUUGU UUACCUUUAtt | Ambion, Life Technologies |
| | | | #2 | s12223 | AAAUCGUUGU GAAAGUGUUtt | Ambion, Life Technologies |
| | | | #3 | s12224 | CAAUACAGCC UCUAACAAAtt | Ambion, Life Technologies |
| *RpL35* | *uL29* | NM 007209 | #1 | s22151 | CAAGCUCUCU AAGAUCCGAtt | Ambion, Life Technologies |
| | | | #2 | s22152 | GGAAAUCCAU UGCCCGUGUtt | Ambion, Life Technologies |
| | | | #3 | s22153 | UAACCAGACU CAGAAAGAAtt | Ambion, Life Technologies |
| *RpL35 A* | *eL33* | NM 000996 | #1 | s12225 | GUAUAUAAAG CAAAGAACAtt | Ambion, Life Technologies |
| | | | #2 | s12226 | ACAGAAUUCU AUUUGGGCAtt | Ambion, Life Technologies |
| | | | #3 | s12227 | GAGAUGCGCU UAUGUAUAUtt | Ambion, Life Technologies |
| *RpL36* | *eL36* | NM 015414 | #1 | s24653 | CCCUCAAAUU UAUCAAGAAtt | Ambion, Life Technologies |
| | | | #2 | s24654 | AAACGGGCCC UCAAAUUUAtt | Ambion, Life Technologies |
| | | | #3 | s226046 | GCGCCAUGGA GUUACUGAAtt | Ambion, Life Technologies |
| *RpL36 A* | *eL42* | NM 021029 | #1 | s199106 | AGUGUCAUCU UUUAUUAUGtt | Ambion, Life Technologies |
| | | | #2 | s231826 | GACUUUCUGU AAGAAGUGUtt | Ambion, Life Technologies |
| | | | #3 | s231827 | CUAAGCCGAU UUUCCGGAAtt | Ambion, Life Technologies |
| *RpL37* | *eL37* | NM 000997 | #1 | s12230 | GAACAACACC UAAACCCAAtt | Ambion, Life Technologies |
| | | | #2 | s 12231 | GGUCGAAUGA GGCACCUAAtt | Ambion, Life Technologies |
| | | | #3 | s12232 | GCUAAAAGAC GAAAUACCAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| RpL37A | eL43 | NM 000998 | #1 | s12233 | CACUUCCGCUG UCACGGUAtt | Ambion, Life Technologies |
| | | | #2 | s194751 | CGCCAAGUAC ACUUGCUCUtt | Ambion, Life Technologies |
| | | | #3 | s194752 | GGUAAAGUCC GCCAUCAGAtt | Ambion, Life Technologies |
| RpL38 | eL38 | NM 000999 | #1 | s12234 | AGGACAACGU GAAGUUUAAtt | Ambion, Life Technologies |
| | | | #2 | s194753 | GGACAACGUG AAGUUUAAAtt | Ambion, Life Technologies |
| | | | #3 | s194754 | CCGACGAAAG GAUGCCAAAtt | Ambion, Life Technologies |
| RpL39 | eL39 | NM 001000 | #1 | s12235 | UCACGAUCAU GUUACCAUAtt | Ambion, Life Technologies |
| | | | #2 | s194755 | GGAGAUUUCG ACGUGUUUUtt | Ambion, Life Technologies |
| | | | #3 | s194756 | CCACUAUCUG GAGAUUUCGtt | Ambion, Life Technologies |
| RpL40 | eL40 | NM_001033 930 | #1 | s14556 | AGACAAGGAG GGUAUCCCAtt | Ambion, Life Technologies |
| | | | #2 | s14557 | AAAUACAACU GCGACAAGAtt | Ambion, Life Technologies |
| | | | #3 | s14558 | GUCUGAUAUU UGCCGGCAAtt | Ambion, Life Technologies |
| RpL41 | eL41 | NM_001035 267 | #1 | s12236 | CAAUGGAUCU AGAACUUCAtt | Ambion, Life Technologies |
| | | | #2 | s12237 | CCACCUUGCUC AUAAACAAtt | Ambion, Life Technologies |
| | | | #3 | s12238 | GUAACAACCA UAUAAUAAAtt | Ambion, Life Technologies |
| P0 | uL10 | NM 001002 | #1 | s226965 | AGAUCAUCCA ACUAUUGGAtt | Ambion, Life Technologies |
| | | | #2 | s226966 | CGAGGGCACC UGGAAAACAtt | Ambion, Life Technologies |
| | | | #3 | s785 | GUUUCAUUGU GGGAGCAGAtt | Ambion, Life Technologies |
| P1 | P1 | NM 001003 | #1 | s12239 | GCACGACGAU GAGGUGACAtt | Ambion, Life Technologies |

(continued)

| Ribosomal proteins (RPs) | | | | | | |
|---|---|---|---|---|---|---|
| Gene name | New nomenclature | GenBank accession number | siRNA number | siRNA ID | siRNA sequence (5' to 3') | Source |
| | | | #2 | s194758 | GAGCCUCAUC UGCAAUGUAtt | Ambion, Life Technologies |
| | | | #3 | s194759 | ACAUGGGCUU UGGUCUUUUtt | Ambion, Life Technologies |
| P2 | P2 | NM 001004 | #1 | s194760 | CCAGGGUAUU GGCAAGCUUtt | Ambion, Life Technologies |
| | | | #2 | s194761 | GACAUGGGAU UUGGCCUUUtt | Ambion, Life Technologies |
| | | | #3 | s194762 | GACCGGCUCA ACAAGGUUAtt | Ambion, Life Technologies |
| e, eukaryotic; u, universal | | | | | | |

| CP assembly factors | GenBank accession number | siRNA number | siRNA ID | siRNA sequence (5' to 3') | Source |
|---|---|---|---|---|---|
| BXDC1 | NM_03219 4 | SEQ ID NO:1 | HSS.RNAI. N032194.12 .1 | rUrCrUrUrCrUrArUrArArUrC rUrUrCrUrGrUrUrArCrArUrCr GrArA | IDT |
| | | SEQ ID NO:2 | HSS.RNAI. N032194.12 .2 | rArArUrArCrCrUrArArUrUrC rArArUrCrArUrArUrCrCrArGr CrArC | IDT |
| | | SEQ ID NO:3 | HSS.RNAI. N032194.12 .3 | rUrCrUrUrGrCrUrUrCrUrGrCr ArUrArUrGrArArUrCrCrUrUr CrCrA | IDT |
| RRS1 | NM_01516 9 | SEQ ID NO:4 | HSS.RNAI. N015169.12 .1 | rUrCrCrUrCrCrUrCrCrCrUrCr ArUrCrUrGrCrUrUrArUrUrGr GrUrG | IDT |
| | | SEQ ID NO:5 | HSS.RNAI. N015169.12 .4 | rArUrCrArGrCrCrArUrUrCrUr UrUrGrGrUrGrUrCrGrUrCrCr CrGrG | IDT |
| | | SEQ ID NO:6 | HSS.RNAI. N015169.12 .6 | rCrUrUrUrCrCrUrUrCrUrUrGr GrCrCrUrGrArArUrCrCrGrCr UrUrG | IDT |

| Calibration set Gene name | GenBank accession number | siRNA ID | siRNA or snoRNA sequence (5' to 3') | Source |
|---|---|---|---|---|
| Scramble (SCR) | / | s4390844 | not available | Ambion, Life Technologies |

(continued)

| Calibration set Gene name | GenBank accession number | siRNA ID | siRNA or snoRNA sequence (5' to 3') | Source |
|---|---|---|---|---|
| GFP | / | AM4626 | not available | Ambion, Life Technologies |
| Fibrillarin (FBL) | NM_001436 3 | s4821 | UGACAUCGUUGGUCCGGAUtt | Ambion, Life Technologies |
| Nucleophosmi n (NPM) | NM 002520 | s9677 | GCAAUGAAUUACGAAGGCAtt | Ambion, Life Technologies |
| Nucleolin (NCL) | NM 005381 | s9312 | GGAAAUGGCCAAACAGAAAtt | Ambion, Life Technologies |
| TIF1A | NM 018427 | s29325 | CGAUGUUGCUCUCCAUGGAtt | Ambion, Life Technologies |
| UTP18 | NM 016001 | s27417 | CAUUCUCUUCAGAUAGUAAtt | Ambion, Life Technologies |
| NOL9 | NM 024654 | s36145 | GCCCUGGAAGAGUUAGUCAtt | Ambion, Life Technologies |
| U8 | NR 033294 | SEQ ID NO:7 | mGmGmAmUmUATCCCACCTGmAmCmGmAmU | IDT |

**Imaging**

Imaging was performed on a Zeiss Axio Observer.Z1 microscope with a motorized stage, driven by MetaMorph (MDS Analytical Technologies, Canada). Images were captured in widefield mode with a 20x objective (Plan NeoFluar, Zeiss), a LED illumination (CoolLed pE-2) and a CoolSnap HQ2 camera. Sixteen independent fields of view were captured automatically for each well. The correct focal plane was maintained by using the built-in autofocus module of MetaMorph. High-resolution images were captured in confocal mode using a Yokogawa spindisk head and the HQ2 camera with a laser from Roper (405 nm 100 mW Vortran, 491 nm 50 mW Cobolt Calypso, and 561 nm 50 mW Cobolt Jive) and a 40x objective (Plan NeoFluar, Zeiss).

**PES1 detection by indirect immunofluorescence**

After 3 days siRNA-mediated depletion, cells were fixed in 2% formaldehyde, washed in PBS and blocked in PBS supplemented with 5% BSA and 0.3% Triton X-100 during 1 hour at RT. Anti-PES1 antibody (anti-rat, 1:1,000; courtesy from E. Kremmer) was diluted in PBS supplemented with 1% BSA, 0.3% Triton X-100 and incubated with the cells overnight at 4°C. Cells were washed in PBS and incubated with a secondary Alexa Fluor 594 anti-rat antibody (1:1,000; Invitrogen) in PBS, 1% BSA, 0.3% Triton X-100 during 1 hour at RT. Cells were finally washed in PBS, treated with DAPI and imaged with the Zeiss microscope as described above.

**Pre-rRNA processing analysis**

For the pre-rRNA analysis, we used a colon carcinoma cell line (HCT116) expressing normally p53 (39). Northern-blot analyses were performed essentially as described in (38) and www.RibosomeSynthesis.com. Briefly, HCT116 cells were transfected with one siRNA specific to transcripts encoding each r-protein in 6-well plates and incubated for 2 days prior to total RNA extraction and Northern-blot analysis. The probes used are described in Table 3. Two distinct siRNAs were used in two independent experiments. The "R" software was used to generate and cluster the heatmaps. These heatmaps are a visual representation of the logarithm of the ratio of the pre-rRNA level in the knockdown condition respective to its level in the non-targeting (Scramble, SCR) control. The calibration set used in the pre-rRNA processing analysis consists of mock-treated cells, and cells treated with a non-targeting siRNA (Scramble, SCR), or with siRNAs specific to UTP18 or NOL9 (see Table 2 and (38) and www.RibosomeSynthesis.com). In our clustering analysis, we did not average the processing data obtained with the two different siRNAs used in this work for each r-protein, but rather, we considered them as individual experiments. In most cases, the two independent processing datasets obtained for any particular r-protein are highly clustered, demonstrating the robustness of our screens. In a few cases (denoted with a star in Fig. 4, and observed only for two SSU and six LSU r-proteins), the heatmaps do not belong to the same class, reflecting the inherent variation in depletion efficiency from one individual siRNA to another. Note that all RNA species detected were used to cluster the heatmaps shown in Fig. 4c and Fig. 4d but only those directly relevant to synthesis of the small (in panel c), or large (in panel d) subunit are shown for simplicity. The clusters with all the RNA species are shown in Supplementary Fig. 5,6. The 28S/18S rRNA ratios were calculated from Agilent bioanalyzer electropherograms according to the manufacturer's instructions. Examples of uncropped Northern blots are show in Supplementary Fig. 12.

Table 3

| Oligo probe name | Northern blot probes | |
|---|---|---|
| LD1844 (5'-ETS) | CGGAGGCCCAACCTCTCCGACGACAGGTCGCCAGAGGACAGCGTGTCAGC | SEQ ID NO:8 |
| LD1827 (ITS1) | CCTCGCCCTCCGGGCTCCGGGCTCCGTTAATGATC | SEQ ID NO:9 |
| LD1828 (ITS2°) | CTGCGAGGGAACCCCCAGCCGCGCA | SEQ ID NO: 10 |
| Oligo probe name | RTqPCR primers | |
| LD1818 (GAPDH Forward) | TGCACCACCAACTGCTTAG | SEQ ID NO:11 |
| LD1819 (GAPDH Reverse) | GTTCAGCTCAGGGATGACC | SEQ ID NO:12 |
| LD3657 (eL22 Forward) | TTGGTGCTCTGTGGATTGAG | SEQ ID NO:13 |
| LD3658 (eL22 Reverse) | TTGCCAAAGAGCAACTGATG | SEQ ID NO:14 |
| LD3659 (eS24 Forward) | AACTGGCTTTGGCATGATTT | SEQ ID NO:15 |
| LD3660 (eS24 Reverse) | GCAGCACCTTTACTCCTTCG | SEQ ID NO:16 |
| LD3661 (uS12 Forward) | CCCACTTTCCGTAGGATCAA | SEQ ID NO:17 |
| LD3662 (uS12 Reverse) | GTCCTGAGGCTGGATATGGA | SEQ ID NO:18 |
| LD3663 (eS10 Forward) | CGCAGAGATGTTGATGCCTA | SEQ ID NO:19 |
| LD3664 (eS10 Reverse) | TCCAGGCAAACTGTTCCTTC | SEQ ID NO:20 |
| LD2855 (eS6 Forward) | CTAGGACCAAAGCACCCAAG | SEQ ID NO:21 |
| LD2856 (eS6 Reverse) | GGAAAGTCTGCGTCTCTTCG | SEQ ID NO:22 |
| LD3665 (RACK1 Forward) | CAAGCTGAAGACCAACCACA | SEQ ID NO:23 |

(continued)

| Oligo probe name | RTqPCR primers | |
|---|---|---|
| LD3666 (RACK1 Reverse) | CACACAGCCAGTAGCGGTTA | SEQ ID NO:24 |
| LD3667 (uL23 Forward) | AACTTGCCTCCATGGTTGAG | SEQ ID NO:25 |
| LD3668 (uL23 Reverse) | TTGTCCTTTATTGGGCAAGG | SEQ ID NO:26 |
| LD3669 (eS7 Forward) | ACGGCAGCTAAGGAAATTGA | SEQ ID NO:27 |
| LD3670 (eS7 Reverse) | GTACGGCTTTTTCGAGTTGG | SEQ ID NO:28 |
| LD3671 (eL41 Forward) | CTCGGCACTTAGCATCATCA | SEQ ID NO:29 |
| LD3672 (eL41 Reverse) | TCCATGTTTCTGCTCCTGTG | SEQ ID NO:30 |
| LD3576 (eS1 Forward) | CCGGAAGAAGATGATGGAAA | SEQ ID NO:31 |
| LD3577 (eS1 Reverse) | TCCATGAGCTTTCCCAATTC | SEQ ID NO:32 |
| LD3673 (uS7 Forward) | GTGAACGCCATCATCAACAG | SEQ ID NO:33 |
| LD3674 (uS7 Reverse) | AGGCACTCAGCAATGGTCTT | SEQ ID NO:34 |
| LD2859 (eS12 Forward) | GAAGCTGCCAAAGCCTTAGA | SEQ ID NO:35 |
| LD2860 (eS12 Reverse) | AACCACTTTACGGGGTTTCC | SEQ ID NO:36 |
| LD3675 (uS19 Forward) | ACAACGGCAAGACCTTCAAC | SEQ ID NO:37 |
| LD3676 (uS19 Reverse) | GGAGTCATGTGCGCCTTTAT | SEQ ID NO:38 |
| LD3677 (uS15 Forward) | GGACTTGCTCCTGATCTTCCT | SEQ ID NO:39 |
| LD3678 (uS15 Reverse) | AGGGCAGAGGCTGTAGATGA | SEQ ID NO.40 |
| LD3679 (eL42 Forward) | TGATTGCTCCTACCGACTCC | SEQ ID NO.41 |
| LD3680 (eL42 Reverse) | GGCGTACAGAGAATCCTTGC | SEQ ID NO:42 |
| LD3431 (uL5 Forward) | GCATCCGGAGAAATGAAAAG | SEQ ID NO:43 |
| LD3432 (uL5 Reverse) | GTCCAGGCCGTAGATACCAA | SEQ ID NO:44 |

(continued)

| Oligo probe name | RTqPCR primers | |
|---|---|---|
| LD3681 (eS8 Forward) | TGAAGAATTGCATCGTGCTC | SEQ ID NO:45 |
| LD3682 (eS8 Reverse) | TCCTCCAGGAGACTGCTGAT | SEQ ID NO:46 |
| LD3683 (eL29 Forward) | ACATGGCCAAGTCCAAGAAC | SEQ ID NO:47 |
| LD3684 (eL29 Reverse) | ATTGTTGGCCTGCATCTTCT | SEQ ID NO:48 |
| LD3785 (uS2 Forward) | AGACGGCTGTGCTGAAGTTT | SEQ ID NO:49 |
| LD3786 (uS2 Reverse) | CAGCGCAATGGTAGGTAGGT | SEQ ID NO:50 |
| LD3685 (eL15 Forward) | AGCTCTCTGCTCTCCACAGG | SEQ ID NO:51 |
| LD3686 (eL15 Reverse) | TGAAGGCTTCGAGCAAACTT | SEQ ID NO:52 |
| LD3687 (eS4 Forward) | CCTGGATCTTTTGACGTGGT | SEQ ID NO:53 |
| LD3688 (eS4 Reverse) | TTCACCCACTGCTCTGTTTG | SEQ ID NO:54 |
| LD3689 (uS5 Forward) | TTATGCCAGTGCAGAAGCAG | SEQ ID NO:55 |
| LD3690 (uS5 Reverse) | ATCTTGTTCCCCCAGTAGCC | SEQ ID NO:56 |
| LD3691 (P2 Forward) | AGCTTGCCAGTGTACCTGCT | SEQIDNO:57 |
| LD3692 (P2 Reverse) | GGGGAGCAGGAATTTAATCA | SEQ ID NO:58 |
| LD3693 (eL31 Forward) | TGCCATCAACGAAGTGGTAA | SEQ ID NO:59 |
| LD3694 (eL31 Reverse) | TATTCCTTTGGCCCAGACAG | SEQ ID NO:60 |
| LD3695 (eS30 Forward) | TCGCITCITCCTCTITCTCG | SEQ ID NO:61 |
| LD3696 (eS30 Reverse) | GGAGCACGACTTGATCTTCC | SEQ ID NO:62 |
| LD2861 (eS27L Forward) | GTCTGGTAGGGCTGAGCTTG | SEQ ID NO:63 |
| LD2862 (eS27L Reverse) | ACTGTCTGAGCATGGCTGAA | SEQ ID NO:64 |
| LD3697 (uS3 Forward) | CTGGGCATCAAGGTGAAGAT | SEQ ID NO:65 |
| LD3698 (uS3 Reverse) | CCTGTTATGCTGTGGGGACT | SEQ ID NO:66 |

(continued)

| Oligo probe name | RTqPCR primers | |
|---|---|---|
| LD3699 (uS9 Forward) | GGCAATGGTCTCATCAAGGT | SEQ ID NO:67 |
| LD3700 (uS9 Reverse) | GGCTTTGGAGATGGACTGAC | SEQ ID NO:68 |
| LD3701 (eS17 Forward) | CCTGTGCTTCCTGTTTCCTC | SEQ ID NO:69 |
| LD3702 (eS17 Reverse) | GCTATCTTGTTGCGGAGCTT | SEQ ID NO:70 |
| LD3703 (uS11 Forward) | CTTTCAGGGAGGAGCTTGTG | SEQ ID NO:71 |
| LD3704 (uS11 Reverse) | ACCTTCATCCCACCAGTCAC | SEQ ID NO:72 |
| LD2863 (eS28 Forward) | TCCATCATCCGCAATGTAAA | SEQ ID NO:73 |
| LD2864 (eS28 Reverse) | AGTTACGTGTGGCGGACAAA | SEQ ID NO:74 |
| LD3705 (eL28 Forward) | ACAGACATCACGGGAGGAAG | SEQ ID NO:75 |
| LD3706 (eL28 Reverse) | GGACAATGCTAAGGCTGCTC | SEQ ID NO:76 |
| LD3707 (uS14 Forward) | AACCAGAGACCCTGGCTTTT | SEQ ID NO:77 |
| LD3708 (uS14 Reverse) | ACTTGGGAGGCTGAGACAGA | SEQ ID NO:78 |
| LD3709 (uL10 Forward) | TCGACAATGGCAGCATCTAC | SEQ ID NO:79 |
| LD3710 (uL10 Reverse) | ATCCGTCTCCACAGACAAGG | SEQ ID NO:80 |
| LD2045 (uS13 Forward) | GCAGCCATGTCTCTAGTGATCC | SEQ ID NO:81 |
| LD2046 (uS13 Reverse) | GGATCTTGTACTGGCGTGGA | SEQ ID NO:82 |
| LD3711 (eS26 Forward) | GAACGCATTTCCACCCTAGA | SEQ ID NO:83 |
| LD3712 (eS26 Reverse) | GCACGACCATTGTTCCTTCT | SEQ ID NO:84 |
| LD3713 (uL24 Forward) | AGGCATTTCAATGCACCTTC | SEQ ID NO:85 |
| LD3714 (uL24 Reverse) | CTGCACCCGTTCAATGTAGA | SEQ ID NO:86 |
| LD2841 (uL18 Forward) | GCAGGATGGGGTTTGTTAAA | SEQ ID NO:87 |
| LD2842 (uL18 Reverse) | ACGGGCATAAGCAATCTGAC | SEQ ID NO:88 |

(continued)

| Oligo probe name | RTqPCR primers | |
|---|---|---|
| LD3715 (uS8 Forward) | GACCTGGAAAAATGGCAGAA | SEQ ID NO:89 |
| LD3716 (uS8 Reverse) | CCAGAGTCCATGAGGCATTT | SEQ ID NO:90 |
| LD2005 (uS17 Forward) | AAGATGGCGGACATTCAGAC | SEQ ID NO:91 |
| LD2006 (uS17 Reverse) | TACCAGTGAAGGGGCATTTC | SEQ ID NO:92 |
| LD3717 (P1 Forward) | GGTCCTTCCGAGGAAGCTAA | SEQ ID NO:93 |
| LD3718 (P1 Reverse) | AACATTTACACCGGCTGCTT | SEQ ID NO:94 |
| LD3719 (eS17 Forward) | ACAAGAAGAAACGCCTGGTG | SEQ ID NO:95 |
| LD3720 (eS17 Reverse) | AGTGCTGCTTCCTCCTGAAG | SEQ ID NO:96 |
| LD3721 (eL24 Forward) | CAAATTCCAGAGGGCCATTA | SEQ ID NO:97 |
| LD3722 (eL24 Reverse) | TTTGCTTAGGTGCTGCCTTT | SEQ ID NO:98 |
| LD2857 (uS4 Forward) | GATTACATCCTGGGCCTGAA | SEQ ID NO:99 |
| LD2858 (uS4 Reverse) | CGCAGAGAGAAGTCGATGTG | SEQ ID NO:100 |
| LD3723 (uL14 Forward) | CTGACAACACAGGAGCCAAA | SEQ ID NO:101 |
| LD3724 (uL14 Reverse) | ACACGCCATCTTTTCTACGG | SEQ ID NO:102 |
| LD3725 (eS19 Forward) | TCTCCACCACTGTTCCTTCC | SEQ ID NO:103 |
| LD3726 (eS19 Reverse) | GGTGTCTAGTGAGGGGTGGA | SEQ ID NO:104 |
| LD2845 (uL16 Forward) | AGCAAGGGTAGGTGTGCATC | SEQ ID NO:105 |
| LD2846 (uL16 Reverse) | AGACCTTTGGTCAGGTGGTG | SEQ ID NO:106 |
| LD3727 (eS21 Forward) | GCTGCTTCCTTTCTCTCTCG | SEQ ID NO:107 |
| LD3728 (eS21 Reverse) | GCCTGTGACCTTGTCAACCT | SEQ ID NO: 108 |

**p53 steady-state level analysis**

For p53 steady-state analysis, we used a colon carcinoma cell line (HCT116) expressing p53 (39). For quantitative Western-blot analysis, HCT116 cells were depleted three times independently with one siRNA specific to transcripts encoding each r-protein. The transfection protocol used was similar to the one described above in the rRNA processing analysis section. For total protein extractions, cells from 6-well plates were first detached with 300 μl of trypsin-EDTA (ATCC) and pelleted at 100 g for 10 min at RT. Cells were washed in 1 ml of cold PBS and pelleted again at 100 g for 10 min at RT. Cells were then lysed in 30 μl of lysis buffer (Tris-HCl pH 8.0, 20 mM; NP40, 0.5%; NaCl, 150 mM; EDTA, 1 mM, protease inhibitor-Roche) during 15 min on ice. Lysed cells were then centrifuged at 20,000 g for 10 min at 4°C and supernatants were recovered from the pellet of cellular debris. As controls, we used the non-targeting scramble siRNA and an antisense oligonucleotide targeting the U8 snoRNA (IDT) (Table 2). Forty μg of total protein were separated on a 4-12 % polyacrylamide gel (Novex, Life Technologies, Bolt Bis-Tris Plus) and transferred on low-fluorescence PVDF membrane (Immobilon-FL, Millipore) according to the manufacturer protocol. The membranes were blocked in Odyssey blocking buffer (Li-Cor) for 1 hour at RT. Primary antibodies (1:4,000 anti-$\beta$-actin, Santa Cruz, SC69879; and 1:1,000 anti-p53, Bethyl Laboratories, A300-247A) were added to the Odyssey blocking buffer supplemented with 0.2% Tween-20 (Sigma) and membranes were incubated overnight at 4°C with agitation. Membranes were washed three times in TBS supplemented with 0.1% Tween-20 (TBS-T). Secondary antibodies carrying fluorescent dyes (1:2,000 DyLight 550 anti-mouse, Thermo Scientific, 84540; and 1:2,000 IRDye 680 anti-rabbit, Westburg, 926-68071) were added to Odyssey blocking buffer supplemented with 0.1% SDS and 0.2% Tween-20 and membranes were incubated 1 hour at RT with agitation. Membranes were washed three times in TBS-T before imaging of the fluorescent signals with the Chemidoc (Biorad). Cellular p53 steady-state level was assessed by calculating a ratio between the red fluorescent signal (corresponding to p53) and the green fluorescent signal (corresponding to $\beta$-actin). For each experiment, two independent lanes corresponding to HCT116 cells treated with the SCR siRNA were loaded on the gel, and the results from these two lanes were averaged to determine the level of p53 in this control condition. All data were then harmonized to this averaged value in order to determine the variation in the p53 steady-state level under this reference condition. Examples of uncropped Western-blots are shown in Supplementary Fig. 13. In Supplementary Fig. 9, the Western-blots were performed according to the same protocol, except that the gels were transferred onto PVDF (Amersham Hybond-P, RPN303F), and revealed with an HRP-conjugated secondary antibody (Santa Cruz) and the Supersignal WestPico chemiluminescent ECL substrate (Thermo Scientific). The anti-p21 antibody was purchase from Cell signalling (2947S).

For forty-eight r-proteins whose depletion did not affect the p53 level, the residual mRNA level was established by RTqPCR (see Supplementary Fig. 8). Reverse transcription was performed with the qScript cDNA supermix (Quanta Biosciences). qPCR was performed on a StepOne Plus Real-Time PCR machine (ThermoFisher Scientific) with specific primer pairs (Supplementary Table 3) and the perfecta SYBR green supermix (Quanta Biosciences). Each reaction was performed in triplicate. The residual level of mRNA was normalized to that of GAPDH and expressed with respect to that observed in cells treated with a non-targeting siRNA control (SCR).

Example 2: Image processing analysis

In this Example, we describe our procedure to extract qualitative and quantitative morphometric information from nucleoli using a low dimensional feature vector to provide a statistically validated tool to discriminate between populations of normal and altered nucleoli.

**Image processing and iNo index**

Example 2 presents the methodology for distinguishing populations of normal and altered nucleoli, based on statistical morphometric information. Shape and textural features were first derived to characterize nucleolar morphology in individual cell nuclei, so as to distinguish normal from altered nucleoli morphology in FIB-GFP images. Each feature was systematically defined as a parametric function, so that its parameters could be optimized over the entire database to maximize Fisher's criterion computed between the distributions of the features observed in r-protein-depleted cells and SCR-treated control cells. Given these features, we then performed a quantitative analysis of differences between their statistical distributions in a population of r-protein-depleted cells, compared to their distributions in a reference population. For this, we introduced a so-called discrepancy vector, each component of this vector being associated with a specific feature, and measured the distance between the distribution observed for a population of cells depleted of a given r-protein and that observed for a reference population of cells (SCR-treated cells). We then defined the index of nucleolar disruption, iNo, as the L1-norm of the discrepancy vector. This index reflects the degree of severity of nucleolar disruption, i.e. it ranks the r-proteins according to their impact on nucleolar structure. Additionally, Principal Component Analysis (PCA) of the discrepancy vectors was used to extract and visualize the major trends affecting the morphology of the nucleolus upon gene product depletion. PCA assumes linear embedding for dimensionality reduction and allows unsupervised clustering of the nucleolar disruption phenotypes. The computer code is described in the Supplementary information section and available upon request.

**Overview**

First, we describe how individual cell nuclei are segmented within each image of the database, in order to localize individual cells nucleoli. Then, we present the methodology adopted to derive a small set of shape and textural features that characterize the nucleolar morphology in each individual cell nucleus. Finally, we present a quantitative analysis of the differences observed between the distributions of these features in populations of cells depleted for specific gene products and the ones of a reference population. This leads us to use dimensionality reduction techniques, to stratify and rank the r-proteins according to their impact on the nucleolar structure. The stratification is based on a Principal Components Analysis (PCA) of the five distances ($d_k$, $k \leq 5$) measured between the distributions observed for a population of cells depleted for a given r-protein and those observed for a reference population of cells (SCR-treated cells), each distribution being associated to a specific shape/texture feature. For the ranking, we introduce an index of nucleolar disruption, or iNo, corresponding to the sum of the five absolute $|d_k|$ values.

**1. Cell nuclei segmentation**

As an initial step to delimit cell nucleoli from individual cells we segmented the cell nucleus. Each cell has a single nucleus. The nucleoli are specialized subnuclear domains, which, by definition, are all contained within the nucleus. Segmenting the nucleus, which is dense, compact and easy to score, is thus a mean to delimit the cellular volume that contains nucleoli in each individual cell.

Nuclei are stained with the DNA stain DAPI. The nuclei contours are extracted from the DAPI channel (blue) in two steps. Firstly, large connected components of relatively high intensity are identified using an original adaptive thresholding method. Secondly, the connected components that show significant concavity, indicating they likely correspond to aggregated nuclei, are rejected (Supplementary Fig. 14). Step 1 consists in a stepwise thresholding of the DAPI images such that nuclei corresponding to sufficiently large connected components of pixels lying above an intensity threshold are selected. Although all parameters of samples preparation (cell seeding, transfection procedure, DAPI staining, cell fixation, etc.) and of image capture (illumination, exposure time, etc.) are fully standardized and automatized, we observed an inherent variability in the DAPI signal intensity of individual nuclei. Supplementary Fig. 15 compares two extreme cases of such variability in panels (a) and (b). To address this we adopt a hierarchical thresholding strategy that progressively refines the segmentation by considering a sequence of K increasing thresholds, while exploiting prior knowledge about the size range of human cell nuclei. Detailed nuclei segmentation pseudo-code is provided below.

**Inputs**:

$I$: image

$\lambda_1 < \lambda_2 < \cdots < \lambda_k < \cdots < \lambda_K$: thresholds on intensity

$S$: minimal area threshold for eligible connected component

$S_{max}$: maximal nucleus area

$\partial I$: boundary of the image $I$ (first and last rows and columns)

**Output**:

$C$: set of connected components associated to segmented nuclei

$I_1 \leftarrow I > \lambda_1$

$D \leftarrow connected\_components(I_1)$

$C_1 \leftarrow \{d \in D \mid area(d) > S\}$

**For** $k = 2 \cdots K$

$\quad I_k \leftarrow I > \lambda_k$

$\quad D \leftarrow connected\_components(I_k)$

$\quad C_k \leftarrow \emptyset$

$\quad$**For all** $c \in C_{k-1}$

$\quad\quad L(c) = \{d \in D \mid d \subset c\}$ $\quad$ % connected components in $c$

$\quad\quad L_S(c) = \{d \in L(c) \mid area(d) > S\}$ % components in $L(c)$ larger than $S$

$\quad\quad$**If** $\left(\left((|L(c)| == 1) \wedge (|L_S(c)| == 1) \wedge \left(\frac{mean\_intensity(L_S(c))}{mean\_intensity(c)} > 1.15\right)\right) \vee (|L_S(c)| \geq 2)\right)$

$\quad\quad$ % one big connected component with important gain in intensity

$\quad\quad$ % OR at least two big connected components

$\quad\quad\quad C_k \leftarrow C_k \cup L_S(c)$

$\quad\quad$**Else** % keep $c$

$\quad\quad\quad C_k \leftarrow C_k \cup \{c\}$

$\quad\quad$**End if**

$\quad$**End for all**

**End for**

% Remove the connected components that are too big or that touch the image boundary

$C \leftarrow \{c \in C_K \mid (area(c) < S_{max}) \wedge is\_empty(c \cap \partial I)\}$

The image processing code was programmed in MatLab. The 3-D models of ribosomal subunits were generated with Pymol v1.5.0.3, the images for microscopy illustrations produced with ImageJ (http://imagej.nih.gov/ij/), and the graphs generated and analyzed with Prism.

In short, let $\lambda_k$ denote the $k^{th}$ intensity threshold, with $0 < k \leq K$, and $\lambda_k < \lambda_{k+1}$, $\forall$ $k < K$. Let $I_k$ denote the thresholded binary image, i.e. Ik (x) = 1 if I (x) > λk, and 0 elsewhere, with x ∈ [1,H] x [1,W], H and W denoting the height and width of the image, respectively. We also introduce $C_k$ to denote the set of sufficiently large (compared to a size threshold S) connected components at step k. At initialization, $C_1$ includes the connected components in image $I_1$ that are larger than S. The set $C_{k+1}$ of connected components at step k+1 is then derived iteratively from $C_k$ and $I_{k+1}$, as

follows. $C_{k+1}$ is initialized to the empty set. For each connected component $c \in C_k$, we considered the list L(c) of connected components in $I_{k+1}$ that are included in c. If L(c) includes at least two connected components with sizes larger than S, then those connected components are added to $C_{k+1}$. If the list L(c) includes a single connected component c' larger than S, then either c' or c are added to $C_{k+1}$, depending on whether the gain in mean intensity between c and c' is larger or smaller than 15%, respectively. Note that a higher gain in mean intensity reveals a more accurate segmentation, and that the value of 15% was set empirically. If the list L(c) does not include any component larger than S, then c is added to $C_{k+1}$. The connected components in $C_K$ that are smaller than a threshold $S_{max}$ are expected to reasonably segment the nuclei. Practically, the threshold S and $S_{max}$ have been set to 500 and 5000 pixels, so as to include most of the size range of human cell nuclei. Regarding $\square_k$, we considered a sequence of thresholds increasing from 250 to 450 by steps of 20, and from 500 to 1300 by steps of 100. The resulting nuclei segmentation appeared to be relatively independent of the actual sequence used, as long as its range and granularity were sufficient.

As a second step, we consider the rejection of the connected components that likely correspond to multiple nuclei in $C_K$. For this, we analyzed the convexity of each connected component (Supplementary Fig. 14). Specifically, a number of lines are drawn in parallel to the two principal axis of the connected component. When the connected component is convex, either one or zero segment lies inside the contour, for all parallel lines. In contrast, when the connected component represents aggregated nuclei, it presents a strong concavity and there exist parallel lines that include two or more segments lying inside the contour. If one parallel line supports two sufficiently long inner segments that are separated by a sufficiently long outer segment, the connected component is rejected. In Supplementary Fig. 14, the length of the outer and inner segments respectively correspond to $\delta_{out}$, $\delta^1_{in}$, and $\delta^2_{in}$. Those lengths have to be larger than a threshold of 5 pixels to reject the component. The threshold value has been set empirically to drastically reduce the number of multiple nuclei while keeping most of the single nuclei, compared to a manually generated ground truth. C denotes the subset of $C_K$ that includes all and only all non-rejected components.

Among the nuclei segmented in C, we were only really interested to analyze further those of cells in interphase. To identify them, the DAPI image and the distribution of FIB-GFP (green channel) in the segmented component are considered. A cell is considered to be in interphase if the DAPI is sufficiently dense and spread (if at least 50% of the pixels in the nucleus have a normalized DAPI value larger than 0.47, with the DAPI image being normalized by its maximal value.), and if the FIB-GFP is sufficiently localized (if at least 50% of the pixels in the nucleus have a normalized FIB-GFP value lower than 0.53, with the FIB-GFP image being normalized by its maximal value). The thresholds were set on the basis of a manual ground truth annotation of the images. These thresholds are quite stringent and their use results in the loss of a small fraction of cells in interphase, however our aim to only consider cells in interphase for further analysis is successfully achieved.

Finally, as a post-processing step, to ascertain all nucleoli of a cell are contained in each segmented regions, we apply basic mathematical morphology to close and enlarge each connected regions. Specifically, a dilation by a 13 x 13 structuring element was followed by a 3 x 3 erosion.

As depicted in Supplementary Fig. 15, our proposed method segments nuclei effectively, both in highly (panel a) and weakly (panel b) contrasted DAPI images. In this figure, the set of connected components that segment interphase nuclei are depicted in red. A reasonable detection rate was achieved, in conjunction with a very small false positive rate. This result is well suited to our needs since we are not interested in detecting all nuclei but rather in collecting a sufficient number of representative nucleoli patterns samples from each FIB-GFP image.

**2. Nucleoli image features**

This section introduces the image features that we consider to discriminate normal and altered nucleoli morphology in FIB-GFP images. For each segmented nucleus, its FIB-GFP signal is normalized by a percentile of 99.9% and all the features presented in this section are computed on this normalized signal. Section 2.1 introduces a number of original parameterized image features to measure the most significant visual differences observed in a set of representative nucleoli. Section 2.2 optimizes the parameters of those features, so as to maximize the discrimination between the distributions of the features in r-proteins-depleted cells and SCR-treated control cells.

2.1 Discriminant nucleolar morphometric features

The distribution of the nucleolar masses within a cell nucleus soon appeared to be subject to important stochastic variability. This is well illustrated in Supplementary Fig. 16, showing digitally resected nucleoli from control cells (panel a) and from cells depleted of specific proteins of interest (panels b, c). In these, the spatial organization of the nucleolar masses with respect to the nucleus center or to its principal axis fluctuates substantially across the images of a given panel, and do not help in differentiating the images from each panel. Hence, any features that would measure how the nucleolar topology is defined in terms of the absolute and normalized position of its components are not relevant to our problem. For example, the popular object recognition approaches that define the object appearance in terms of the intensities and gradients observed on small patches defined by their size and location in a normalized image do not help (12-14). The same holds true regarding transport-based features (15-17), since they measure the discrepancy compared to a reference distribution of masses, which is not available here.

We therefore consider the definition of a set of ad-hoc features that are independent of the actual position of the nucleoli within the nucleus, while still reflecting the spatial spreading of the nucleolar masses. Those features have been defined based on a manually-selected set of image samples, depicting typical normal and abnormal nucleolus patterns (see Supplementary Fig. 16). The manual extraction of representative samples is required to derive features that are relevant to the problem at hand, i.e. to make sure that the set of investigated features are able to discriminate among the variety of nucleolus appearances. However, to avoid (over)fitting our investigated features to those manually annotated samples, in the rest of the section, each feature is systematically defined as a parametric function, so that its parameters can be optimized over the entire database to make the feature can differentiate between normal and gene-depleted cells images (see Section 2.2).

To derive our set of parametric features, Supplementary Fig. 16 presents a set of manually-selected cells nucleoli images that are representative of the appearance diversity in reference control cells, and in cells depleted of proteins of interest. Panel c (resp. b), shows that the nucleoli from cells depleted of specific proteins of interest (resp. nucleoli with very high level of disruption) are generally spread over large and often irregular shapes, which contrasts with the rather circular spot distribution observed in control cells (panel a).

In addition, the 3-D graphs depicted in Supplementary Fig. 17 reveal that the distribution of FIB-GFP intensity of normal nucleoli is smoother and less peaky than in nucleoli of cells depleted of a protein of interest. Those observations motivated us to use features that characterize: (i) the area of support, (ii) the shape regularity, and (iii) the variations of intensities, i.e. the texture, of the nucleolar GFP signal. Practically, for each nucleus, all our proposed features are defined with respect to the segmentation of the nucleolus masses into a set of disjoint connected components. This segmentation is obtained by FIB-GFP image thresholding, which means that a pixel is considered to be part of the nucleolus if its intensity lies above the threshold. For each feature, the segmentation threshold parameter is defined automatically according to the method proposed in the next section, so as to maximize the separation between the distributions of the features for SCR-treated control cells and for cells depleted of proteins of interest. Hence, the segmentation threshold is a feature parameter, and might vary from one feature to the other. Other feature parameters are optimized similarly, and are thus defined automatically, as described in the next section.

*Area of support:*

To characterize the area of support of the nucleolus, we first consider the size and number of connected components obtained after thresholding with a so-called area segmentation threshold $\tau_a$. Specifically,

- $AA_{lcc}$ measures the area of the largest connected component in the thresholded image, and
- $AN_{cc}$ denotes the number of connected components in the nucleus.

In addition, to characterize the sharpness of the intensity gradient along the frontier delimiting the nucleolar masses, we introduced a sharpness index AS that measures the ratio of the nucleus pixels that respectively lie above two thresholds $\tau_{i1}$ and $\tau_{i2}$, with $\tau_{i1} > \tau_{i2}$.

*Shape and texture:*

To characterize the shape and the texture of the nucleolus, we only consider the largest connected component obtained after segmentation, because the small-sized components naturally tend to reduce to single circular peaks, making the largest connected component more representative with respect to shape and texture.

Shape:

To quantify the nucleolus shape regularity, we adopt a shape segmentation threshold $\tau_s$, and consider three distinct shape factors to characterize the shape of the largest connected component in the segmented image. Each factor describes the shape independently of its size.

They are illustrated in Supplementary Fig. 18, and correspond to:

- The elongation shape factor $SE_{lcc}$, which is defined as the square root of the ratio of the two second order moments, $\lambda_1$ and $\lambda_2$, of the connected component c around its principal axes;
- The elliptical regularity factor $SR_{lcc}$, which is defined as the ratio between the area of the connected component, and the area of the smallest ellipse lying outside the connected component, and having the same center, the same principal axes, and the same elongation than the connected component.
- The concavity factor $SC_{lcc}$, which is defined as the ratio between the area of the connected component and the area of its convex hull.

Texture:

To characterize the nucleolar texture pattern, after having investigated without any success (data not shown) some conventional texture descriptors such as the local binary patterns (18), the region covariance (19), or the grey level aura matrices (20), we introduced a number of original scalar metrics to reflect the distribution of intensities inside the largest connected component segmented based on a texture segmentation threshold $\tau_t$. Those metrics are:

- The texture histogram low tail index $TH_{lcc}$, which measures the percentage of pixels that lie below some intensity threshold $\alpha$, while being located inside the erosion of the connected component by a 3 x 3 pixels structuring element21. An erosion is applied to the shape to get rid of the low intensity pixels lying on the border of the shape;
- The texture uplands index $TU_{lcc}$, which is defined to be the number of connected regions lying above a threshold $\beta$, while being inside the connected component;
- The texture peaks index $TP_{lcc}$, which is defined to be the number of local maxima in the connected component;
- The texture valleys index $TV_{lcc}$, which is defined to be the number of local minima in the connected component;
- The texture local minimum $TLM_{lcc}$, which is defined as the intensity of the smallest local minimum in the connected component;

As can be observed in Table 6 and Supplementary Fig. 19, those scalar features have reasonably distinct values for the representative images depicted in Supplementary Fig. 16. Supplementary Fig. 19 illustrates that the number of local minima (red dots) is generally more important in morphologically disrupted nucleoli (panel b and c) than in control nucleoli (panel a). We thus expect that they are appropriate to differentiate normal from altered nucleoli (see below).

Table 6. Histogram low tail index ($\tau$_t=100, $\alpha$=150), number of uplands region ($\tau$_i1=100,$\tau$_i2=200), and smallest local minimum ($\tau$_a=100) ($\infty$ indicates the absence of a local minimum) in the largest connected component of the texture segmented images derived from Supplementary Fig. 16. (a) nucleoli from 12 SCR-treated control cells, (b) nucleoli with high level of nucleolar disruption, (c) nucleoli from cells depleted of a protein of interest (see Supplementary Fig. 16).

| | | | | | |
|---|---|---|---|---|---|
| (a) | 1.22% | 12.57% | 2.83% | 1.20% | 1.40% |
| | 2 | 1 | 1 | 1 | 1 |
| | 200 | $\infty$ | $\infty$ | 216 | 240 |
| | 6.02% | 4.05% | 0% | 9.12% | 5.05% |
| | 2 | 1 | 1 | 3 | 1 |
| | $\infty$ | $\infty$ | $\infty$ | 173 | $\infty$ |
| (b) | 31.51% | 27.72% | 51.30% | 29.93% | 4.93% |
| | 1 | 8 | 5 | 7 | 6 |
| | 104 | 101 | 112 | 142 | 190 |
| | 29.58% | 11.17% | 8.57% | 20.83% | 7.78% |
| | 5 | 2 | 2 | 2 | 7 |
| | $\infty$ | $\infty$ | $\infty$ | $\infty$ | 140 |
| (c) | 22.42% | 0% | 2.18% | 6.83% | 11.21% |
| | 6 | 1 | 1 | 3 | 10 |
| | 132 | $\infty$ | 165 | 162 | 118 |
| | 22.51% | 1.77% | 4.78% | 6.17% | 14.55% |
| | 5 | 2 | 4 | 1 | 4 |
| | 111 | $\infty$ | 159 | 149 | 160 |

22. Supervised optimization of features parameters

This section explains how the parameters involved in the definition of the above features are selected to best discriminate between normal and altered nucleoli.

Following the Fisher's criterion introduced by the popular Linear Discriminant Analysis (LDA) (22), we propose to select those parameters so as to maximize the separation between the features distributions that we want to discriminate.

In short, the Fisher's optimization criterion considers the problem of estimating whether a feature can discriminate between two classes of data, knowing the feature values for a representative set of data samples from each class. A natural step to answer this question consists in looking at the average (or the mean) of the feature values from each class. Intuitively, the closer the means are, the less discriminant the feature is. This is because a large distance between the means implies that the gap between the classes is expected to be large in the corresponding feature space. However, before drawing a conclusion about this gap, we also need to account for the spreading of the features around their respective mean, so that we can decide whether a given distance between the means is significant or not. Based on this reasoning, Fisher has defined the separation between the distributions associated to two classes of observations to be the ratio of the squared distance between the means to the sum of the variance within each class (22). We adopted the same criterion to optimize the parameters of our features. In other words, a discriminant feature is one for which the class-means are well separated, measured relative to the (sum of the) variances of the data assigned to a particular class.

Formally, considering a feature, parameterized by a vector p lying in a parameter space P, we assume that the feature distributions are known as a function of p, for the two classes of observations that we want to best discriminate. Then, the Fishers' optimization criterion informs us that the vector p* that maximizes the separation between the class distributions is defined as:

$$p^* = \operatorname*{argmax}_{p \,\in P} \frac{[\mu_1(p) - \mu_2(p)]^2}{\sigma_1(p)^2 + \sigma_2(p)^2} \quad,$$

where $\mu_1(p)$, $\mu_2(p)$ and $\sigma_1(p)$, $\sigma_2(p)$ respectively denote the means and standard deviations of the distributions of the feature of interest, measured with parameter p for the two classes. This Fisher's criterion is equivalent to the Welch's adaptation of the t-test (23), widely used in image-based morphometry (24).

Since it relies on the distributions of features that are observed for the two classes to discriminate, our proposed parameter optimization method has to be supervised. In our case, we know by design of our experimental set up which cells images correspond to control and (gene-depleted) test cells. Hence, we can readily identify pairs of distributions that should be discriminated one from the other. Practically, we selected the parameters so as to maximize the sum of the separation measured between each pair of distributions extracted from SCR-treated control cells, and from cells depleted of a protein of interest.

Supplementary Fig. 20 presents the Fisher's optimization criterion for three different features, as a function of their associated parameter. We observe that the parameter selection significantly impact the discriminative power of the feature.

It is worth noting here that our methodology has been defined to limit the impact of supervision on the outcome of the data mining process presented in Section 3. Specifically, supervision is deliberately restricted to the independent selection of individual feature parameters, without being involved in how the resulting features will be combined in the next section, based on a strictly unsupervised approach. Moreover, by defining the features parameters to differentiate distributions of samples extracted from the same culture, we avoid biasing the selection of features induced by the exploitation of a class containing different kind of deviations compared to the reference class.

## 3. Nucleolar features distribution analysis

This section analyzes how the distributions of the features of nucleoli observed in SCR-treated control cells compare to those of nucleoli of cells depleted of a protein of interest. As a primary objective, we aimed at quantifying the degree of nucleolar disruption associated with the depletion of a specific protein, based on the analysis of the distribution of the features of the associated nucleoli. Therefore, we introduced a discrepancy vector. Each component of this vector is associated to a specific feature, and measures the separation between the reference distribution and the gene-depleted distribution of interest. We then defined the index of nucleolar disruption, or iNo, to be the L1-norm of the discrepancy vector. This allowed us to rank the degree of severity of nucleolar disruption.

As a second and complementary outcome, we analyzed the principal components among the set of discrepancy vectors, assuming linear embedding for dimensionality reduction. It allows for extracting and visualizing the major trends affecting the morphology of the nucleolus when it is subject to gene-depletion. This allowed us to regroup nucleolar disruption phenotypes in classes in an unsupervised fashion.

### 3.1. Discrepancy-based distribution characterization

A discrepancy vector is defined so as to summarize how the features distributions associated to a set of nucleoli differ from their corresponding reference distributions.

Formally, let $S_r$ and $S_k$ denote two sets of nucleoli images, respectively obtained from normal reference cells and from cells that have been subject to the $k^{th}$ gene depletion process, i.e. to the $k^{th}$ silencer. For a given image feature f, we define the discrepancy $d_k$ between set $S_k$ and the reference $S_r$ to be the ratio of the difference of the mean feature values on each set to the sum of their variance. The definition naturally extends to N features fi, $0 < i \leq N$, and the $i^{th}$ component of the discrepancy vector $d_k$ associated to the set $S_k$ writes

$$\boldsymbol{p}^* = \underset{\boldsymbol{p} \in P}{\operatorname{argmax}} \frac{[\mu_1(\boldsymbol{p}) - \mu_2(\boldsymbol{p})]^2}{\sigma_1(\boldsymbol{p})^2 + \sigma_2(\boldsymbol{p})^2} \quad,$$

with $\mu_k(i)$, $\mu_r(i)$ and $\sigma_k(i)$, $\sigma_r(i)$ denoting the means and standard deviations of the $i^{th}$ feature over sets $S_k$ and $S_r$, respectively.

### 32. L1 norm of discrepancy vectors

To quantify the disruption level associated with the depletion of a specific protein of interest, we defined an index of nucleolar disruption, or iNo, as the L1-norm of the discrepancy vector computed over the set of nucleoli images obtained from cells depleted for that given protein of interest (Fig. 1c).

Letting $S_k$ denote the set of nucleoli obtained upon treatment with silencer k, and N be the 11 features defined in Section 2.1, the nucleolus disruption index $\Delta k$ is measured as:

$$\Delta_k = \sum_{i=1}^{N} |d_k(i)|$$

### 3.3. Principal component analysis of discrepancy vectors

Principal components analysis (PCA) is an unsupervised method for dimensionality reduction. PCA is used to visualize the most important phenotypic classes observed in our work. PCA searches for directions in the data that have the largest variance, and subsequently projects the data onto it. Following such an approach, we obtained a lower dimensional representation of the data, which removes some of the 'noisy', supposedly less meaningful, directions.

Since we are interested to score the nucleolar disruption associated with the depletion of particular proteins of interest, we applied the PCA to the discrepancy vectors that capture the average trends associated with nucleoli in cells depleted with a specific silencer, and not to individual nucleolar feature vectors.

To facilitate the interpretation of the eigenvectors associated to the principal components, we only consider the 5 features that have the largest Fisher's score, i.e. which best discriminates normal and altered nucleoli. Those features are listed in the first column of Supplementary Table 7.

Table 7. Two most significant PCA vectors.

| Feature | Principal component | Second principal component |
|---------|---------------------|----------------------------|
| $AA_{Icc}$ | 0.4808 | -0.4864 |
| $SR_{Icc}$ | -0.5503 | -0.6191 |
| $TH_{Icc}$ | 0.3772 | 0.3789 |
| $TLM_{Icc}$ | -0.4470 | 0.2151 |
| $TV_{Icc}$ | 0.3521 | -0.4363 |

Fig. 1b presents a PCA scatter plot depicting the 2-D points obtained by projecting each discrepancy vector on the two most significant PCA components. We observed that the PCA analysis has successfully found linear combinations of the proposed features that separate out the ground truth clusters, corresponding to different levels of disruption and phenotypic classes. We indeed observe visually that the nature of the disruption changes depending on the position in the scatter plot.

Supplementary Table 7 presents the first two principal components, i.e. the two directions of maximal variability of the projected discrepancy vectors. It reveals the main trends in the disruption process.

From the signs of the components in the first vector, we learned that the dominant disruption process increases the area of the nucleolus (AAIcc), and reduces its elliptical regularity (SRIcc). It also increases the number of low intensity pixels in the segmented nucleoli (THIcc), as well as the deepness (TLMIcc) and number of local minima (TVIcc), which reflects the scattered nature of the nucleoli spread.

The second vector induces an opposite trend compared to the one induced by the first vector, except for the elliptical regularity (SRIcc) and for the histogram low tail index (THIcc). Hence, a positive second PCA coefficient tends to foster the decrease of elliptical regularity, while a negative second PCA coefficient mitigates it, compared to what would result from the single vector only. This is reflected in Fig. 1b by more regular and circular shapes of the nucleolus masses in case of negative second PCA coefficient. In contrast, a positive second PCA coefficient corresponds to a more severe disruption, with less regular shape than the one observed for nucleoli of similar size in absence of second PCA component.

It is well established in the literature that inhibition of RNA polymerase I (Pol I) leads to a very specific nucleolar morphology alterations referred to as "nucleolar segregation" or "nucleolar caps" (25). Such caps are for example observed when cells are treated with low doses of actinomycin D, or in our experimental set up, in cells depleted of the Pol I transcription factor TIF1A (Fig. 1b, caps are seen as tiny bright dots). As an illustration that the PCA analysis is a powerful method to classify in an unsupervised fashion distinct nucleolar disruption phenotypes, nucleoli of cells depleted of TIF 1A, which in agreement with the literature (25) have a markedly different nucleolar disruption phenotype by comparison to the other control or test cells, correspond to two magenta triangles totally isolated in the upper left corner of the graph and characterized by a negative first PCA component value, which is in contrast to most other magenta triangles corresponding to cells depleted for other test genes.

References specific for Example 2 and Supplementary Figures

1. Tafforeau, L. et al. The complexity of human ribosome biogenesis revealed by systematic nucleolar screening of Pre-rRNA processing factors. Molecular cell 51, 539-551 (2013).
2. O'Donohue, M.F., Choesmel, V., Faubladier, M., Fichant, G. & Gleizes, P.E. Functional dichotomy of ribosomal proteins during the synthesis of mammalian 40S ribosomal subunits. The Journal of cell biology 190, 853-866 (2010).
3. Robledo, S. et al. The role of human ribosomal proteins in the maturation of rRNA and ribosome production. Rna 14, 1918-1929 (2008).
4. Chen, S.S. & Williamson, J.R. Characterization of the ribosome biogenesis landscape in E. coli using quantitative mass spectrometry. Journal of molecular biology 425, 767-779 (2013).
5. Sykes, M.T. & Williamson, J.R. A complex assembly landscape for the 30S ribosomal subunit. Annual review of biophysics 38, 197-215 (2009).
6. Talkington, M.W., Siuzdak, G. & Williamson, J.R. An assembly landscape for the 30S ribosomal subunit. Nature 438, 628-632 (2005).
7. Mulder, A.M. et al. Visualizing ribosome biogenesis: parallel assembly pathways for the 30S subunit. Science 330, 673-677 (2010).
8. Jomaa, A. et al. Functional domains of the 50S subunit mature late in the assembly process. Nucleic acids research 42, 3419-3435 (2014).
9. Ferreira-Cerca, S. et al. Analysis of the in vivo assembly pathway of eukaryotic 40S ribosomal proteins. Molecular cell 28, 446-457 (2007).
10. Ferreira-Cerca, S., Poll, G., Gleizes, P.E., Tschochner, H. & Milkereit, P. Roles of eukaryotic ribosomal proteins in maturation and transport of pre-18S rRNA and ribosome function. Molecular cell 20, 263-275 (2005).
11. Gamalinda, M. et al. A hierarchical model for assembly of eukaryotic 60S ribosomal subunit domains. Genes & development 28, 198-210 (2014).
12. Viola, P. & Jones, M. Robust real-time object detection. IJCV 57, 137-154 (2004).
13. Dalal, N. & Triggs, B. Histograms of oriented gradients for human detection. IEEE International Conference on Computer Vision and Pattern Recognition (2005).
14. Felzenszwalb, P., Girshick, R.B., McAllester, D. & Ramanan, D. Object detection with discriminatively trained part-based models. IEEE Trans on PAM 32, 1627-1645 (2010).
15. Gangbo, W. & McCann, R. The geometry of optimal transportation. Acta Math 177, 113-161 (1996).

16. Haker, S., Angenent, S. & Tannenbaum, A. Minimizing flows for the monge-kantorovich problem. SIAM J. Math Analysis 35 (2003).

17. Wang, W., Slepcev, D., Basu, S., Ozolek, J.A. & Rohde, G.K. A linear optimal transportation framework for quantifying and visualizing variations in sets of images. Int. J. Computer Vision 101, 254-269 (2013).

18. Ojala, T., Pietikäinen, M. & Harwood, D. A comparative study of texture measures with classification based on feature distributions. Pattern Recognition 29, 51-59 (1996).

19. Tuzel, O., Porikli, F. & Meer, P. Region covariance: a fast descriptor for detection and classification. ECCV (2006).

20. Qin, X. & Yang, Y.-H. Similarity measure and learning with gray level aura matrices (GLAM) for texture image retrieval. CVPR (2004).

21. Serra, J. Image analysis and mathematical morphology. (1982).

22. Fisher, R.A. The use of multiple measurments in taxonomic problems. Annals of Eugenics 7, 179-188 (1936).

23. Welch, B.L. The generalization of "Student's" problem when several different population variances are involved. Biometrika 34, 28-35 (1947).

24. Wolfe, P. et al. Using nuclear morphometry to discriminate the tumorigenic potential of cells: a comparison of statistical methods. Cancer Epidmiol Biomarkers Prev 13, 976-988 (2004).

25. Hernandez-Verdun, D., Roussel, P., Thiry, M., Sirri, V. & Lafontaine, D.L.J. The nucleolus: structure/function relationship in RNA metabolism. Wiley interdisciplinary reviews. RNA 1, 415-431 (2010).

Example 3 - not covered by the claims: Effects of r- protein depletion on nucleolar structure

Human cells stably producing the nucleolar methyltransferase fibrillarin (FBL) fused to a green fluorescent protein (GFP) were transfected with siRNAs targeting the appropriate transcripts, incubated for 3 days, and imaged by fluorescence microscopy (see Methods). Each r-protein was depleted in three experiments, a different siRNA being used in each experiment. The entire screen was duplicated. A non-targeting siRNA (SCR), mock-treated cells (MOCK), and a calibration set were included (see Methods and Supplementary Fig. 1). The calibration set consisted of proteins whose depletion leads to moderate to severe nucleolar disruption, formation of nucleolar "caps" (see below), or a reduction in fluorescence intensity (Supplementary Fig. 1).

To characterize nucleolar morphology defects both qualitatively and quantitatively, we developed a specific image-processing algorithm (see Example 2). Briefly, we first segmented the observed nuclei on the basis of shape- and size-consistent adaptive thresholding of a nuclear stain (DAPI signal). Then, within each nuclear mass, GFP signal thresholding and mathematical morphology (see Example 1) were applied to segment nucleoli into connected components. In order to optimize discrimination of nucleoli of cells depleted of an r-protein from those of SCR-treated control cells, five shape and textural features were extracted from the largest connected components (LCC) of each nucleolus. These five features, selected from a set of eleven as the most discriminant ones, were: area, elliptical regularity, percentage of pixels below an optimized intensity threshold, smallest intensity, and number of local minima (see Methods). For each of the five features, a $d_k$ value corresponding to a statistically significant distance between the feature distribution in cells depleted of an r-protein and control cells was computed. Each population of cells was thus characterized by five $d_k$ values. Principal component analysis (PCA) was used to reduce these five dimensions to two, allowing ready visualization of the data in a scatter plot (Fig. 1b) where each dot corresponds to a population of cells treated with one siRNA.

The PCA revealed groups of proteins whose depletion leads to similar nucleolar morphological phenotypes (Fig. 1b). Four major groups emerged. The largest one, indicated by a gray ellipse containing the SCR control (shown as a red dot), comprises r-proteins whose depletion had no significant impact on nucleolar structure. Importantly, most of the r-proteins are in this group, i.e. nearly all of the SSU proteins (shown in green) and roughly two-thirds of the LSU proteins (in magenta). A second group, beneath the SCR control, comprises proteins whose depletion did not alter the nucleolar structure but reduced the fluorescence intensity (e.g. control cells treated with siRNAs against GFP or FBL, see also Supplementary Fig. 1). Cells depleted of the RNA Pol I transcription factor TIF1A formed distinctive "nucleolar caps", in keeping with the known effects of RNA Pol I inhibitions (14), and appeared isolated in the upper left part of the graph. The fourth group comprises the few r-proteins whose depletion was found to impact nucleolar structure very severely, remarkably they are almost exclusively LSU proteins. This cluster forms a tail in the right part of the graph. In cells depleted of these major contributors to normal nucleolar structure, the nucleoli were detected as "unfolded beaded necklaces" (Fig. 1b). Our automated classification was benchmarked with a manual one and found to be extremely robust (Supplementary Fig. 2).

To stratify the r-proteins according to the severity of nucleolar disruption caused by their absence, we defined an index of nucleolar disruption (iNo) as the sum of the absolute values of the five $d_k$ distances (see Methods). For each r-protein, an average iNo, based on the values obtained with the three different siRNAs used, was calculated and plotted. In the resulting graph, the r-proteins are listed from top to bottom in the order of increasing impact on nucleolar structure (Fig. 1c). As concluded from the PCA, depletion of most r-proteins appears to have no significant impact on nucleolar structure (iNo <0.05), and the proteins whose depletion has the greatest effect belong to the LSU (magenta). Unexpectedly, the r-proteins uL5 (formerly RPL11, (24)) and uL18 (formerly RPL5) appear among the strongest contributors to maintenance of nucleolar structural integrity (Fig. 1c). These are precisely the proteins which, together with the 5S rRNA, form a small ribonucleoprotein complex, the 5S RNP, which acts as an HDM2 trap and controls the steady-state level of p53 in a regulatory circuit known as p53-dependent anti-tumor nucleolar surveillance (25, 26). Briefly, in unstressed cells, p53 is constitutively targeted for proteosomal degradation by Hdm2-mediated ubiquitination. In the event of a nucleolar stress, such as a ribosome biogenesis dysfunction, unassembled ribosomal components accumulate. These include the 5S RNP, which interacts with Hdm2, sequestering it away from p53. As a result, p53 is stabilized and induces cell cycle arrest and cell death (9). In mature 60S subunits, the 5S RNP constitutes the central protuberance (CP), a late-assembling structure (see below).

Ribosomal subunit assembly is a sequential process involving progressive binding of r-proteins to nascent rRNAs and gradual formation of ribosomal landmarks (23, 27-31). We wondered if the r-proteins important for nucleolar structure might map to particular areas on mature ribosomal subunits. Color-coding of the r-proteins according to their iNo values, on a 3-D model based on the crystal structure of the human ribosome32 (Fig. 2a), made it obvious that the strongest contributors to nucleolar structure maintenance belong to the LSU and are not randomly distributed over it: rather, they are preferentially located at the subunit interface in areas corresponding to the CP, the L1-stalk, and a region directly below the L1-stalk (Fig. 2a). All of these are late-forming structures (see below).

The nucleolus is a highly dynamic structure capable of responding through profound morphological alterations to cellular stresses such as drug treatment or viral infection (20). In interphase, however, it is quite stable. It is disassembled at the onset of mitosis and reassembled at the end of this process (14). In our nucleolar screens, cells were imaged after three days of r-protein depletion, as we reasoned that cells might have to undergo at least two cycles of nucleolar breakdown/nucleolar genesis for nucleolar alterations to become readily detectable. This assumption was confirmed when we established the time course of the appearance of nucleolar morphological defects (Supplementary Fig. 3). Focusing on thirteen representative r-proteins, and monitoring changes at 24-h intervals over a 3-day depletion period, we indeed found nucleolar disruption to increase steadily (Supplementary Fig. 3b), in parallel with an increase in iNo values. Nucleolar disruption became obvious only after 72 h of depletion (Supplementary Fig. 3a).

The nucleoli of cells of amniotic organisms have three nucleolar subcompartments (12,13,33). In our original screens, we used FBL, a dense fibrillar component (DFC) marker, to assess nucleolar morphology. To extend our conclusions, we examined whether nucleolar structural defects due to r-protein depletion might be equally observable with a marker of a different nucleolar subcompartment. We chose to monitor by immunofluorescence a granular component (GC) marker, the PES1 antigen, in depletion experiments focusing on thirteen representative r-proteins (Supplementary Fig. 4). As expected for a GC protein, PES1 staining was peripheral to the FBL signal (Supplementary Fig. 4b). Remarkably, we observed extreme closeness between the iNo scores computed from the FBL and PES1 signals, and the ranking of r-proteins according to phenotype severity was largely similar (Supplementary Fig. 4a). We conclude that the nucleolar structural defects due to r-protein depletion can be monitored similarly with a DFC or a GC antigen.

We conducted our nucleolar screens in HeLa cells because of the large size of their nucleus, which makes them ideal for use in high-throughput screens with visual readouts (e.g. 34, 35). To see how general the effects observed in HeLa-GFP-FBL cells might be, we tested five cell lines: two cervical carcinoma cell lines (HeLa-GFP-FBL and HeLa), one colon carcinoma cell line (HCT116), and two lung cancer cell lines (A549 and H1944). We selected eight representative r-proteins, depleted them for three days in each of the five cell lines, and monitored nucleolar structure by immunostaining of endogenous PES 1 and iNo score computation (Fig. 3). For the r-proteins tested, we found the weak and strong contributors to nucleolar structure maintenance to be largely the same in all five cell lines (Fig. 3), with uL5 and uL18 playing an important role in each case.

Example 4 - not covered by the claims: Effects of r-protein depletion on pre-rRNA processing

In an attempt to correlate the effects of r-protein depletion on nucleolar structure with defects in ribosome biogenesis, we determined which r-proteins are essential to pre-rRNA processing (Fig 4). Mature rRNAs are produced from long precursor molecules. They are embedded in non-coding spacers and require extensive processing to be generated (2, 36). Pre-rRNA processing analysis is a good proxy for ribosomal assembly analysis, because failure of an r-protein to bind to nascent ribosomes leads to ribosome biogenesis blockade, pre-rRNA processing inhibitions, and subunit biogenesis abortion (23, 30, 31, 37).

The synthesis of each of the eighty r-proteins was knocked down for 2 days in HCT116 cells with an appropriate siRNA. Total RNA was then extracted, run on a bioanalyzer, and analyzed by high-resolution quantitative northern blotting. Two different siRNAs were used for each r-protein and yielded largely similar results (Fig 4). As controls, we used UTP18 and NOL9 because their depletion leads to well-established pre-rRNA processing defects (Supplementary Figs 5,6,11; see (38)). As further controls, non-targeting siRNA (SCR) and mock-treated cells were used (Supplementary Figs 5,6). HCT116 and HeLa cells are both of epithelial origin and, as shown above, their nucleolar structure is similarly affected by r-protein depletion (Fig. 3). We performed our RNA processing work and p53 steady-state accumulation analysis (see below) on HCT116 cells because, unlike HeLa cells, they express p53 normally[39]. For the RNA analysis, cells were depleted for only 2 days, as we had established beforehand, precisely in HCT116 cells, that bona fide pre-rRNA processing inhibitions are early defects preceding cell-cycle arrest and apoptosis and are best captured at this time point (discussed in (38)).

The ratio of 28S to 18S mature rRNA was extracted from bioanalyzer electropherograms (Fig. 4a). The accumulation of small subunit 18S rRNA was strongly decreased, and the 28S/18S ratio accordingly increased, by SSU r-protein depletion (Fig. 4a). Reciprocally, LSU r-protein depletion led to decreased accumulation of the large subunit 28S rRNA and to a reduced 28S/18S ratio (Fig. 4a). Northern blots were probed with specific radioactively labeled oligonucleotides, revealing all major known pre-rRNA intermediates (Fig. 4b, Supplementary Figs. 5,6,11). Each band detected was quantified with a phosphoimager and normalized with respect to the SCR control. The signals were represented on heatmaps (Fig. 4c for SSU r-proteins, Fig. 4d for LSU r-proteins and Supplementary Fig. 11; see also (38)). The heatmaps were clustered with the software "R", revealing functionally related groups of r-proteins whose depletion affects similar processing steps (Fig. 4c,d, and Supplementary Figs S5,6 and Supplementary Fig. 11). For the SSU r-proteins, three groups emerged: proteins whose depletion affects early processing (class 1), late processing (class 3), or has no significant effect on processing (class 2)(Fig. 4c, see representative examples in Supplementary Fig. 5 and Supplementary Fig. 11 for a full dataset). Our classification of the SSU r-proteins corresponds largely to that previously established in HeLa cells (23). We identified four classes of LSU r-proteins (Fig. 4d and Supplementary Figs. 6,11): those whose depletion affects early cleavage steps (class 1), intermediate cleavage steps (class 3), late cleavage steps (class 4), or has no substantial impact on processing (class 2)(Fig. 4d and Supplementary Fig. 6). Importantly, no such classification of LSU r-proteins has been reported previously. Our classification of r-proteins' involvement in pre-rRNA processing thus confirms and largely extends previous work (Supplementary Fig. 7).

The r-proteins were mapped on a 3-D model of the human ribosome according to their involvement in processing (Fig. 2b). This revealed, on both subunits, a strikingly asymmetric distribution. On the mature small subunit, the r-proteins required for early processing steps are those forming the body and platform (Fig. 2b), both of which are known as early-assembling subunit structures (27,28,40). The r-proteins affecting late cleavage steps, in contrast, correspond to the head and beak (Fig. 2b), which are late-forming structures (27,28,40). On the large subunit, the r-proteins important for early processing are mainly exposed on the solvent side of the ribosome (in blue on the right-hand-side cartoon Fig. 2b), while those required for intermediate cleavages are at the interface side (in orange on the left-hand-side cartoon), below the L1 stalk, and those important for late processing correspond largely to the CP and L1-stalk (in red). Remarkably, this is precisely the order in which these structures have been shown to form in budding yeast (30).

A comparison of our nucleolar structure and rRNA processing data reveals that the r-proteins whose depletion has the greatest effect on nucleolar structure (in red in Fig. 2a) are largely those required for intermediate or late processing steps in the formation of the large ribosomal subunit (in orange and red in Fig. 2b). Within this subunit, they belong mostly to late-assembling structures, including the L1-stalk and CP (uL5 and uL18). In conclusion, while practically all r-proteins appear important for pre-rRNA processing, most of them have no incidence on the structural integrity of the nucleolus.

Several trans-acting factors, including BXDC1 and RRS1, are required for CP assembly (26,41). This function is conserved between yeast and human (26,41). Considering the strong effect of uL5 or uL18 depletion on nucleolar structure, and because both of these proteins are CP components, we predicted that depletion of factors involved in CP formation should also cause profound nucleolar structure alterations. This proved to be true: we found depletion of BXDC1, RRS1, or both to affect nucleolar structure severely (Fig. 5a), almost as strongly as does uL5 or uL18 depletion (Fig. 5b).

Example 5 - not covered by the claims: Effects of r-protein depletion on p53 steady-state levels

Given the numerous connections between p53 and ribosomal component synthesis on the one hand (42), and between the functional integrity of the nucleolus and p53 metabolic stability on the other (43), we examined systematically how depletion of each individual r-protein might affect the steady-state level of p53. Colon carcinoma cells expressing p53 (HCT116 p53+/+, (39)) were transfected with one siRNA targeting each r-protein transcript and incubated for 2 days. In this analysis we used a single siRNA, selected on the basis of its proven efficacy in the nucleolar and processing screens, and carried out depletion for 2 days to allow a direct comparison with the RNA analysis. Total protein was then extracted and analyzed by quantitative fluorescent western blotting (Fig. 6a and Supplementary Fig. 11).

The p53 steady-state level increase observed ranged from 0 to 10-fold (Fig. 6b). About a third of the r-proteins (24/80) were found to affect p53 level at least 5-fold (Fig. 6b, gray box), the cut-off we adopted arbitrarily as significance threshold. As observed for the effects on nucleolar structure, we found nearly all of these r-proteins to belong to the LSU, the sole exception being eS31. Interestingly, depletion of uL5 or uL18, involved in p53-dependent nucleolar surveillance (discussed above), had no significant impact on the p53 steady-state level, in keeping with previous reports (25,26,44) and in contrast to the role of these proteins in forming an Hdm2 trap when they accumulate in cells (44-46).

As an additional control we established by RTqPCR, for forty-eight r-proteins whose depletion did not significantly affect p53 accumulation (see Fig. 6b, from eL22 to eS21), the efficiency of r-protein depletion at the mRNA level (Supplementary Fig. 8). We found depletion to be effective for all the r-proteins tested, the residual mRNA level for most of them (40 out of 48) being below 20%. Note that 31 of the 48 candidates tested showed a marked processing defect upon depletion (see Fig. 4, Supplementary Figs S5,S6,S11), a further indication that depletion was efficient.

In view of the model of nucleolar stress above, we wondered if the significant increase in p53 observed upon depletion of 24 r-proteins might involve uL5 and uL18. We found this to be the case: co-depletion of any one of the 24 r-proteins and either uL5 or uL18 led to normal levels of both p53 and its transcriptional target p21 (Supplementary Fig. 9a). The effects of BXDC1 and RRS1 were also investigated. As expected from the role of these proteins as ribosome (CP) assembly factors, their depletion also caused p53 and p21 to increase, and this rise was dependent on uL5 and uL18 (Supplementary Fig. 9b).

Figure 2c shows the distribution of the r-proteins on mature subunits according to their impact on p53 expression. This shows that the significant contributors to p53 homeostasis all correspond to late-assembling structures on the subunits (Fig. 2b,c).

Table 5. Comparison between the effects of r- protein depletion on nucleolar structure integrity and p53 homeostasis

| SSU | iNo | p53 | LSU | iNo | p53 |
|---|---|---|---|---|---|
| eS1 (Rps3a) | 0.02/0.05 | 0.8 | uL1 (RpL10a) | 0.17/0.19 | 7.2 |
| uS2 (RpSA) | 0.02/0.05 | 1.0 | uL2 (RpL8) | 0.16/0.19 | 7.1 |
| uS3 (RpS3) | 0.01/0.04 | 1.3 | uL3 (RpL3) | 0.04/0.06 | 4.2 |
| uS4 (RpS9) | 0.02/0.04 | 2.2 | uL4 (RpL4) | 0.04/0.05 | 4.2 |
| eS4 (RpS4) | 0.02/0.03 | 1.1 | uL5 (RpL11) | 0.19/0.20 | 1.0 |
| uS5 (RpS2) | 0.02/0.03 | 1.1 | uL6 (RpL9) | 0.04/0.04 | 6.3 |
| eS6 (RpS6) | 0.02/0.04 | 0.6 | eL6 (RpL6) | 0.05/0.05 | 4.8 |
| uS7 (RpS5) | 0.03/0.04 | 0.8 | eL8 (RpL7a) | 0.02/0.03 | 7.2 |
| eS7 (RpS7) | 0.02/0.05 | 0.8 | uL10 (P0) | 0.03/0.04 | 1.7 |
| uS8 (RpS15a) | 0.03/0.05 | 1.9 | uL11 (RpL12) | 0.04/0.03 | 3.3 |
| eS8 (RpS8) | 0.02/0.03 | 1.0 | uL13 (RpL13a) | 0.07/0.07 | 8.5 |
| uS9 (RpS16) | 0.03/0.05 | 1.4 | eL13 (RpL13) | 0.03/0.05 | 8.0 |
| uS10 (RpS20) | 0.03/0.05 | 4.6 | uL14 (RpL23) | 0.02/0.04 | 2.2 |
| eS10 (RpS10) | 0.03/0.07 | 0.5 | eL14 (RpL14) | 0.04/0.04 | 10.0 |
| uS11 (RpS14) | 0.03/0.09 | 1.4 | uL15 (RpL27a) | 0.05/0.12 | 5.2 |
| uS12 (RpS23) | 0.02/0.03 | 0.5 | eL15 (RpL15) | 0.03/0.02 | 1.1 |
| eS12 (RpS12) | 0.01/0.04 | 0.9 | uL16 (RpL10) | 0.03/0.03 | 2.5 |
| uS13 (Rps18) | 0.03/0.06 | 1.7 | uL18 (RpL5) | 0.19/0.20 | 1.9 |
| uS14 (RpS29) | 0.05/0.06 | 1.6 | eL18 (RpL18) | 0.03/0.05 | 3.5 |
| uS15 (RpS13) | 0.02/0.11 | 0.9 | eL19 (RpL19) | 0.08/0.1 | 5.4 |
| uS17 (Rps11) | 0.02/0.04 | 2.0 | eL20 (RpL18a) | 0.05/0.05 | 6.2 |
| eS17 (RpS17) | 0.01/0.04 | 1.4 | eL21 (RpL21) | 0.11/0.16 | 8.3 |
| uS19 (RpS15) | 0.03/0.05 | 0.9 | uL22 (RpL17) | 0.05/0.06 | 8.2 |
| eS19 (RpS19) | 0.04/0.06 | 2.4 | eL22 (RpL22) | 0.02/0.02 | 0.5 |
| eS21 (RpS21) | 0.02/0.04 | 2.9 | uL23 (RpL23a) | 0.02/0.04 | 0.7 |
| eS24 (RpS24) | 0.02/0.04 | 0.5 | uL24 (RpL26) | 0.03/0.06 | 1.8 |
| eS25 (RpS25) | 0.02/0.04 | 3.2 | eL24 (RpL24) | 0.03/0.07 | 2.2 |
| eS26 (RpS26) | 0.01/0.05 | 1.8 | eL27 (RpL27) | 0.05/0.11 | 6.3 |
| eS27 (RpS27) | 0.03/0.03 | 2.1 | eL28 (RpL28) | 0.02/0.03 | 1.5 |
| eS27L (RpS27L) | 0.03/0.04 | 1.3 | uL29 (RpL35) | 0.06/0.04 | 5.0 |
| eS28 (RpS28) | 0.02/0.03 | 1.5 | eL29 (RpL29) | 0.03/0.03 | 1.0 |
| eS30 (RpS30) | 0.05/0.05 | 1.3 | uL30 (RpL7) | 0.06/0.06 | 5.1 |
| eS31 (RpS27a) | 0.03/0.03 | 6.9 | eL30 (RpL30) | 0.07/0.15 | 5.7 |
| RACK1 | 0.02/0.02 | 0.6 | eL31 (RpL31) | 0.05/0.09 | 1.1 |
| | | | eL32 (RpL32) | 0.03/0.05 | 4.8 |
| | | | eL33 (RpL35a) | 0.02/0.04 | 8.9 |
| | | | eL34 (RpL34) | 0.04/0.07 | 8.3 |

| eL36 (RpL36) | 0.03/0.04 | 5.5 |
|---|---|---|
| eL37 (RpL37) | 0.04/0.10 | 6.9 |
| eL38 (RpL38) | 0.08/0.11 | 8.9 |
| eL39 (RpL39) | 0.04/0.09 | 10.4 |
| eL40 (RpL40) | 0.04/0.08 | 3.2 |
| eL41 (RpL41) | 0.02/0.02 | 0.8 |
| eL42 (RpL36a) | 0.03/0.05 | 0.9 |
| eL43 (RpL37a) | 0.13/0.20 | 5.7 |
| P1 | 0.02/0.04 | 2.1 |
| P2 | 0.02/0.03 | 1.1 |

The r-proteins are listed according to a recently revised nomenclature (Ban et al, 2014, PMID 24524803). The former nomenclature is provided in parentheses. The index of nucleolar disruption (iNo score) ranges from 0 (normal nucleolus) to 0.2 (disrupted nucleolus) (see Fig 1C). The iNo values were computed from two independent screens (screen I/screen II). The p53 level is expressed in fold increase (from 0 to 10.4, see Fig 4B). Arbitrarily set significance cut-off: p53 fold increase $\geq 5$ ; iNo $\geq 0.1$. -, above cut-off; medium grey, below cut-off; white, curated "near cut-off" cases. SSU, small subunit r-protein; LSU, large subunit r-protein; iNo, index of nucleolar disruption. The iNo scores and p53 levels were established in epithelial human cells. The iNo scores were determined in HeLa-FBL-GFP cells after r-protein depletion for 3 days; the p53 levels in HCT116 p53 +/+ cells after r-protein depletion for 2 days.

No effect on nucleolar structure & no effect on p53 level: 50 r-proteins

No effect on nucleolar structure & p53 induction: 21 r-proteins

Effect on nucleolar structure & no p53 induction: 2 r-proteins (uL5 and uL18)

Effect on nucleolar structure & p53 induction: 8 r-proteins

uL5 and uL18 are unique in that, remarkably, they are the only two r-proteins, out of eighty, whose depletion impact strongly nucleolar structure without inducing p53.

**References related to embodiments not covered by the claims**

1. Boisvert, F.M., van Koningsbruggen, S., Navascues, J. & Lamond, A.I. The multifunctional nucleolus. Nature reviews. Molecular cell biology 8, 574-585 (2007).
2. Lafontaine, D.L.J. Noncoding RNAs in eukaryotic ribosome synthesis and function. Nature Structural and Molecular Biology 22, 11-19 (2015).
3. Steitz, T.A. A structural understanding of the dynamic ribosome machine. Nature reviews. Molecular cell biology 9, 242-253 (2008).
4. Melnikov, S. et al. One core, two shells: bacterial and eukaryotic ribosomes. Nature structural & molecular biology 19, 560-567 (2012).
5. Schmeing, T.M. & Ramakrishnan, V. What recent ribosome structures have revealed about the mechanism of translation. Nature 461, 1234-1242 (2009).
6. Danilova, N. & Gazda, H.T. Ribosomopathies: how a common root can cause a tree of pathologies. Dis Model Mech 8, 1013-1026 (2015).
7. Narla, A. & Ebert, B.L. Ribosomopathies: human disorders of ribosome dysfunction. Blood 115, 3196-3205 (2010).
8. Zhang, Y. & Lu, H. Signaling to p53: ribosomal proteins find their way. Cancer cell 16, 369-377 (2009).
9. Chakraborty, A., Uechi, T. & Kenmochi, N. Guarding the 'translation apparatus': defective ribosome biogenesis and the p53 signaling pathway. Wiley interdisciplinary reviews. RNA 2, 507-522 (2011).
10. Lam, Y.W., Evans, V.C., Heesom, K.J., Lamond, A.I. & Matthews, D.A. Proteomics analysis of the nucleolus in adenovirus-infected cells. Molecular & cellular proteomics : MCP 9, 117-130 (2010).

11. Moore, H.M. et al. Quantitative proteomics and dynamic imaging of the nucleolus reveal distinct responses to UV and ionizing radiation. Molecular & cellular proteomics : MCP 10, M111 009241 (2011).

12. Thiry, M. & Lafontaine, D.L.J. Birth of a nucleolus: the evolution of nucleolar compartments. Trends in cell biology 15, 194-199 (2005).

13. Lamaye, F., Galliot, S., Alibardi, L., Lafontaine, D.L.J. & Thiry, M. Nucleolar structure across evolution: the transition between bi- and tri-compartmentalized nucleoli lies within the class Reptilia. Journal of structural biology 174, 352-359 (2011).

14. Hernandez-Verdun, D., Roussel, P., Thiry, M., Sirri, V. & Lafontaine, D.L.J. The nucleolus: structure/function relationship in RNA metabolism. Wiley interdisciplinary reviews. RNA 1, 415-431 (2010).

15. Sirri, V., Hernandez-Verdun, D. & Roussel, P. Cyclin-dependent kinases govern formation and maintenance of the nucleolus. The Journal of cell biology 156, 969-981 (2002).

16. Leung, A.K. et al. Quantitative kinetic analysis of nucleolar breakdown and reassembly during mitosis in live human cells. The Journal of cell biology 166, 787-800 (2004).

17. Derenzini, M., Montanaro, L. & Trere, D. What the nucleolus says to a tumour pathologist. Histopathology 54, 753-762 (2009).

18. Bywater, M.J. et al. Inhibition of RNA polymerase I as a therapeutic strategy to promote cancer-specific activation of p53. Cancer cell 22, 51-65 (2012).

19. Peltonen, K. et al. A targeting modality for destruction of RNA polymerase I that possesses anticancer activity. Cancer cell 25, 77-90 (2014).

20. Boulon, S., Westman, B.J., Hutten, S., Boisvert, F.M. & Lamond, A.I. The nucleolus under stress. Molecular cell 40, 216-227 (2010).

21. Hein, N., Hannan, K.M., George, A.J., Sanij, E. & Hannan, R.D. The nucleolus: an emerging target for cancer therapy. Trends in molecular medicine 19, 643-654 (2013).

22. de la Cruz, J., Karbstein, K. & Woolford Jr, J.L. Functions of Ribosomal Proteins in Assembly of Eukaryotic Ribosomes In Vivo. Annual review of biochemistry 84, 93-129 (2015).

23. O'Donohue, M.F., Choesmel, V., Faubladier, M., Fichant, G. & Gleizes, P.E. Functional dichotomy of ribosomal proteins during the synthesis of mammalian 40S ribosomal subunits. The Journal of cell biology 190, 853-866 (2010).

24. Ban, N. et al. A new system for naming ribosomal proteins. Curr Opin Struct Biol 24, 165-169 (2014).

25. Donati, G., Peddigari, S., Mercer, C.A. & Thomas, G. 5S ribosomal RNA is an essential component of a nascent ribosomal precursor complex that regulates the Hdm2-p53 checkpoint. Cell reports 4, 87-98 (2013).

26. Sloan, K.E., Bohnsack, M.T. & Watkins, N.J. The 5S RNP couples p53 homeostasis to ribosome biogenesis and nucleolar stress. Cell reports 5, 237-247 (2013).

27. Chen, S.S. & Williamson, J.R. Characterization of the ribosome biogenesis landscape in E. coli using quantitative mass spectrometry. Journal of molecular biology 425, 767-779 (2013).

28. Talkington, M.W., Siuzdak, G. & Williamson, J.R. An assembly landscape for the 30S ribosomal subunit. Nature 438, 628-632 (2005).

29. Sashital, D.G. et al. A combined quantitative mass spectrometry and electron microscopy analysis of ribosomal 30S subunit assembly in E. coli. eLife 3 (2014).

30. Gamalinda, M. et al. A hierarchical model for assembly of eukaryotic 60S ribosomal subunit domains. Genes & development 28, 198-210 (2014).

31. Ferreira-Cerca, S. et al. Analysis of the in vivo assembly pathway of eukaryotic 40S ribosomal proteins. Molecular cell 28, 446-457 (2007).

32. Anger, A.M. et al. Structures of the human and Drosophila 80S ribosome. Nature 497, 80-85 (2013).

33. Thiry, M., Lamaye, F. & Lafontaine, D.L.J. The nucleolus: when 2 became 3. Nucleus 2, 289-293 (2011).

34. Schmitz, M.H. et al. Live-cell imaging RNAi screen identifies PP2A-B55alpha and importin-beta1 as key mitotic exit regulators in human cells. Nature cell biology 12, 886-893 (2010).

35. Wild, T. et al. A protein inventory of human ribosome biogenesis reveals an essential function of exportin 5 in 60S subunit export. PLoS biology 8, e1000522 (2010).

36. Mullineux, S.T. & Lafontaine, D.L.J. Mapping the cleavage sites on mammalian pre-rRNAs: where do we stand? Biochimie 94, 1521-1532 (2012).

37. Robledo, S. et al. The role of human ribosomal proteins in the maturation of rRNA and ribosome production. Rna 14, 1918-1929 (2008).

38. Tafforeau, L. et al. The complexity of human ribosome biogenesis revealed by systematic nucleolar screening of Pre-rRNA processing factors. Molecular cell 51, 539-551 (2013).

39. Bunz, F. et al. Requirement for p53 and p21 to sustain G2 arrest after DNA damage. Science 282, 1497-1501 (1998).

40. Sykes, M.T. & Williamson, J.R. A complex assembly landscape for the 30S ribosomal subunit. Annual review of biophysics 38, 197-215 (2009).

41. Zhang, J. et al. Assembly factors Rpf2 and Rrs1 recruit 5S rRNA and ribosomal proteins rpL5 and rpL11 into nascent ribosomes. Genes & development 21, 2580-2592 (2007).

42. Ruggero, D. & Pandolfi, P.P. Does the ribosome translate cancer? Nature reviews. Cancer 3, 179-192 (2003).

43. James, A., Wang, Y., Raje, H., Rosby, R. & DiMario, P. Nucleolar stress with and without p53. Nucleus 5 (2014).

44. Fumagalli, S., Ivanenkov, V.V., Teng, T. & Thomas, G. Suprainduction of p53 by disruption of 40S and 60S ribosome biogenesis leads to the activation of a novel G2/M checkpoint. Genes & development 26, 1028-1040 (2012).

45. Sun, X.X., Wang, Y.G., Xirodimas, D.P. & Dai, M.S. Perturbation of 60 S ribosomal biogenesis results in ribosomal protein L5- and L11-dependent p53 activation. The Journal of biological chemistry 285, 25812-25821 (2010).

46. Bursac, S. et al. Mutual protection of ribosomal proteins L5 and L11 from degradation is essential for p53 activation upon ribosomal biogenesis stress. Proceedings of the National Academy of Sciences of the United States of America 109, 20467-20472 (2012).

47. Lo, K.Y. et al. Defining the pathway of cytoplasmic maturation of the 60S ribosomal subunit. Molecular cell 39, 196-208 (2010).

48. Ferreira-Cerca, S., Poll, G., Gleizes, P.E., Tschochner, H. & Milkereit, P. Roles of eukaryotic ribosomal proteins in maturation and transport of pre-18S rRNA and ribosome function. Molecular cell 20, 263-275 (2005).

49. Ben-Shem, A. et al. The structure of the eukaryotic ribosome at 3.0 A resolution. Science 334, 1524-1529 (2011).

50. Khatter, H., Myasnikov, A.G., Natchiar, S.K. & Klaholz, B.P. Structure of the human 80S ribosome. Nature 520, 640-645 (2015).

51. Madru, C. et al. Chaperoning 5S RNA assembly. Genes & development 29, 1432-1446 (2015).

52. Leidig, C. et al. 60S ribosome biogenesis requires rotation of the 5S ribonucleoprotein particle. Nature communications 5, 3491 (2014).

53. Bassler, J. et al. A network of assembly factors is involved in remodeling rRNA elements during preribosome maturation. The Journal of cell biology 207, 481-498 (2014).

54. Yusupov, M.M. et al. Crystal structure of the ribosome at 5.5 A resolution. Science 292, 883-896 (2001).

55. Rubbi, C.P. & Milner, J. Disruption of the nucleolus mediates stabilization of p53 in response to DNA damage and other stresses. The EMBO journal 22, 6068-6077 (2003).

56. Fumagalli, S. et al. Absence of nucleolar disruption after impairment of 40S ribosome biogenesis reveals an rpL11-translation-dependent mechanism of p53 induction. Nature cell biology 11, 501-508 (2009).

## Claims

1. A computer implemented method for scoring nucleolar disruption , comprising determining morphometric characteristics of the nucleolar support area and the nucleolar intensity pattern;

   wherein nucleolar disruption is scored based on a nuclear stain image of eukaryotic cell or tissue images, preferably based on a nuclear stain image of population of cells;wherein said morphometric characteristics are

   - the nucleolus area of the largest nucleolus or largest nucleolar connected component in a cell,
   - the elliptical regularity factor, wherein the elliptical regularity factor is determined as the ratio between the area of the largest nucleolus or nucleolar connected component and the area of the smallest ellipse lying outside the largest nucleolus or nucleolar connected component and having the same center, the same principal axes, and the same elongation as the largest nucleolus or nucleolar connected component,
   - the texture histogram low tail index, wherein the texture histogram low tail index is defined as the percentage of pixels that lie below a pixel intensity threshold $\alpha$ of the largest nucleolus or largest nucleolar connected component in a cell,
   - the texture valleys index, wherein the texture valleys index is defined as the number of local minima in the nucleolus or nucleolar connected component of the largest nucleolus or largest nucleolar connected component in a cell, and
   - the texture local minimum, wherein the texture local minimum is defined as the intensity of the smallest local minimum in the nucleolus or nucleolar connected component of the largest nucleolus or largest nucleolar connected component in a cell;

   wherein the nucleolus is identified based on image pixel intensity thresholding as a connected component in the thresholded nuclear stain image,;
   wherein the nucleolar support area is determined based on an area segmentation threshold, wherein the nucleolar shape regularity is determined based on a shape segmentation threshold, and the nucleolar texture pattern is determined based on a texture segmentation threshold, each threshold being defined to maximize the discriminant power of corresponding morphometric characteristics;

wherein the nucleolar disruption is scored based on the discrepancy with respect to a standard or reference nucleolus;

wherein said discrepancy is determined as a discrepancy vector based on the distances between the distributions of each morphometric characteristic of a population of said nucleoli or nucleolar connected components and the distribution of the corresponding morphometric characteristic of a population of standard or reference nucleoli or nucleolar connected components, wherein the discrepancy vector component associated with a given morphometric characteristic (i) for a particular nucleolus or nucleolar connected component population d is preferably determined according to the following formula representing a discrepancy vector component:

$$d(i) = \frac{\mu(i) - \mu_r(i)}{\sqrt{\sigma^2(i) + \sigma_r^2(i)}}$$

wherein $\mu$(i) and $\mu_r$(i) are the mean of morphometric characteristic (i) of said nucleoli or nucleolar connected components and said standard or reference nucleoli or nucleolar connected components, respectively; and wherein $\sigma$(i) and $\sigma_r$(i) are the standard deviation of morphometric characteristic (i) of said nucleoli or nucleolar connected components and said standard or reference nucleoli or nucleolar connected components, respectively;

wherein nucleolar disruption is scored based on the index of nucleolar disruption (iNo) which is derived from an increasing function of the absolute value of the morphometric characteristic discrepancy vector components, preferably as the L1-norm of the discrepancy vector, wherein said iNo is determined according to the following formula:

$$iNo = \sum^{N} (a(i) * |d(i)|)$$

wherein N is equal to 5 and is the number of said morphometric characteristics; wherein a(i) is a weighing factor for the ith morphometric characteristic, wherein preferably 0 < a(i) < 1; and wherein d(i) is the discrepancy vector component for the ith morphometric characteristic;

wherein the iNo value is the score and is is indicative of the severity of said nucleolar disruption.

2. A computer configured for executing the method according to claim 1.

3. A computer readable medium or data-carrier comprising computer-readable instructions which, when loaded on the internal memory of a computer, cause the computer to execute the method according to claim 1.

**Patentansprüche**

1. Computerimplementiertes Verfahren zum Scoring einer nukleolären Disruption, die das Bestimmen von morphometrischen Merkmalen der nukleolären Unterstützungsfläche und des nukleolären Intensitätsmusters umfasst;

wobei die nukleoläre Disruption basierend auf einem Kernfärbungsbild einer Eukaryontenzelle oder Gewebebildern, vorzugsweise basierend auf einem Kernfärbungsbild einer Population von Zellen, bewertet wird; wobei es sich bei den morphometrischen Merkmalen um Folgende handelt:

- die Nukleolusfläche des größten Nukleolus oder der größten nukleolären verbundenen Komponente in einer Zelle,
- den Faktor der elliptischen Regelmäßigkeit, wobei der Faktor der elliptischen Regelmäßigkeit als das Verhältnis zwischen der Fläche des größten Nukleolus oder der größten nukleolären verbundenen Komponente und der Fläche der kleinsten Ellipse bestimmt wird, die außerhalb des größten Nukleolus oder der größten nukleolären verbundenen Komponente liegt und denselben Mittelpunkt, dieselben Hauptachsen und dieselbe Ausdehnung wie der größte Nukleolus oder die größte nukleoläre verbundene Komponente aufweist,

- den Index für den niedrigen Schwanz des Texturhistogramms, wobei der Index für den niedrigen Schwanz des Texturhistogramms als der Prozentwert von Pixeln definiert ist, die unter einem Pixelintensitätsschwellenwert $\alpha$ des größten Nukleolus oder der größten nukleolären verbundenen Komponente in einer Zelle liegen,

- den Texturtälerindex, wobei der Texturtälerindex als die Zahl von lokalen Minima im Nukleolus oder in der nukleolären verbundenen Komponente des größten Nukleolus oder der größten nukleolären verbundenen Komponente in einer Zelle definiert ist, und

- das lokale Texturminimum, wobei das lokale Texturminimum als die Intensität des kleinsten lokalen Minimums im Nukleolus oder in der nukleolären verbundenen Komponente des größten Nukleolus oder der größten nukleolären verbundenen Komponente in einer Zelle definiert ist;

wobei der Nukleolus basierend auf einer Schwellenwertbildung der Bildpixelintensität als verbundene Komponente im schwellenwertbegrenzten Kernfärbungsbild identifiziert wird;

wobei die nukleoläre Unterstützungsfläche basierend auf einem Flächensegmentierungsschwellenwert bestimmt wird, wobei die Regelmäßigkeit der nukleolären Form basierend auf einem Formsegmentierungschwellenwert bestimmt wird und das Muster der nukleolären Textur basierend auf einem Textursegmentierungsschwellenwert bestimmt wird, wobei jeder Schwellenwert so definiert ist, dass die diskriminante Validität entsprechender morphometrischer Merkmale maximiert wird;

wobei die nukleoläre Disruption basierend auf der Diskrepanz in Bezug auf einen Standard- oder Referenz-Nukleolus bewertet wird;

wobei die Diskrepanz als ein Diskrepanzvektor bestimmt wird, der auf den Abständen zwischen den Verteilungen eines jeden morphometrischen Merkmals einer Population der Nukleoli oder der nukleolären verbundenen Komponenten und der Verteilung des entsprechenden morphometrischen Merkmals einer Population von Standard- oder Referenz-Nukleoli oder nukleolären verbundenen Standard- oder Referenzkomponenten basiert, wobei die Diskrepanzvektorkomponente, die mit einem gegebenen morphometrischen Merkmal (i) für eine bestimmte Nukleolus- oder eine nukleoläre verbundene Komponenten-Population d vorzugsweise gemäß der folgenden Formel bestimmt wird, die eine Diskrepanzvektorkomponente:

$$ d(i) = \frac{\mu(i) - \mu_r(i)}{\sqrt{\sigma^2(i) + \sigma_r^2(i)}} $$

darstellt, wobei $\mu(i)$ bzw. $\mu_r(i)$ der Mittelwert des morphometrischen Merkmals (i) der Nukleoli oder der nukleolären verbundenen Komponenten bzw. der Standard- oder Referenz-Nukleoli oder der nukleolären verbundenen Standard- oder Referenz-Komponenten sind und wobei $\sigma(i)$ bzw. $\sigma_r(i)$ die Standardabweichung des morphometrischen Merkmals (i) der Nukleoli oder der nukleolären verbundenen Komponenten bzw. der Standard- oder Referenz-Nukleoli oder der nukleolären verbundenen Standard- oder Referenz-Komponenten sind;

wobei die nukleoläre Disruption basierend auf dem Index (iNo) der nukleolären Disruption bewertet ist, der von einer steigenden Funktion des Absolutwerts der Komponenten des Diskrepanzvektors für morphometrische Merkmal vorzugsweise als L1-Norm des Diskrepanzvektors abgeleitet ist, wobei der iNo gemäß der folgenden Formel bestimmt wird:

$$ iNo = \sum_{i=1}^{N} a(i) * |d(i)|) $$

,

wobei N gleich 5 ist und die Zahl der morphometrischen Merkmale bedeutet; wobei a(i) ein Gewichtungsfaktor für das i. morphometrische Merkmal ist, wobei vorzugsweise $0 < a(i) < 1$; und wobei d(i) die Komponente des Diskrepanzvektors für das i. morphometrische Merkmal ist;

wobei der iNo-Wert der Score ist und den Schweregrad der nukleolären Disruption angibt.

**2.** Computer, der zur Ausführung des Verfahrens nach Anspruch 1 konfiguriert ist.

**3.** Computerlesbares Medium oder Datenträger, das bzw. der computerlesbare Anweisungen umfasst, die, wenn sie in den internen Speicher eines Computers geladen werden, bewirken, dass der Computer das Verfahren nach Anspruch 1 ausführt.

**Revendications**

1. Procédé mis en oeuvre par ordinateur pour noter une perturbation nucléolaire, comprenant la détermination de caractéristiques morphométriques de la zone de support nucléolaire et du motif d'intensité nucléolaire ;

   dans lequel la perturbation nucléolaire est notée sur la base d'une image de coloration nucléaire d'une cellule eucaryote ou d'images de tissu, de préférence sur la base d'une image de coloration nucléaire d'une population de cellules ; dans lequel lesdites caractéristiques morphométriques sont

   - la zone de nucléole du plus grand nucléole ou du plus grand composant nucléolaire relié dans une cellule,
   - le facteur de régularité elliptique, le facteur de régularité elliptique étant déterminé comme le rapport entre la zone du plus grand nucléole ou composant nucléolaire relié et la zone de la plus petite ellipse se situant à l'extérieur du plus grand nucléole ou composant nucléolaire relié et ayant le même centre, les mêmes axes principaux et le même allongement que le plus grand nucléole ou composant nucléolaire relié,
   - l'indice de queue basse d'histogramme de texture, l'indice de queue basse d'histogramme de texture étant défini comme le pourcentage de pixels qui se situent en dessous d'un seuil d'intensité de pixel $\sigma$ du plus grand nucléole ou du plus grand composant nucléolaire relié dans une cellule,
   - l'indice de vallées de texture, l'indice de vallées de texture étant défini comme le nombre de minima locaux dans le nucléole ou composant nucléolaire relié du plus grand nucléole ou du plus grand composant nucléolaire relié dans une cellule, et
   - le minimum local de texture, le minimum local de texture étant défini comme l'intensité du plus petit minimum local dans le nucléole ou composant nucléolaire relié du plus grand nucléole ou du plus grand composant nucléolaire relié dans une cellule ;

   dans lequel le nucléole est identifié sur la base d'un seuillage d'intensité de pixel d'image comme un composant relié dans l'image de coloration nucléaire seuillée ;
   dans lequel la zone de support nucléolaire est déterminée sur la base d'un seuil de segmentation de zone, dans lequel la régularité de forme nucléolaire est déterminée sur la base d'un seuil de segmentation de forme, et le motif de texture nucléolaire est déterminé sur la base d'un seuil de segmentation de texture, chaque seuil étant défini pour maximiser le pouvoir discriminant de caractéristiques morphométriques correspondantes ;
   dans lequel la perturbation nucléolaire est notée sur la base d'une divergence par rapport à un nucléole normal ou de référence ;
   dans lequel ladite divergence est déterminée comme un vecteur divergence sur la base des distances entre les distributions de chaque caractéristique morphométrique d'une population desdits nucléoles ou composants nucléolaires reliés et la distribution de la caractéristique morphométrique correspondante d'une population de nucléoles ou composants nucléolaires reliés normaux ou de référence, dans lequel la composante de vecteur divergence associée à une caractéristique morphométrique donnée (i) pour une population de nucléoles ou de composants nucléolaires reliés particulière d est de préférence déterminée selon la formule suivante représentant une composante de vecteur divergence :

   $$d(i) = \frac{\mu(i) - \mu_r(i)}{\sqrt{\sigma^2(i) + \sigma_r^2(i)}}$$

   dans lequel $\mu(i)$ et $\mu_r(i)$ sont la moyenne de la caractéristique morphométrique (i) desdits nucléoles ou composants nucléolaires reliés et desdits nucléoles ou composants nucléolaires reliés normaux ou de référence, respectivement ; et dans lequel $\sigma(i)$ et $\sigma_r(i)$ sont l'écart type de la caractéristique morphométrique (i) desdits nucléoles ou composants nucléolaires reliés et desdits nucléoles ou composants nucléolaires reliés normaux ou de référence, respectivement ;
   dans lequel la perturbation nucléolaire est notée sur la base de l'indice de perturbation nucléolaire (*iNo*) qui est dérivé d'une fonction croissante de la valeur absolue des composantes de vecteur divergence de caractéristique morphométrique, de préférence comme la norme L1 du vecteur divergence, dans lequel ledit *iNo* est déterminé selon la formule suivante :

   $$iNo = \sum_{i=1}^{N} (a(i) * |d(i)|)$$

dans lequel *N* est égal à 5 et est le nombre desdites caractéristiques morphométriques ; dans lequel *a*(*i*) est un facteur de pondération pour la i^ième caractéristique morphométrique ; de préférence dans lequel 0 < *a*(*i*) < 1 ; et dans lequel *d*(*i*) est la composante de vecteur divergence pour la i^ième caractéristique morphométrique ; dans lequel la valeur *iNo* est la note et est indicative de la sévérité de ladite perturbation nucléolaire.

2. Ordinateur configuré pour exécuter le procédé selon la revendication 1.

3. Moyen lisible par ordinateur ou support de données comprenant des instructions lisibles par ordinateur qui, lorsqu'elles sont chargées sur la mémoire interne d'un ordinateur, conduisent l'ordinateur à exécuter le procédé selon la revendication 1.

FIG. 1

Fig.2

**b** ——————— Effect of r-protein depletion on pre-rRNA processing ———————

Fig.2

EP 3 452 985 B1

C ————— Effect of r-protein depletion on p53 steady-state level —————

Fig.2

EP 3 452 985 B1

FIG. 3

FIG. 4

FIG. 5

**FIG. 6**

a

b

p53 steady-state level

FIG. S1

EP 3 452 985 B1

FIG. S2

64

FIG. S3

a

b

FIG. S4

a

b

# FIG. S5

a

b

Fig. S6

Fig. S6

Fig. S7

Fig. S7

FIG. S8

FIG. S9

Fig. S10

Fig. S10 continued

EP 3 452 985 B1

Fig. S10 continued

EP 3 452 985 B1

FIG. S11

a

Ethidium Bromide

Fig. S12

b

ITS1

Fig. S12

C

ITS2

Fig. S12

5'-ETS

Fig. S12

FIG. S13

EP 3 452 985 B1

FIG. S13
cont.

FIG. S14

FIG. S15

FIG. S16

Fig. S17

FIG. S18

FIG. S19

a

b

Fig. S20

Fig. S20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015157254 A

**Non-patent literature cited in the description**

- **WU et al.** Microscope Image Processing. Elsevier Inc, Academic Press, 2008
- **TAFFOREAU, L. et al.** The complexity of human ribosome biogenesis revealed by systematic nucleolar screening of Pre-rRNA processing factors. *Molecular cell,* 2013, vol. 51, 539-551
- **O'DONOHUE, M.F. ; CHOESMEL, V. ; FAUBLADIER, M. ; FICHANT, G. ; GLEIZES, P.E.** Functional dichotomy of ribosomal proteins during the synthesis of mammalian 40S ribosomal subunits. *The Journal of cell biology,* 2010, vol. 190, 853-866
- **ROBLEDO, S. et al.** The role of human ribosomal proteins in the maturation of rRNA and ribosome production. *Rna,* 2008, vol. 14, 1918-1929
- **CHEN, S.S. ; WILLIAMSON, J.R.** Characterization of the ribosome biogenesis landscape in E. coli using quantitative mass spectrometry. *Journal of molecular biology,* 2013, vol. 425, 767-779
- **SYKES, M.T. ; WILLIAMSON, J.R.** A complex assembly landscape for the 30S ribosomal subunit. *Annual review of biophysics,* 2009, vol. 38, 197-215
- **TALKINGTON, M.W. ; SIUZDAK, G. ; WILLIAMSON, J.R.** An assembly landscape for the 30S ribosomal subunit. *Nature,* 2005, vol. 438, 628-632
- **MULDER, A.M. et al.** Visualizing ribosome biogenesis: parallel assembly pathways for the 30S subunit. *Science,* 2010, vol. 330, 673-677
- **JOMAA, A. et al.** Functional domains of the 50S subunit mature late in the assembly process. *Nucleic acids research,* 2014, vol. 42, 3419-3435
- **FERREIRA-CERCA, S. et al.** Analysis of the in vivo assembly pathway of eukaryotic 40S ribosomal proteins. *Molecular cell,* 2007, vol. 28, 446-457
- **FERREIRA-CERCA, S. ; POLL, G. ; GLEIZES, P.E. ; TSCHOCHNER, H. ; MILKEREIT, P.** Roles of eukaryotic ribosomal proteins in maturation and transport of pre-18S rRNA and ribosome function. *Molecular cell,* 2005, vol. 20, 263-275
- **GAMALINDA, M. et al.** A hierarchical model for assembly of eukaryotic 60S ribosomal subunit domains. *Genes & development,* 2014, vol. 28, 198-210
- **VIOLA, P. ; JONES, M.** Robust real-time object detection. *IJCV,* 2004, vol. 57, 137-154
- **DALAL, N. ; TRIGGS, B.** Histograms of oriented gradients for human detection. *IEEE International Conference on Computer Vision and Pattern Recognition,* 2005
- **FELZENSZWALB, P. ; GIRSHICK, R.B. ; MCALLESTER, D. ; RAMANAN, D.** Object detection with discriminatively trained part-based models. *IEEE Trans on PAM,* 2010, vol. 32, 1627-1645
- **GANGBO, W. ; MCCANN, R.** The geometry of optimal transportation. *Acta Math,* 1996, vol. 177, 113-161
- **HAKER, S. ; ANGENENT, S. ; TANNENBAUM, A.** Minimizing flows for the monge-kantorovich problem. *SIAM J. Math Analysis,* 2003, vol. 35
- **WANG, W. ; SLEPCEV, D. ; BASU, S. ; OZOLEK, J.A. ; ROHDE, G.K.** A linear optimal transportation framework for quantifying and visualizing variations in sets of images. *Int. J. Computer Vision,* 2013, vol. 101, 254-269
- **OJALA, T. ; PIETIKÄINEN, M. ; HARWOOD, D.** A comparative study of texture measures with classification based on feature distributions. *Pattern Recognition,* 1996, vol. 29, 51-59
- **TUZEL, O. ; PORIKLI, F. ; MEER, P.** Region covariance: a fast descriptor for detection and classification. *ECCV,* 2006
- **QIN, X. ; YANG, Y.-H.** Similarity measure and learning with gray level aura matrices (GLAM) for texture image retrieval. *CVPR,* 2004
- **SERRA, J.** *Image analysis and mathematical morphology,* 1982
- **FISHER, R.A.** The use of multiple measurements in taxonomic problems. *Annals of Eugenics,* 1936, vol. 7, 179-188
- **WELCH, B.L.** The generalization of "Student's" problem when several different population variances are involved. *Biometrika,* 1947, vol. 34, 28-35

- **WOLFE, P. et al.** Using nuclear morphometry to discriminate the tumorigenic potential of cells: a comparison of statistical methods. *Cancer Epidmiol Biomarkers Prev,* 2004, vol. 13, 976-988
- **HERNANDEZ-VERDUN, D. ; ROUSSEL, P. ; THIRY, M. ; SIRRI, V. ; LAFONTAINE, D.L.J.** The nucleolus: structure/function relationship in RNA metabolism. *Wiley interdisciplinary reviews. RNA,* 2010, vol. 1, 415-431
- **BAN et al.** *PMID 24524803,* 2014
- **BOISVERT, F.M. ; VAN KONINGSBRUGGEN, S. ; NAVASCUES, J. ; LAMOND, A.I.** The multifunctional nucleolus. *Nature reviews. Molecular cell biology,* 2007, vol. 8, 574-585
- **LAFONTAINE, D.L.J.** Noncoding RNAs in eukaryotic ribosome synthesis and function. *Nature Structural and Molecular Biology,* 2015, vol. 22, 11-19
- **STEITZ, T.A.** A structural understanding of the dynamic ribosome machine. *Nature reviews. Molecular cell biology,* 2008, vol. 9, 242-253
- **MELNIKOV, S. et al.** One core, two shells: bacterial and eukaryotic ribosomes. *Nature structural & molecular biology,* 2012, vol. 19, 560-567
- **SCHMEING, T.M. ; RAMAKRISHNAN, V.** What recent ribosome structures have revealed about the mechanism of translation. *Nature,* 2009, vol. 461, 1234-1242
- **DANILOVA, N. ; GAZDA, H.T.** Ribosomopathies: how a common root can cause a tree of pathologies. *Dis Model Mech,* 2015, vol. 8, 1013-1026
- **NARLA, A. ; EBERT, B.L.** Ribosomopathies: human disorders of ribosome dysfunction. *Blood,* 2010, vol. 115, 3196-3205
- **ZHANG, Y. ; LU, H.** Signaling to p53: ribosomal proteins find their way. *Cancer cell,* 2009, vol. 16, 369-377
- **CHAKRABORTY, A. ; UECHI, T. ; KENMOCHI, N.** Guarding the 'translation apparatus': defective ribosome biogenesis and the p53 signaling pathway. *Wiley interdisciplinary reviews. RNA,* 2011, vol. 2, 507-522
- **LAM, Y.W. ; EVANS, V.C. ; HEESOM, K.J. ; LAMOND, A.I. ; MATTHEWS, D.A.** Proteomics analysis of the nucleolus in adenovirus-infected cells. *Molecular & cellular proteomics : MCP,* 2010, vol. 9, 117-130
- **MOORE, H.M. et al.** Quantitative proteomics and dynamic imaging of the nucleolus reveal distinct responses to UV and ionizing radiation. *Molecular & cellular proteomics : MCP,* 2011, vol. 10 (M111), 009241
- **THIRY, M. ; LAFONTAINE, D.L.J.** Birth of a nucleolus: the evolution of nucleolar compartments. *Trends in cell biology,* 2005, vol. 15, 194-199
- **LAMAYE, F. ; GALLIOT, S. ; ALIBARDI, L. ; LAFONTAINE, D.L.J. ; THIRY, M.** Nucleolar structure across evolution: the transition between bi- and tri-compartmentalized nucleoli lies within the class Reptilia. *Journal of structural biology,* 2011, vol. 174, 352-359
- **HERNANDEZ-VERDUN, D. ; ROUSSEL, P. ; THIRY, M. ; SIRRI, V. ; LAFONTAINE, D.L.J.** The nucleolus: structure/function relationship in RNA metabolism. *Wiley interdisciplinary reviews. RNA,* 2010, vol. 1, 415-431
- **SIRRI, V. ; HERNANDEZ-VERDUN, D. ; ROUSSEL, P.** Cyclin-dependent kinases govern formation and maintenance of the nucleolus. *The Journal of cell biology,* 2002, vol. 156, 969-981
- **LEUNG, A.K. et al.** Quantitative kinetic analysis of nucleolar breakdown and reassembly during mitosis in live human cells. *The Journal of cell biology,* 2004, vol. 166, 787-800
- **DERENZINI, M. ; MONTANARO, L. ; TRERE, D.** What the nucleolus says to a tumour pathologist. *Histopathology,* 2009, vol. 54, 753-762
- **BYWATER, M.J. et al.** Inhibition of RNA polymerase I as a therapeutic strategy to promote cancer-specific activation of p53. *Cancer cell,* 2012, vol. 22, 51-65
- **PELTONEN, K. et al.** A targeting modality for destruction of RNA polymerase I that possesses anti-cancer activity. *Cancer cell,* 2014, vol. 25, 77-90
- **BOULON, S. ; WESTMAN, B.J. ; HUTTEN, S. ; BOISVERT, F.M. ; LAMOND, A.I.** The nucleolus under stress. *Molecular cell,* 2010, vol. 40, 216-227
- **HEIN, N. ; HANNAN, K.M. ; GEORGE, A.J. ; SANIJ, E. ; HANNAN, R.D.** The nucleolus: an emerging target for cancer therapy. *Trends in molecular medicine,* 2013, vol. 19, 643-654
- **DE LA CRUZ, J. ; KARBSTEIN, K. ; WOOLFORD JR, J.L.** Functions of Ribosomal Proteins in Assembly of Eukaryotic Ribosomes In Vivo. *Annual review of biochemistry,* 2015, vol. 84, 93-129
- **BAN, N. et al.** A new system for naming ribosomal proteins. *Curr Opin Struct Biol,* 2014, vol. 24, 165-169
- **DONATI, G. ; PEDDIGARI, S. ; MERCER, C.A. ; THOMAS, G.** 5S ribosomal RNA is an essential component of a nascent ribosomal precursor complex that regulates the Hdm2-p53 checkpoint. *Cell reports,* 2013, vol. 4, 87-98
- **SLOAN, K.E. ; BOHNSACK, M.T. ; WATKINS, N.J.** The 5S RNP couples p53 homeostasis to ribosome biogenesis and nucleolar stress. *Cell reports,* 2013, vol. 5, 237-247
- **SASHITAL, D.G. et al.** A combined quantitative mass spectrometry and electron microscopy analysis of ribosomal 30S subunit assembly in E. coli. *eLife,* 2014, vol. 3

- **ANGER, A.M. et al.** Structures of the human and Drosophila 80S ribosome. *Nature,* 2013, vol. 497, 80-85
- **THIRY, M. ; LAMAYE, F. ; LAFONTAINE, D.L.J.** The nucleolus: when 2 became 3. *Nucleus,* 2011, vol. 2, 289-293
- **SCHMITZ, M.H. et al.** Live-cell imaging RNAi screen identifies PP2A-B55alpha and importin-beta1 as key mitotic exit regulators in human cells. *Nature cell biology,* 2010, vol. 12, 886-893
- **WILD, T. et al.** A protein inventory of human ribosome biogenesis reveals an essential function of exportin 5 in 60S subunit export. *PLoS biology,* 2010, vol. 8, e1000522
- **MULLINEUX, S.T. ; LAFONTAINE, D.L.J.** Mapping the cleavage sites on mammalian pre-rRNAs: where do we stand?. *Biochimie,* 2012, vol. 94, 1521-1532
- **BUNZ, F. et al.** Requirement for p53 and p21 to sustain G2 arrest after DNA damage. *Science,* 1998, vol. 282, 1497-1501
- **ZHANG, J. et al.** Assembly factors Rpf2 and Rrs1 recruit 5S rRNA and ribosomal proteins rpL5 and rpL11 into nascent ribosomes. *Genes & development,* 2007, vol. 21, 2580-2592
- **RUGGERO, D. ; PANDOLFI, P.P.** Does the ribosome translate cancer?. *Nature reviews. Cancer,* 2003, vol. 3, 179-192
- **JAMES, A. ; WANG, Y. ; RAJE, H. ; ROSBY, R. ; DIMARIO, P.** Nucleolar stress with and without p53. *Nucleus,* 2014, vol. 5
- **FUMAGALLI, S. ; IVANENKOV, V.V. ; TENG, T. ; THOMAS, G.** Suprainduction of p53 by disruption of 40S and 60S ribosome biogenesis leads to the activation of a novel G2/M checkpoint. *Genes & development,* 2012, vol. 26, 1028-1040
- **SUN, X.X. ; WANG, Y.G. ; XIRODIMAS, D.P. ; DAI, M.S.** Perturbation of 60 S ribosomal biogenesis results in ribosomal protein L5- and L11-dependent p53 activation. *The Journal of biological chemistry,* 2010, vol. 285, 25812-25821
- **BURSAC, S. et al.** Mutual protection of ribosomal proteins L5 and L11 from degradation is essential for p53 activation upon ribosomal biogenesis stress. *Proceedings of the National Academy of Sciences of the United States of America,* 2012, vol. 109, 20467-20472
- **LO, K.Y. et al.** Defining the pathway of cytoplasmic maturation of the 60S ribosomal subunit. *Molecular cell,* 2010, vol. 39, 196-208
- **BEN-SHEM, A. et al.** The structure of the eukaryotic ribosome at 3.0 A resolution. *Science,* 2011, vol. 334, 1524-1529
- **KHATTER, H. ; MYASNIKOV, A.G. ; NATCHIAR, S.K. ; KLAHOLZ, B.P.** Structure of the human 80S ribosome. *Nature,* 2015, vol. 520, 640-645
- **MADRU, C. et al.** Chaperoning 5S RNA assembly. *Genes & development,* 2015, vol. 29, 1432-1446
- **LEIDIG, C. et al.** 60S ribosome biogenesis requires rotation of the 5S ribonucleoprotein particle. *Nature communications,* 2014, vol. 5, 3491
- **BASSLER, J. et al.** A network of assembly factors is involved in remodeling rRNA elements during preribosome maturation. *The Journal of cell biology,* 2014, vol. 207, 481-498
- **YUSUPOV, M.M. et al.** Crystal structure of the ribosome at 5.5 A resolution. *Science,* 2001, vol. 292, 883-896
- **RUBBI, C.P. ; MILNER, J.** Disruption of the nucleolus mediates stabilization of p53 in response to DNA damage and other stresses. *The EMBO journal,* 2003, vol. 22, 6068-6077
- **FUMAGALLI, S. et al.** Absence of nucleolar disruption after impairment of 40S ribosome biogenesis reveals an rpL11-translation-dependent mechanism of p53 induction. *Nature cell biology,* 2009, vol. 11, 501-508